(19)

Europäisches Patentamt
European Patent Office
Office européen des brevets

(11) **EP 4 116 403 A1**

(12) **EUROPEAN PATENT APPLICATION**

(43) Date of publication:
**11.01.2023 Bulletin 2023/02**

(21) Application number: **21184299.2**

(22) Date of filing: **07.07.2021**

(51) International Patent Classification (IPC):
**C12M 1/36** $^{(2006.01)}$   **G05B 13/04** $^{(2006.01)}$
**G16B 40/00** $^{(2019.01)}$

(52) Cooperative Patent Classification (CPC):
**C12M 41/48; G05B 13/048**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sartorius Stedim Data Analytics AB 903 33 Umeå (SE)**

(72) Inventors:
• **MCCREADY, Christopher**
  **Oakville, L6M 2V9 (CA)**
• **SJÖGREN, Rickard**
  **903 33 Umea (SE)**
• **CEDERGREN, Linnéa**
  **903 25 Umeå (SE)**

(74) Representative: **Mewburn Ellis LLP**
**Aurora Building**
**Counterslip**
**Bristol BS1 6BX (GB)**

(54)   **MONITORING, SIMULATION AND CONTROL OF BIOPROCESSES**

(57)   Methods for monitoring, controlling, optimising and simulating a bioprocess comprising a cell culture in a bioreactor are provided. The methods comprise obtaining the values of one or more state variables of a state space model at one or more maturities, and predicting the value of one or more critical quality attributes of a product of the bioprocess using a machine learning model trained to predict the value of the one or more critical quality attributes based on input variables comprising values of the one or more state variables or variables derived therefrom, at one or more maturities. The state space model comprises a kinetic growth model representing changes in the state of the cell culture and a material balance model representing changes in the bulk concentration of one or more metabolites in the bioreactor. Systems, computer readable media implementing such methods, and methods for providing tools to implement such methods, are also provided.

Fig. 4

EP 4 116 403 A1

## Description

Field of the Present Disclosure

The present disclosure relates to computer implemented methods, computer programs and systems for the monitoring, simulation, optimisation and control of bioprocesses. Particular methods, programs and systems of the disclosure use machine learning to predict one or more critical quality attributes of a product in a bioprocess.

Background

Bioprocesses use biological systems to produce a specific biomaterial, for example a biomolecule with therapeutic effects. This process typically involves placing cells and/or microbes into a bioreactor with media containing nutrients under controlled atmospheric conditions. The media is consumed by the cells and used for growth and other metabolic functions, including production of the specific biomaterial and by-products.

The bioreactor typically contains or is associated with instrumentation that continuously (e.g., once every second, minute, hour) measures process conditions, such as temperature, pH, and dissolved oxygen, as well as addition of nutrients and gasses, and flow and content of streams leaving the bioreactor. Typically, samples of the culture are taken periodically (e.g., once or twice per day, or more) to measure the content of the bulk fluid, including the concentration of one or more metabolites (e.g., glucose, glutamine, lactate, NH4, etc.), cell metrics such as total cells and viable cell density (VCD), and the concentration of the product biomaterial (also referred to as titer). Quality metrics of the product biomaterial (sometimes also called "quality attributes" or "critical quality attributes" (CQA) such as e.g. the glycosylation profile or activity of the product are also typically measured. These are typically only measured once at the end of the process, and are available with a significant delay (e.g. days or weeks) after offline measurement. Because measurements of many CQAs is very slow, quality is often only determined after the process has run, and corrective actions can no longer be taken.

Statistical process analysis methods can be used to assess satisfactory performance of a bioprocess. In particular, multivariate statistical models (including principal component analysis - PCA - and (orthogonal) partial least square regression - (O)PLS) have become a popular tool for identifying process conditions that are important to ensure that CQAs are within specification (collectively referred to as "critical process parameters", CPPs) and to establish acceptable ranges of these process conditions as a bioprocess progresses to completion. Such tools have been implemented in the Umetrics® software suite (Sartorius Stedim Data Analytics), which is a leading data analytics software for modelling and optimizing biopharmaceutical development and manufacturing processes.

In a typical bioprocess analysis, a series of process variables (e.g. a few dozen process variables including temperature, concentration of key nutrients and metabolites, pH, volume, gas concentrations, viable cell density, etc.) are measured during completion of the bioprocess. These process variables together represent the "process condition". Many of these variables are highly correlated and as such methods such as PCA and PLS can be used to identify summary variables that capture the correlation structure in the data. These (typically relatively few) variables can then be extracted and the range of values of theses variables that define a "normal" process condition (expected to result in a product with acceptable CQAs) can be estimated. Using these approaches, a bioprocess is run by keeping CPPs within defined limits or on a specified trajectory, which have been determined to result in an a product with CQAs considered to represent an acceptable quality product. The connection between the CPPs and the CQAs is often not well understood, resulting in CPP limits that are based solely on historical observation. These can be both unnecessarily constraining and insufficient to ensure the CQAs depending on the situation.

Therefore, a need exists for a system and method for improved methods for monitoring, simulating and controlling bioprocesses so as to ensure acceptable CQAs, which do not suffer from the all of the drawbacks of the prior art.

Summary

According to a first aspect of the disclosure, there is provided a method for monitoring a bioprocess comprising a cell culture in a bioreactor, the method including the steps of: obtaining the values of one or more state variables of a state space model and optionally one or more variables derived therefrom, at one or more maturities, the state space model comprising a kinetic growth model representing changes in the state of the cell culture and optionally a material balance model representing changes in the bulk concentration of one or more metabolites in the bioreactor; and predicting the value of one or more critical quality attributes of a product of the bioprocess using a machine learning model trained to predict the value of the one or more critical quality attributes based on input variables comprising values of the one or more state variables or variables derived therefrom, at one or more maturities.

Within the context of the present disclosure, maturity may be expressed in units of time. For example, maturity may refer to an amount of time since the start of a bioprocess or any other reference time. Thus, reference to particular "maturities" and "functions of maturity" should be interpreted to encompass "time points", "times" and "functions of time".

The method of the first aspect may have any one or any combination of the following optional features.

The method may further comprise obtaining values of one or more process conditions including one or more process parameters and/or one or more metabolite concentrations at one or more maturities. The input variables may further comprise values of the one or more process conditions. The input variables may comprise values of at least one of the one or more state variables. The input variables may comprise values of at least one of the one or more state variables, and values of at least one variable derived from one or more state variables. Obtaining the values of one or more process conditions may comprise directly or indirectly measuring the values of one or more process conditions. Obtaining the values of one or more process conditions may comprise receiving the values, for example from a user, computing device or memory.

A kinetic growth model may comprise one or more equations representing changes in one or more of the viable cell density $x_v$, the dead cell density $x_d$, the total cell density $x_t$ and the lysed cell density $x_l$ of the cell culture as a function of time/maturity. The kinetic growth model may comprise a Monod kinetic model or a saturation kinetic model. The kinetic growth model may be configured to estimate microbial cell growth as a function of time. For example, any or all of equations (11) to (14), or equations derived therefrom, may be used. For example, equations may be derived from these by: substituting one or more terms in equations (11)-(13) by a corresponding term derived from equation (14), removing one or more terms of any of equations (11)-(14) in line with corresponding assumptions (such as e.g. where a particular flow is assumed to be absent), or adding one or more terms of any of equations (11)-(14) in line with corresponding assumptions (such as e.g. where an additional flow is assumed to be present). In equations (11)-(13), the terms $\mu_{eff}$ and/or $\mu_d$ may be modeled using any of equations (15)-(24). A material balance model may comprise one or more equations representing changes in the bulk concentration of one or more metabolites in the bioreactor. For example, for each of the one or more metabolites, a material balance model may comprise an equation of the form given by equation (25), such as e.g. equation (26) or equivalents (e.g. equations (26a)-(26d)).

The product may comprise one or more biomolecules, such as one or more small or macromolecules produced by the cells. The product may comprise the biomass in the culture, and/or parts thereof such as one or more organelles. The critical quality attribute may comprise any physical, chemical, biological or microbiological property or characteristic that should be below or above an appropriate threshold, within an appropriate range or belong to an appropriate distribution, to ensure the desired quality of the product (where the appropriate threshold, range or distribution may be predetermined depending on the property/characteristic and the desired quality of the product). The one or more critical quality attributes may be selected from: the molecular structure of a small molecule or macromolecule that is comprised in the product or is the product, the glycosylation profile of a protein or peptide that is comprised in the product or is the product, the activity of the product, the yield of the product, the presence or absence of one or more host cell protein(s), and the purity of the product. The purity of the product may also be expressed as the concentration of one or more contaminants. The activity of a product may refer to any activity of interest, such as e.g. the rate of a particular reaction or set of reactions in the presence of the product (e.g. through catalysis of the reaction by the product), the binding affinity of a product to one or more targets, etc. The activity of the product may refer to an enzymatic activity associated with a product comprising an enzyme, where the product can be a cell culture or a purified composition comprising an enzyme produced by the cell culture. The purity and/or activity of a product may refer to the purity and/or activity of the raw product of the bioprocess or to the purity and/or activity of the product of the bioprocess after one or more downstream processing steps such as e.g. purification.

The one or more state variables or variables derived therefrom may comprise at least one variable selected from: a specific transport rate of a metabolite, a bulk fluid concentration of a metabolite, a bulk fluid concentration of a product, the bulk fluid concentration of a biomaterial that accumulates in the culture as a result of cell growth and inhibits cell growth, specific productivity of titer, and a cell state variable. The cell state variable may be selected from: viable cell density, dead cell density, total cell density, cell viability, effective growth rate, death rate, and lysed cell density. The one or more state variables or variables derived therefrom are obtained using any of equations (11)-(30), and equivalents thereof, in particular any or all of equations (11)-(16), (22), (25)-(27) and equivalents thereof. The specific transport rate of a metabolite i at a first maturity may be derived from one or more state variables comprising the concentration of the metabolite i at the first maturity and a second maturity preceding the first maturity. This can be performed using a material balance model, for example using equation (27) or equivalents (e.g. equations (27a)-(27f), or any other equation capturing the flows in equation (25)). The specific transport rate of a metabolite i at a first maturity may be derived from one or more state variables comprising the concentration of the metabolite i at one or more maturities and the value of one or more process conditions at the one or more maturities using a machine learning model trained to predict the specific transport rate of one or more metabolites comprising metabolite i based on input variables comprising the concentration of the metabolite i or a precursor thereof at one or more maturities and/or the value of one or more process conditions at the one or more maturities. The specific transport rate of a metabolite i may be the net amount of the metabolite transported between the cells and the culture medium, per cell and per unit of maturity. The one or more maturities may comprise the first maturity, and/or one or more maturities preceding the first maturity. Preferably, the one or more values of process conditions used to predict the specific transport rates of the one or more metabolites include at least one metabolite concentration value. The one or more values of process conditions may further include at least one further value of a process condition, preferably at least two further values. The one or more values of metabolite concentrations may include the concentration of one or more metabolites for which specific transport rates are determined. The one or more metabolites may instead or in addition include the concentration of one or more metabolites that are precursors of one or more metabolites for which specific transport rates are determined. The one or more metabolites may instead or in addition include the concentration of one or more metabolites that are products of reactions that produce or consume one or more metabolites for which specific transport rates are determined. A bulk fluid concentration of a metabolite may be obtained using material balance equations, such as equation (26) or equivalents thereof (e.g. equations (25) and (26a)-(26d)). The viable cell density may be obtained using equation (11) or equivalents thereof. The cell viability may be obtained as the ratio of the viable and total cell densities, using equations (11)-(14) or equivalents thereof. The dead cell density may be obtained using equation (12) or equivalents thereof. The lysed cell density may be obtained using equation (13) or equivalents thereof. The bulk fluid concentration of a biomaterial that accumulates in the culture as a result of cell growth and inhibits cell growth may be obtained using equation (16) or equivalents thereof. The bulk fluid concentration of a product may be obtained using material balance equations, such as equation (26) or equivalents thereof (e.g. equations (25) and (26a)-(26d)), or equation (30). The effective growth rate may be obtained using equation (22) or equivalents thereof. The death rate may be obtained using equation (15) or equivalents thereof. The specific productivity of titer may be obtained using a multivariate model (such as PLS or (O)PLS) configured to predict the specific productivity of titer as a function of metabolic condition as described herein, such as e.g. using variables selected from the specific transport rate of one or more metabolites, one or more metabolite concentrations and one or more process variables. Equivalents of the equations provided may refer to equations that capture the same processes but differ by one or more underlying model assumptions.

The one or more process conditions include one or more process parameters selected from dissolved oxygen, dissolved CO2, pH, temperature, osmolality, agitation speed, agitation power, headspace gas composition (such as e.g. $CO_2$ pressure), flow rates (such as feed rate, bleed rate, harvest rate), feed medium composition and volume of the culture; and/or wherein the one or more metabolite concentrations include the concentration of one or more metabolites in the cellular compartment, in the culture medium compartment, or in the cell culture as a whole.

The method may further comprise comparing the value of the one or more critical quality attributes to one or more predetermined values. The method may further comprise determining whether the bioprocess is operating normally (or according to specification) based on the comparing. The predicted value of the one or more critical quality attributes of a product of the bioprocess may be associated with a maturity. The maturity associated with the predicted value of the one or more critical quality attributes may be one of the one or more maturities associated with values provided as inputs to the machine learning model. Alternatively, the maturity associated with the predicted value of the one or more critical quality attributes may not be one of the one or more maturities associated with values provided as inputs to the machine learning model. For example, it may be a future maturity such as the final maturity (e.g. end of a batch or fed-batch bioprocess). The one or more critical quality attributes may be associated with a maturity and comparing the one or more critical quality attributes to one or more predetermined values may comprise comparing the one or more critical quality attributes to one or more predetermined values associated with a corresponding maturity (e.g. the same or a similar maturity such as the closest maturity for which a predetermined value is available). Comparing the value(s) of the one or more critical quality attributes to one or more predetermined values may comprise comparing the value(s) of the one or more critical quality attributes to one or more respective predetermined threshold, such as e.g. a lower threshold and/or an upper threshold. For example, comparing the value(s) of the one or more critical quality attributes to one or more predetermined values may comprise determining whether the value(s) of a critical quality attribute is within a predetermined range, above a predetermined lower threshold, or below a predetermined upper threshold. The thresholds and/or ranges may be determined based on the value of the CQA that is considered to result in an acceptable product. Comparing the value(s) of the one or more critical quality attributes to one or more predetermined values may comprise comparing the value(s) of the one or more critical quality attributes to the average value for the corresponding variables in a set of bioprocesses that are considered to operate according to specification. The one or more predetermined values may be determined based on one or more desired features of the product. For example, a minimum yield, purity or activity, or a particular glycosylation profile may be necessary for the product to be usable. A bioprocess may be considered to operate according to specification if the value(s) of the one or more critical quality attributes is/are within a predetermined range of the average value for the respective corresponding variable in a set of bioprocesses that are considered to operate normally. The predetermined range may be defined as a function of the standard deviation associated with the average value of the respective corresponding variables across the set of bioprocesses considered to operate normally. A bioprocess may be considered to operate normally if the value of one or more variables c is within a range defined as *average(c)*±*n*SD(c)*, where *average(c)* is the average value of the variable c in a set of bioprocesses that are considered to operate normally, *SD(c)* is the standard deviation associated with *average(c)*, and *n* is a predetermined constant (which may be the same for the subrange *average(c)+n*SD(c)* and for the subrange *aver-*

*age(c)-n\*SD(c),* or may differ between these subranges). The value of *n* may be chosen as 1, 2, 3 or a value that results in a chosen confidence interval, for example a 95% confidence interval. A bioprocess may be considered to operate normally if the value of one or more variables c is within a range defined as a confidence interval, e.g. a 95% confidence interval, around *average(c),* based on an assumed distribution of c. An assumed distribution may be a Gaussian (normal) distribution, a chi-squared distribution, etc. Where the assumed distribution is a normal distribution, a p% confidence interval (where p can be e.g. 95) may be equivalent to a range of *average(c) $\pm$ n\*SD(c),* where *n* is a single value that results in the p% confidence interval (e.g. *n* may be about 1.96 for a 95% confidence interval). The method may further comprise outputting a signal to a user if the comparison step indicates that the bioprocess is not operating normally. A signal may be output through a user interface such as a screen, or through any other means such as audio or haptic signalling.

The machine learning model may be a regression model. The machine learning model may be selected from a linear regression model, a random forest regressor, an artificial neural network (ANN), and a combination thereof. Suitably, the machine learning model may be an artificial neural network. The machine learning model may comprise a plurality of machine learning models, wherein each machine learning model has been trained to predict the values of an individually selected subset of the one or more critical quality attributes. The machine learning model may have been trained to jointly predict value of the one or more critical quality attributes. Advantageously, the machine learning model is an ANN or an ensemble of ANNs. The present inventors have found that ANNs were particularly suited to the task at hand. Without wishing to be bound by theory, the inventors believe that this is at least in part because ANNs are well suited to predict values using input data that has a complex correlation structure. Thus, ANNs performed particularly well in predicting a plurality of critical quality attributes jointly. The machine learning model may comprise a plurality of machine learning models, wherein each machine learning model has been trained to predict one or more of a plurality of critical quality attributes. Predicting a plurality of critical quality attributes (i.e. all or a subset thereof) jointly may advantageously increase the accuracy of the prediction where the critical quality attributes that are jointly predicted are correlated with each other. The machine learning model may have been trained using data from a plurality of similar bioprocesses, wherein a similar bioprocess is one that uses the same cells for the same purpose. The machine learning model may have been trained using data from a plurality of similar bioprocesses in which at least some of the bioprocesses differ from each other by one or more process conditions as a function of maturity. The machine learning model may have been trained to predict the value of the one or more critical quality attributes at a maturity corresponding to the end of the bioprocess based on input variables comprising values of the one or more state variables or variables derived therefrom and optionally values of one or more process conditions, at one or more preceding maturities. In such embodiments, the bioprocess may be a batch or a fed-batch process. The machine learning model may have been trained to predict the value of the one or more critical quality attributes at a current maturity based on input variables comprising values of the one or more state variables or variables derived therefrom and optionally values of one or more process conditions, at one or more maturities including the current maturity and/or one or more preceding maturities. In such embodiments, the bioprocess may be a perfusion process. The one or more maturities may include the current maturity.

Obtaining values of one or more state variables and optionally one or more process conditions at one or more maturities may comprise obtaining values of the one or more state variables and optionally one or more process conditions at a plurality of maturities; and the machine learning model may have been trained to predict the one or more critical quality attributes at a latest of the plurality of maturities or a later maturity using inputs comprising the value of the one or more state variables and optionally one or more process conditions for the bioprocess at the plurality of maturities. For example, the machine learning model may have been trained to predict the one or more critical quality attributes at a latest of two distinct maturities or a later maturity based on input values comprising the values of one or more state variables and optionally one or more process conditions for the bioprocess, at the two distinct maturities. The values of one or more state variables and optionally process conditions used as input to the machine learning model may be associated with a plurality of maturities that are separated from each other by a difference in maturity that is approximately equal to the difference in maturity between the values used to train the machine learning model. Predicting the critical quality attributes using predictor variables (i..e input variables) at a plurality of maturities may advantageously increase the accuracy of the predictions, compared to the use of predictor variables at a single maturity. Without wishing to be bound by theory, it is believed that the accuracy of machine learning predictions are likely to be increased by using predictor variables at a plurality of time points because such data can capture information about the dynamics of the bioprocess.

Obtaining the values of one or more state variables of a state space model and/or variables derived therefrom at one or more maturities may comprise predicting a state trajectory of the bioprocess using the state space model. Predicting a state trajectory of the bioprocess using the state space model may comprise finding values of the one or more state space variables that represent a solution of the state space model at the one or more maturities. In other words, obtaining the values of one or more state variables of a state space model or variables derived therefrom at one or more maturities may comprise. solving the state space model at the or more maturities, for example by integrating one or more equations that together form the model, or using any other suitable approach depending on the model such as e.g. stochastic simulation. The state of the process may include the value of any of the variables in such models, such as e.g. the value of one or more cell culture state variables (also referred to herein as "cell culture parameters" and/or metabolite concentrations). The state trajectory of the process may include the value of state variables at a plurality of time points/maturities. Thus, the concentration of a metabolite i may be seen as a process condition (for example, where the concentration is an initial condition of the bioprocess / state space model, or where the concentration is not a concentration that is captured as a state variable in the model) or as a state variable of the state space model (for example, where the concentration is a state variable of the state space model and is obtained by solving the state space model at a given maturity or plurality of maturities). Obtaining the values of one or more state variables of a state space model or variables derived therefrom at one or more maturities may comprise obtaining values of one or more state variables by directly or indirectly measuring the values of the one or more variables, or by receiving the values, for example from a user, computing device or memory. Further, some of the values of one or more state variables may be received (e.g. obtained by measurement, from a user, computing device or memory) and some may be determined as part of the present method by predicting a state trajectory of the bioprocess using the state space model. Predicting a state trajectory of the bioprocess using the state space model may comprise determining the value of the one or more state space variables at a single maturity, or at a plurality of maturities. In other words, a trajectory may comprise values for a set of state variables at a single maturity.

Predicting a state trajectory of the bioprocess using the state space model may comprise predicting the specific transport rate of every metabolite i in the model at a first maturity using a machine learning model as described above, and using the predicted specific transport rate(s) to determine the concentration of the corresponding one or more metabolites at the later maturity using the material balance model. For example, determining the concentration of the corresponding one or more metabolites at the later maturity comprises solving respective material balance equations. For example, determining the concentration of a metabolite i at maturity k, where k is the maturity associated with the predicted specific transport rate, may comprise integrating any of equations (25), (26a)-(26c) and (29) (e.g. using any of equations (27a)-(27f)) between a preceding maturity at which $m_i$ is known and maturity k. The method may further comprise using one of more of said concentrations to determine the value of a cell state variable at the later maturity. Determining the value of cell state variable at the later maturity may comprises solving the kinetic growth model. Solving a kinetic growth model at maturity k may comprise integrating any of equations (11) to (14) between a preceding maturity at which $x_v, xi, x_d$ and/or $x_t$ are known and maturity k. The method may further comprise predicting the specific transport rate of every metabolite i in the model at a further maturity using one or more of the said metabolite concentrations and/or biomass related metric values as inputs to the machine learning model trained to predict specific transport rates.

Predicting a state trajectory of the bioprocess using the state space model may comprise determining the specific transport rate of one or more metabolites i in the model at a first maturity using a material balance model, for example using equation (27) or equivalents (e.g. equations (27a)-(27f), or any other equation capturing the flows in equation (25)), and the concentration of the one or more metabolites at a first and preceding maturities. The method may further comprise using one of more of said concentrations at the first maturity to determine the value of a cell state variable at the first maturity. Determining the value of a cell state variable at the first maturity may comprises solving the kinetic growth model. The process may be repeated at one or more further maturities to determine the specific transport rate of one or more metabolites and the value of one or more cell state variables at each of said further maturities. The method may further comprise predicting the effect of a particular value of a process parameter by including the particular value in the material balance model, the kinetic growth model and/or the input values used by the machine learning model to predict specific transport rates and/or the input values used by the machine learning model to predict critical quality attributes. The one or more metabolites may comprise a desired product. Similarly, the biomass itself may comprise a desired product. Thus, the method may comprise predicting the concentration of a desired product in a bioprocess using a state space model.

The methods of the present aspect may be performed using process condition values that are received from a user, computing device or memory. The methods of the present aspect may be performed using process condition values acquired in real time during the operation of a bioprocess. Such process condition values may comprise operational settings (i.e. parameters that are set by an operator), and/or values that are measured inline or offline during operation of the bioprocess. Therefore, the methods of monitoring a bioprocess described herein may be implemented in real time, i.e. during operation of the bioprocess. In such embodiments, the step of obtaining values of one or more process conditions may comprise receiving values for the one or more process conditions at the latest maturity for which such values have been measured or determined, and optionally obtaining values of the one or more process conditions at one or more preceding maturities from a data store.

Any of the methods of the present aspect may comprise recording the values of one or more process conditions, the values of the one or more state variables, the determined critical quality attributes and/or values derived therefrom in a data store. The methods may further comprise predicting the effect of a particular value of a process parameter at a later maturity by including the particular value in the material balance equations, the kinetic growth model and/or the input values used by the machine learning model to predict critical quality attributes at a further maturity.

Predicting a state trajectory of the bioprocess may be equivalent to simulating a bioprocess. Thus, also described according to the present aspect are methods of simulating a bioprocess comprising a cell culture in a bioreactor, the methods comprising: obtaining a set of initial conditions comprising: values of one or more process conditions including one or more process parameters, and values of one or more state variables of a state space model, at one or more initial maturities, the state space model comprising a kinetic growth model representing changes in the state of the cell culture and optionally a material balance model representing changes in the bulk concentration of one or more metabolites in the bioreactor; obtaining the values of one or more state variables of the state space model and optionally one or more variables derived therefrom at one or more subsequent maturities by predicting a state trajectory of the bioprocess using the state space model and the set of initial conditions; and predicting the value of one or more critical quality attributes of a product of the bioprocess using a machine learning model trained to predict the value of the one or more critical quality attributes based on input variables comprising values of the one or more state variables or variables derived therefrom, and optionally values of one or more process conditions, at one or more maturities selected, and input variables comprising values of the one or more state variables and/or variables derived therefrom at one or more maturities selected from the one or more subsequent maturities. Values for any process parameters used as input to the machine learning model may be obtained from a user interface, computing device or memory. For example, trajectories for one or more process parameters may be provided as input to the method, wherein a trajectory for a process parameter comprises values for the process parameter at a plurality of maturities.

According to a second aspect, there is provided a method for controlling a bioprocess, the method comprising: performing the method of any embodiment of the first aspect; comparing the value of the one or more critical quality attributes or values derived therefrom to one or more predetermined values; and determining on the basis of the comparison whether to implement a corrective action. The method may further comprise sending a signal to one or more effector device(s) to implement a corrective action if the determining step indicates that a corrective action is to be implemented.

The method according to the present aspect may have any of the features disclosed in relation to the first aspect. The method of the present aspect may further have any one or any combination of the following optional features.

Determining on the basis of the comparison whether to implement a corrective action may comprise determining on the basis of the comparison whether the bioprocess is operating normally, and sending a signal to one or more effector device(s) to implement a corrective action if the comparison step indicates that the bioprocess is not operating normally. Determining on the basis of the comparison whether to implement a corrective action may comprise determining on the basis of the comparison whether the bioprocess is operating optimally, and sending a signal to one or more effector device(s) to implement a corrective action if the comparison step indicates that the bioprocess is not operating optimally. Determining whether the bioprocess is operating optimally may comprise determining whether a different set of one or more process conditions is associated with improved values of the one or more critical quality attributes or values derived therefrom. In such embodiments, the one or more predetermined values may comprise values associated with one or more different sets of process conditions.

The method may further comprise repeating the steps of the method of monitoring the bioprocess, after a predetermined period of time has elapsed since obtaining the values of the one or more state variables and optionally the values of the one or more process conditions. A corrective action may be associated with a change in the value of one or more process conditions. The method may further comprise determining a corrective action to be implemented by predicting the effect of the corrective action by including the value in the state space model and/or the input used by the machine learning model to predict critical quality attributes. An effector device may be any device coupled to the bioreactor and which is configured to change one or more physical or chemical conditions in the bioreactor.

According to a third aspect, there is provided a method of optimising a bioprocess comprising a cell culture in a bioreactor, the method comprising:

performing the method of any embodiment of the first aspect using a first set of process conditions and at least a further set of process conditions (e.g. each being used to predict a state trajectory using the state space model, thereby obtaining values for one or more state variables and optionally one or more variables derived therefrom associated with each set of process conditions); and determining whether the further set of process conditions is preferable to the first set of process conditions by comparing the critical quality attributes or values derived therefrom associated with the respective sets of process conditions.

Comparing the critical quality attributes or values derived therefrom associated with the respective sets of process conditions may comprise determining, for each set of process conditions, the value of a desirability function which penalises values of critical quality attributes that are outside of predetermined ranges. The ranges may be closed or openended. The desirability function may reward one or more objectives that apply to metrics of the bioprocess, such as maximising yield, concentration of a desired product, total amount of a desired product produced over a period of time, etc. For example, the desirability function may comprise one or more terms that decrease the value of the function if critical quality attributes or values derived therefrom are outside of predetermined ranges, and optionally one or more terms that increase the value of the function as one or more metrics of the bioprocess increase (e.g. in the case of yield) or decrease (e.g. in the case of feed consumption).

The method may further comprise selecting a further set of process conditions, and comparing the critical quality attributes or values derived therefrom associated with the further set of process conditions with those associated with one or more previously used sets of conditions. The step of selecting a further set of process conditions may comprise receiving a further set of conditions from a user interface, obtaining a further set of conditions from a database or computing device, using an optimisation algorithm to determine a further set of conditions, or combinations thereof. The method of the present aspect may further comprise: identifying a set of process conditions as optimal process conditions for the bioprocess, and operating a bioprocess according to the set of process conditions identified. The method may further comprise: measuring experimental process conditions of the bioprocess using one or more sensors as a function of maturity; monitoring the measured experimental process conditions to detect deviations from the optimal process conditions; and when a deviation is detected, sending a notification to a user and/or providing feedback to a controller controlling the bioreactor to automatically adjust the experimental process conditions to minimize deviation from the optimal process conditions.

According to a fourth aspect, there is provided a method of providing a tool for monitoring a bioprocess comprising a cell culture in a bioreactor, the method including the steps of: obtaining the values of one or more state variables of a state space model and optionally one or more variables derived therefrom and/or the values of one or more process conditions, at one or more maturities for a plurality of bioprocesses, the state space model comprising a kinetic growth model representing changes in the state of the cell culture and optionally a material balance model representing changes in the bulk concentration of one or more metabolites in the bioreactor; and using the values obtained to train a machine learning model to predict the value of one or more critical quality attributes based on input variables comprising values of the one or more state variables and/or variables derived therefrom, at one or more maturities. The method may comprise any of the features described in relation to the first aspect.

The method of any aspect may further comprise parameterising the state space model. For example, parameterising the state space model may comprise obtaining a measurement corresponding to a state variable for a bioprocess (for example by obtaining a test sample from a bioprocess), predicting the value of one or more state variables for the bioprocess using the state space model with a first set of parameters, and determining whether the measurement is within a range of the values predicted by the state space model with the first set of parameters. Parameters of the state space model may include coefficients associated with the state space model, such as e.g. any coefficient of equations (11) to (29). The method may further comprise providing a second set of parameters based on the difference between the measurement and the values predicted by the state space model with the first set of parameters.

All of the steps of the methods described herein are computer implemented unless context indicates otherwise. In particular, any of the steps of the present method may be implemented by a computing device, optionally in operable communication with one or more sensors, other computing devices and/or user interfaces.

According to a fifth aspect, there is provided a system for monitoring a bioprocess comprising a cell culture in a bioreactor, the system including: at least one processor; and at least one non-transitory computer readable medium containing instructions that, when executed by the at least one processor, cause the at least one processor to perform operations comprising: obtaining the values of one or more state variables of a state space model and optionally one or more variables derived therefrom, at one or more maturities, the state space model comprising a kinetic growth model representing changes in the state of the cell culture and optionally a material balance model representing changes in the bulk concentration of one or more metabolites in the bioreactor; and predicting the value of one or more critical quality attributes of a product of the bioprocess using a machine learning model trained to predict the value of the one or more critical quality attributes based on input variables comprising values of the one or more state variables or variables derived therefrom, at one or more maturities. The system according to the present aspect may be configured to implement the method of any embodiment of the first aspect. In particular, the at least one non-transitory computer readable medium may contain instructions that, when executed by the at least one processor, cause the at least one processor to perform operations comprising any of the operations described in relation to the first aspect. The system may further comprise, in operable connection with the processor, one or more of: a user interface, optionally wherein the instructions further cause the processor to provide, to the user interface for outputting to a user, one or more of: the value of the one or more critical quality attributes or variables derived therefrom, the result of the comparing step, and a signal indicating that the bioprocess has been determined to operate normally or to not operate normally; one or more biomass sensor(s); one or more metabolite sensor(s); one or more process condition sensors; and one or more effector device(s).

According to a sixth aspect of the disclosure, there is provided a system for controlling a bioprocess, the system including: a system for monitoring a bioprocess according to the preceding aspect; and at least one effector device operably connected to the processor of the system for monitoring a bioprocess. The system according to the present aspect may be configured to implement the method of any embodiment of the second aspect. In particular, the at least one non-transitory computer readable medium may contain instructions that, when executed by the at least one processor, cause the at least one processor to perform operations comprising any of the operations described in relation to the second aspect.

According to a seventh aspect, there is provided a system for optimising a bioprocess comprising a cell culture in a bioreactor, the system including: at least one processor; and at least one non-transitory computer readable medium containing instructions that, when executed by the at least one processor, cause the at least one processor to perform operations comprising: performing the method of any embodiment of the first aspect using a first set of process conditions and at least a further set of process conditions (e.g. each being used to predict a state trajectory using the state space model, thereby obtaining values for one or more state variables associated with each set of process conditions); and determining whether the further set of process conditions is preferable to the first set of process conditions by comparing the critical quality attributes or values derived therefrom associated with the respective sets of process conditions. The system according to the present aspect may be configured to implement the method of any embodiment of the third aspect. In particular, the at least one non-transitory computer readable medium may contain instructions that, when executed by the at least one processor, cause the at least one processor to perform operations comprising any of the operations described in relation to the third aspect.

According to an eighth aspect, there is provided a system for providing a tool for monitoring and/or controlling and/or optimising a bioprocess comprising a cell culture in a bioreactor, the system including: at least one processor; and at least one non-transitory computer readable medium containing instructions that, when executed by the at least one processor, cause the at least one processor to perform operations comprising: obtaining the values of one or more state variables of a state space model and optionally one or more variables derived therefrom, and optionally the values of one or more process conditions, at one or more maturities for a plurality of bioprocesses, the state space model comprising a kinetic growth model representing changes in the state of the cell culture and optionally a material balance model representing changes in the bulk concentration of one or more metabolites in the bioreactor; and using the values obtained to train a machine learning model to predict the value of one or more critical quality attributes based on input variables comprising values of the one or more state variables or variables derived therefrom, at one or more maturities. The system according to the present aspect may be configured to implement the method of any embodiment of the fourth aspect. In particular, the at least one non-transitory computer readable medium may contain instructions that, when executed by the at least one processor, cause the at least one processor to perform operations comprising any of the operations described in relation to the fourth aspect.

According to a further aspect, there is provided a non-transitory computer readable medium comprising instructions that, when executed by at least one processor, cause the at least one processor to perform the method of any embodiment of any aspect described herein.

According to a further aspect, there is provided a computer program comprising code which, when the code is executed on a computer, causes the computer to perform the method of any embodiment of any aspect described herein.

Brief Description of the Drawings

Embodiments of the present disclosure will now be described by way of example with reference to the accompanying drawings in which:

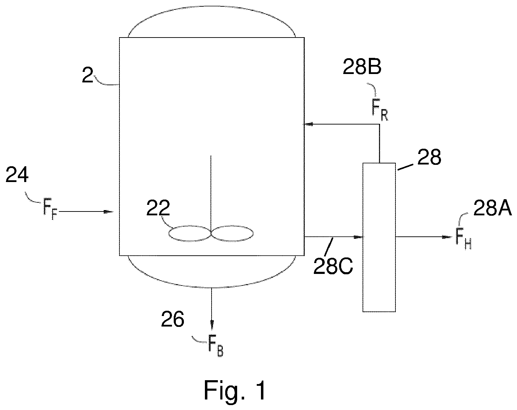

Figure 1 shows a simplified process diagram for a generic bioprocess according to embodiments of the present disclosure;

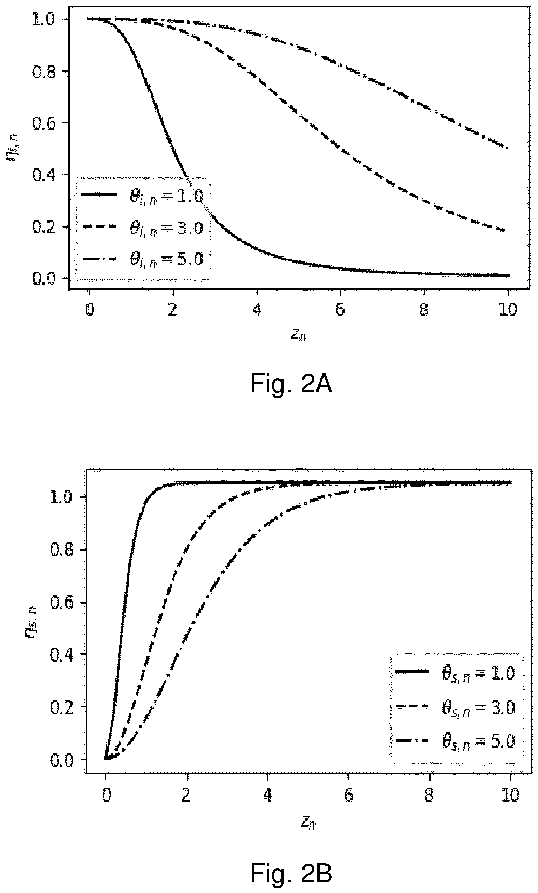

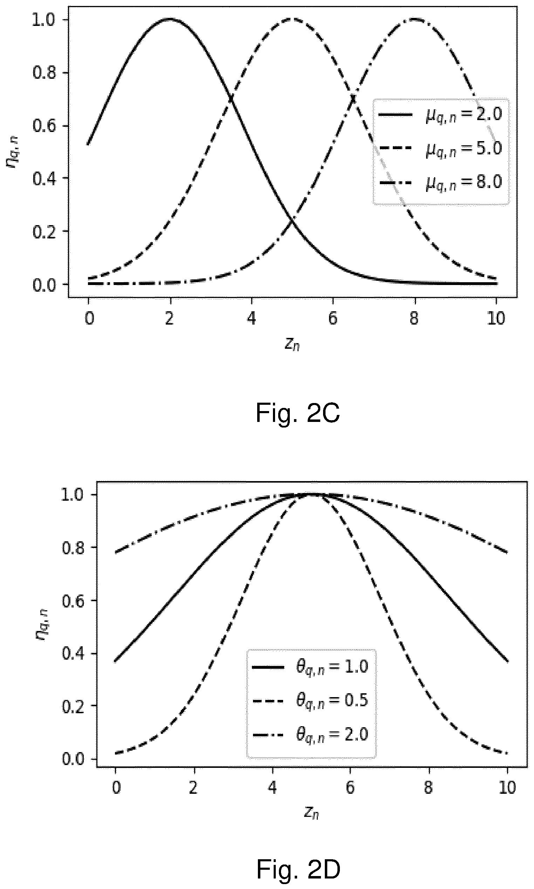

Figure 2 illustrates the impact of selected parameters on variables of a kinetic growth model according to embodiments of the present disclosure; Figure 2A is a plot showing the value of a correction factor (y-axis) capturing the effect of a growth inhibition variable on growth rate as a function of the value of the growth inhibiting variable (x-axis, e.g. concentration of a metabolite that has a cytostatic effect) for three exemplary values of the parameter $\theta_{i,n}$ which in this example represents the approximate value of the variable $z_n$ above which the variable starts to inhibit growth; Figure 2B is a plot showing the value of a correction factor (y-axis) capturing the effect of a substrate limiting variable on growth rate as a function of the value of the substrate limiting variable (x-axis, e.g. concentration of a metabolite such as a nutrient) for three exemplary values of the parameter $\theta_{s,n}$ which in this example represents the approximate value of the variable $z_n$ below which the variable starts to have a limiting effect on growth; Figures 2C and 2D are a plots showing the value of a correction factor (y-axis) capturing the effect of a variable that has a quadratic effect on growth as a function of the value of the of the quadratic effect variable (x-axis, e.g. temperature, pH) for three exemplary values of the parameter parameter $\theta_{q,n}$ at a fixed value of $\mu_{q,n}$ (D) and three exemplary values of the parameter $\mu_{q,n}$ at a fixed value of $\theta_{q,n}$ (C), where in these examples parameter $\theta_{q,n}$ represents the spread of the effect and parameter $\mu_{q,n}$ represents the value at which maximum growth occurs;

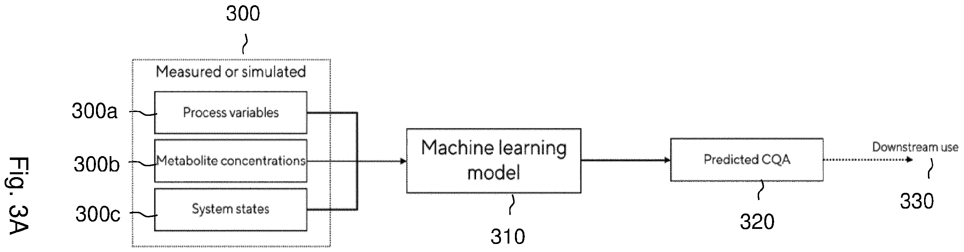

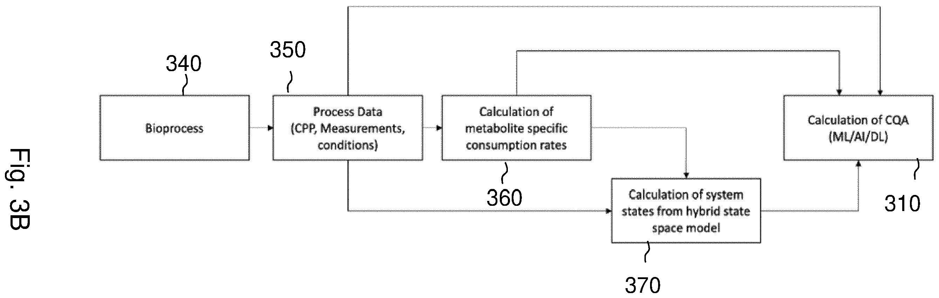

Figure 3 is a flowchart illustrating a method of predicting one or more metabolic condition variables according to embodiments of the present disclosure; in particular, the flowchart in A illustrates a model deployment procedure through which critical quality attributes of product in a bioprocess can be predicted, and the flowchart in B shows in more detail how the variables used to predict critical quality attributes of product in a bioprocess can be obtained;

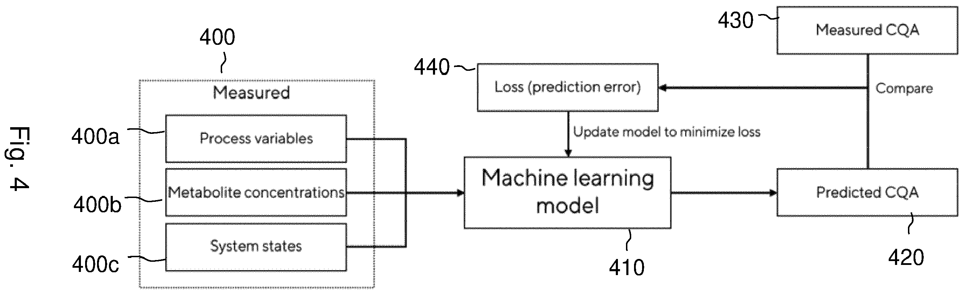

**Figure 4** is a flowchart illustrating a method of providing a tool according to embodiments of the present disclosure; in particular, the flowchart illustrates a model calibration procedure, which results in a calibrated model that can be used to predict one or more critical quality attributes of a product in a bioprocess;

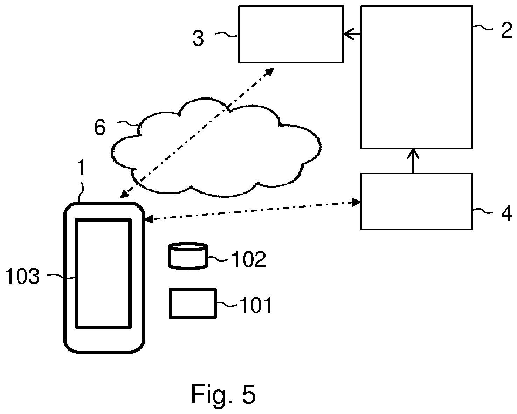

**Figure 5** illustrates schematically a system according to embodiments of the present disclosure;

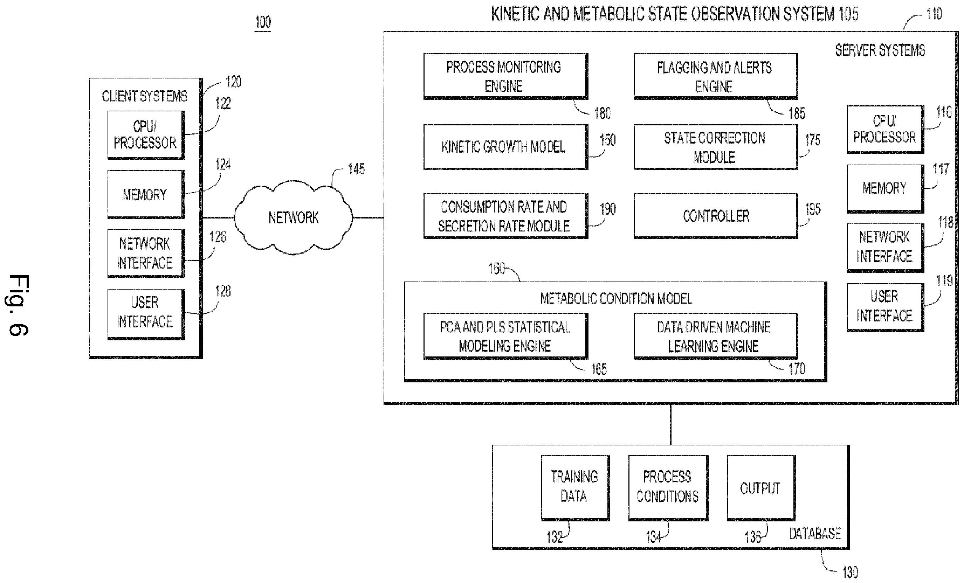

**Figure 6** illustrates schematically a computing architecture for implementing a method according to embodiments of the disclosure;

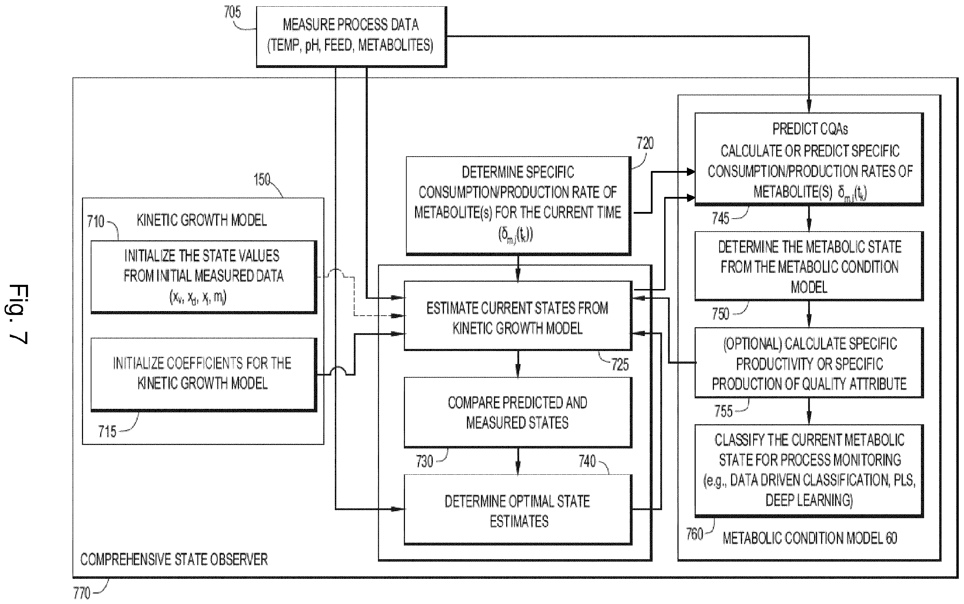

**Figure 7** shows a flowchart of an exemplary method of monitoring a bioprocess using a hybrid model as described herein;

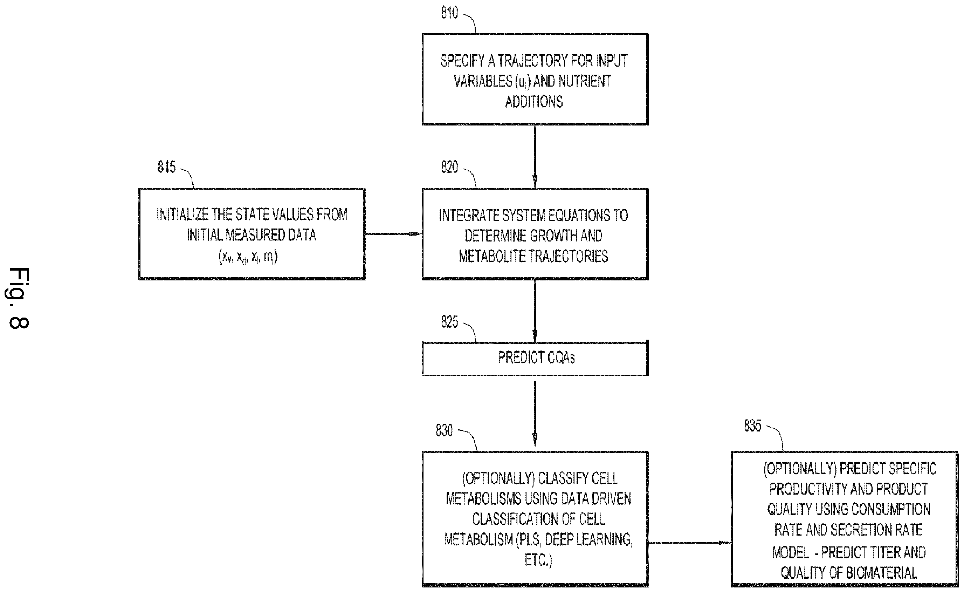

**Figure 8** shows a flowchart of an exemplary method of simulating a bioprocess using a hybrid model as described herein;

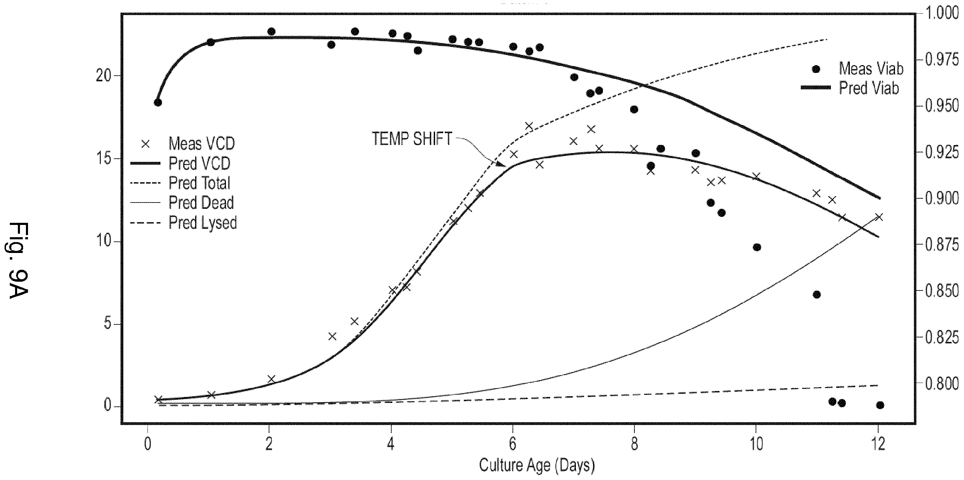

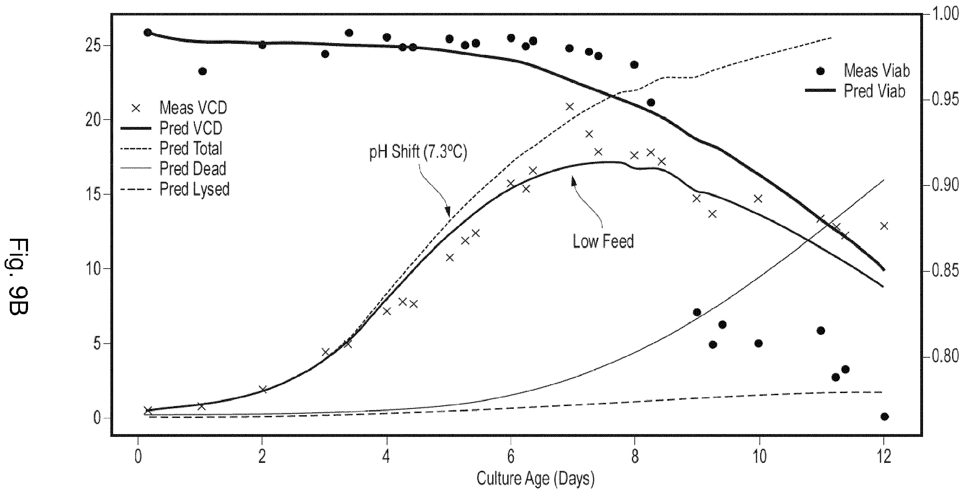

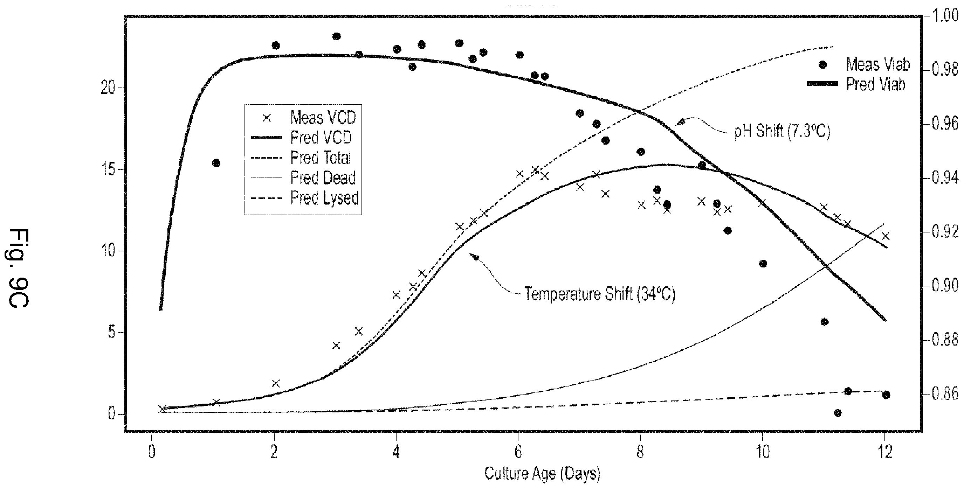

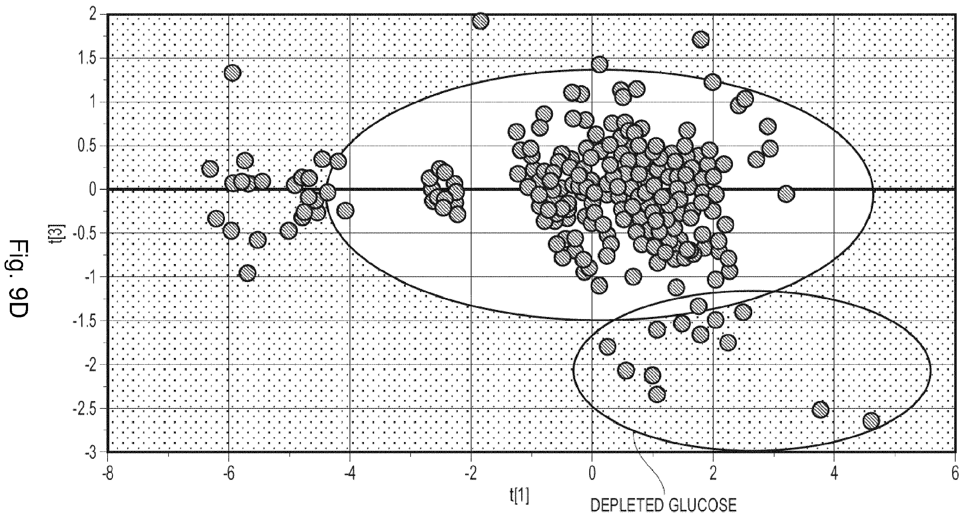

**Figure 9** shows examples of the use of a hybrid model as described herein; Figures 9A-C show examples of the impact of independent variable adjustments to the growth profile according to the present disclosure: for each figure, the predicted profiles for growth states are shown in solid lines and associated data for measured states is included for comparison; (A) example of the measured and predicted trajectories for a batch with a temperature shift (inhibits growth), (B) measured and predicted trajectories for a batch with a pH shift and a low feed rate (glucose depletion), (C) measured and predicted trajectories for a batch with a pH and temperature shift; Figure 9D show example outputs of a cell state classification by the hybrid model, according to the present disclosure; the figure shows a PCA score scatter plot providing observability of a metabolic disturbance caused by a depletion in glucose (e.g., predictions from the state observer using PCA); Glucose concentrations within the circle centered at the origin represent normal operation - glucose values outside of this range indicate increased risk of reduction in titer or product quality issues;

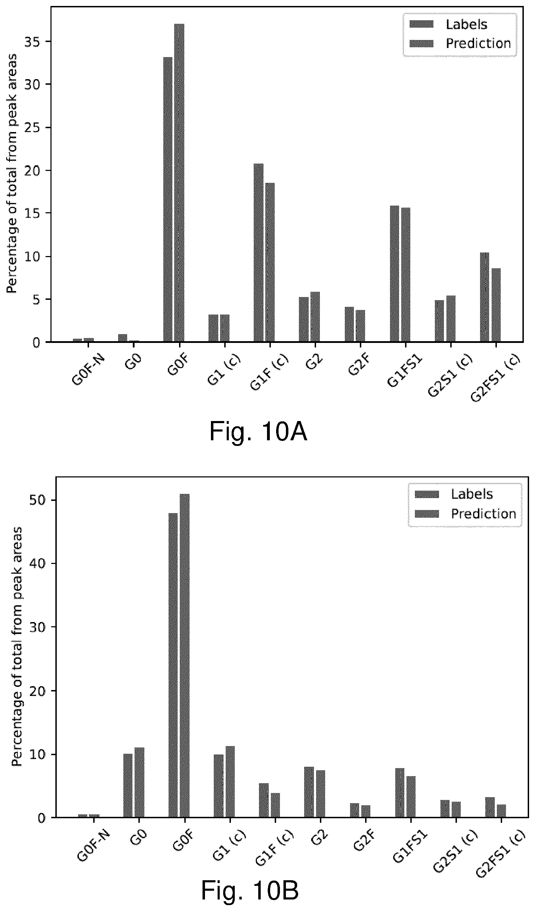

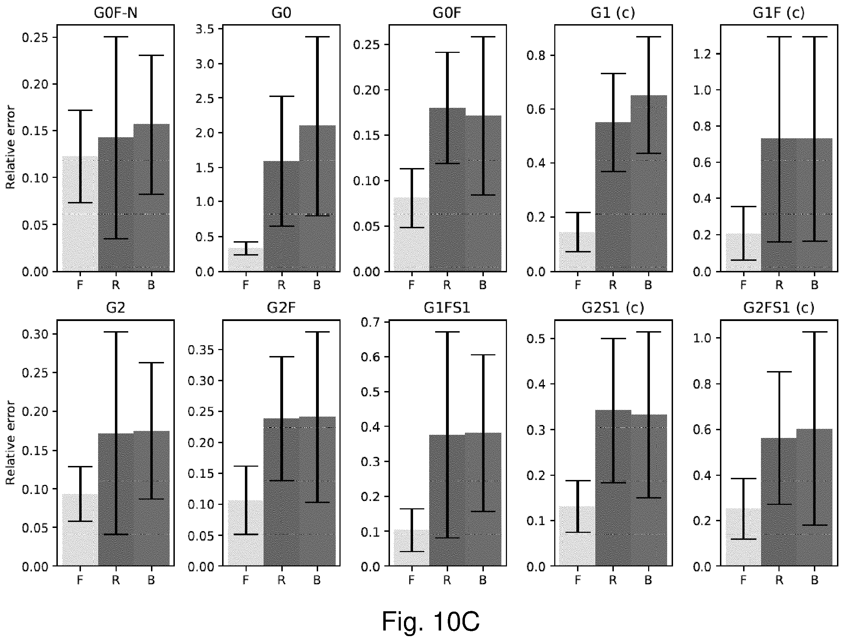

**Figure 10** shows the results of a bioprocess monitoring/control process according to an exemplary embodiment of the present disclosure (A-B) and comparisons between predictions obtained using the methods of the disclosure and comparative methods (C); (A-B) each plot compares, for a respective batch: for each glycosylation feature that is predicted, on the left the measured value for the respective feature in the respective batch, and on the right the predicted value obtained using the methods described herein; (C) each plot shows, for a respective glycosylation feature: (i) on the left (labelled 'F'), the mean square error (MSE) between (a) the value of the glycosylation feature predicted by a neural network using all available measured metabolite concentrations as input features and (b) the corresponding measured glycosylation feature; (ii) in the middle (labelled 'R'), the MSE between (a) the value of the glycosylation feature predicted by a neural network using a subset of the available measured metabolite concentrations as input features and (b) the corresponding measured glycosylation feature; and (iii) on the right (labelled 'B'), the MSE between (a) the value of the glycosylation feature calculated as the average across all batches except for the batch for which the feature is being predicted, and (b) the corresponding measured glycosylation feature. In every plot in (C), the height of the bar represents the average MSE over the 6 folds of a model cross-validation process, and the error bars indicate the 95% confidence interval around the average MSE.

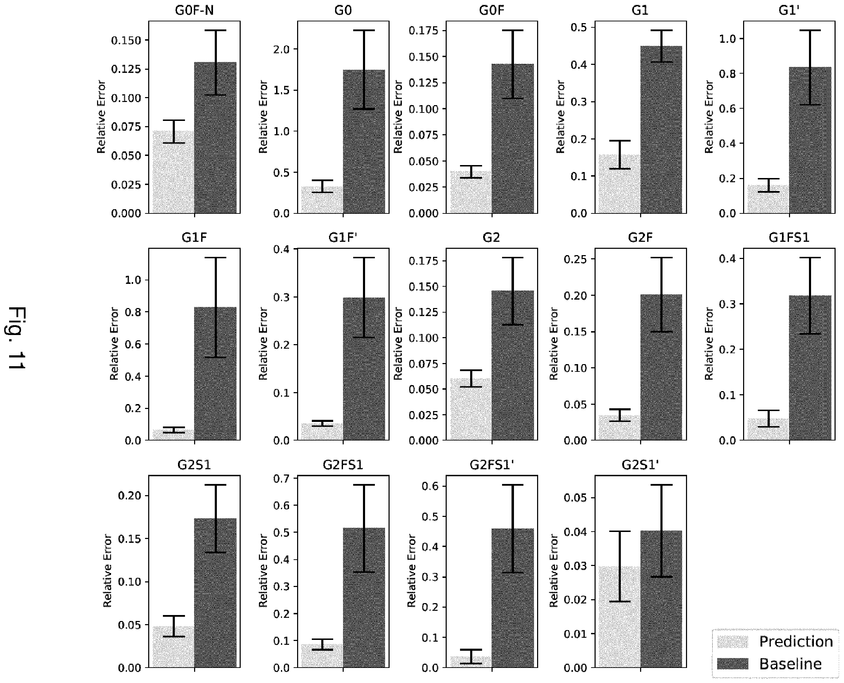

**Figure 11** shows the results of a bioprocess monitoring/control process according to an exemplary embodiment of the present disclosure: each plot compares, for a respective glycosylation feature that is being predicted: (i) on the left, the MSE between the glycosylation feature predicted by a neural network and the corresponding measured glycosylation feature (where the height of the bar represents the average MSE over the 6 folds of a network cross-validation process, and the error bars indicate the 95% confidence interval); and (ii) on the right, the MSE between the glycosylation feature calculated as averages across 5 batches (i.e. all batches other than the one for which the feature is being predicted) and the corresponding measured glycosylation feature (where the height of the bar represents the average MSE over the 6 batches and the error bars indicate the 95% confidence interval).

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.

As used herein, the terms "computer system" includes the hardware, software and data storage devices for embodying a system or carrying out a method according to the above described embodiments. For example, a computer system may comprise one or more processing units such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer.

The methods described herein may be provided as computer programs or as computer program products or computer readable media carrying a computer program which is arranged, when run on a computer, to perform the method(s) described herein. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

**Bioprocess**

As used herein, the term "bioprocess" (also referred to herein as "biomanufacturing process") refers to a process where biological components such as cells, parts thereof such as organelles or multicellular structures such as organoids or spheroids are maintained in a liquid medium in an artificial environment such as a bioreactor. In embodiments, the bioprocess refers to a cell culture. A bioprocess typically results in a product, which can include biomass and/or one or more compounds that are produced as a result of the activity of the biological components. A bioreactor can be a single use vessel or a reusable vessel in which a liquid medium suitable for carrying out a bioprocess can be contained. Example bioreactor systems suitable for bioprocesses are described in US 2016/0152936 and WO 2014/020327. For example, a bioreactor may be chosen from: advanced microbioreactors (such as e.g. Ambr® 250 or Ambr® 15 bioreactors from The Automation Partnership Ltd.), single use bioreactors (e.g. bag-based bioreactors such as Biostat® STR bioreactors from Sartorius Stedim Biotech GmbH), stainless steel bioreactors (such as e.g. 5 to 2,000 l bioreactors available in the Biostat® range from Sartorius Stedim Systems GmbH), etc. The present invention is applicable to any type of bioreactor and in particular to any vendor and any scale of bioreactor from benchtop systems to manufacturing scale systems.

A cell culture refers to a bioprocess whereby live cells are maintained in an artificial environment such as a bioreactor. The methods, tools and systems described herein are applicable to bioprocesses that use any types of cells that can be maintained in culture, whether eukaryotic or prokaryotic. The invention can in particular be used to monitor and/or control bioprocesses using cells types including but not limited to mammalian cells (such as Chinese hamster ovary (CHO) cells, human embryonic kidney (HEK) cells, Vero cells, etc.), non-mammalian animal cells (such as e.g. chicken embryo fibroblast (CEF) cells), insect cells (such as e.g. *D. melanogaster* cells, *B. moricells,* etc.), bacterial cells (such as e.g. E. *coli* cells), fungal cells (such as e.g. S. *cerevisiae* cells), and plant cells (such as e.g. *A. thaliana* cells). A bioprocess typically results in the production of a product, which can be the cells themselves (e.g. a cell population for use in further bioprocesses, a cell population for use in cell therapy, a cell population for use as a product such as a probiotic, feedstock, etc.), a macromolecule or macromolecular structure such as a protein, peptide, nucleic acid or viral particle (e.g. a monoclonal antibody, immunogenic protein or peptide, a viral or non-viral vector for gene therapy, enzymes such as e.g. for use in the food industry, for environmental applications such as water purification, decontamination, etc.), or a small molecule (e.g. alcohols, sugars, amino acids, etc.).

**Figure** 1 shows a simplified process diagram for a generic bioprocess. The bioprocess is implemented in the reactor 2, which in the embodiment shown is equipped with agitation means 22. Four flows (also referred to herein as "streams") are depicted, although depending on the particular situation any or all of these flows may be absent. A first flow 24 is a feed flow $F_F$ containing anything that is added to the culture in the bioreactor (typically this includes fresh medium, in which case the bioprocess may be referred to as a "fed-batch" process, "perfusion" process or "continuous" process), a second flow 26 is a bleed flow $F_B$ that has the same composition as the culture in the bioreactor, a third flow 28A is a harvest flow $F_H$ which is obtained through the processing of an auxiliary harvest stream 28C through a cell separation means 28 to produce the third (harvest) and fourth flow 28B, the fourth flow 28B being a recycle flow $F_R$ comprising the cells and any culture medium that has not been completely separated out in the cell separation means 28. In embodiments, the recycle flow $F_R$ may be ignored as considering only the harvest flow $F_H$ is sufficient to capture the flow that is effectively output from the bioreactor through the harvesting and cell separation process. Therefore, the reference to a harvest flow being present or absent may refer to the auxiliary harvest stream 28C (and derived harvest and recycle flows - $F_H$ and $F_R$) being present or absent. This harvest flow $F_H$ may be assumed to comprise medium that has the same composition as the medium in the reactor, but no or few cells. The feed, bleed and harvest flows ($F_F$, $F_B$, $F_H$ and $F_R$) may all be absent, in which case the bioprocess is referred to as an "unfed batch process" or simply "batch process". When a feed flow $F_F$ and a harvest flow $F_H$ are provided, the bioprocess may be referred to as a "perfusion" culture. When a feed flow $F_F$ and a bleed flow $F_B$ are provided such that the bioprocess is operated at (pseudo) steady-state (from a process condition point of view, i.e. maintaining in particular the volume of the culture constant), the bioprocess may be referred to as a "continuous" culture. When a feed flow $F_F$ is provided in the absence of output flows (bleed and harvest flows, $F_B$ and $F_H$), the bioprocess may be referred to as a "fed-batch" process. The present invention is applicable to all of the above operating modes.

A product of a bioprocess (also referred to herein as "biomaterial" or "target biologic") may include a metabolite, a cell, a desired protein, an antibody, an immunoglobulin, a toxin, one or more by-products, a target molecule, or any other type of molecule manufactured using a bioprocess. There may be more than one biomaterial of interest.

Products of a bioprocess may have one or more critical quality attributes (CQAs). As used herein, a "critical quality attribute" is any property of a product (including in particular any chemical, physical, biological and microbiological property) that can be defined and measured to characterise the quality of a product. The quality characteristics of a product (in terms of the values of one or more CQAs) may be defined to ensure that the safety and efficacy of a product is maintained within predetermined boundaries. According to the present invention, the value of one or more CQAs of a product (or a plurality of products) of a bioprocess are predict using machine learning, CQAs may include one or more of: the molecular structure of a small molecule or macromolecule (including in particular any of the primary, secondary and tertiary structure of a peptide or protein), the glycosylation profile of a protein or peptide (also referred to as "glycan profile" or "glycan analysis"), the activity of a product (such as e.g. enzymatic activity of a protein product, activity of a cellular product, etc.), the yield of a product (also referred to as "productivity" of the bioprocess, which may refer to the amount of product produced relative to the amount of an input to the bioprocess such as e.g. biomass - for example, the term "specific productivity" may refer to the amount of product produced on a per cell basis - or feed, provided or consumed), the presence or absence of one or more host cell protein(s), the purity of the product, and any physical, chemical, biological or microbiological property or characteristic that should be below or above an appropriate threshold, within an appropriate range or belong to an appropriate distribution, to ensure the desired quality of the product (where the appropriate threshold, range or distribution may be predetermined depending on the property/characteristic and the desired quality of the product). The glycosylation profile of a protein product such as a therapeutic protein may affect its safety, immunogenicity and half-life. It is affected by the environment of the cells through effects on their metabolism (availability of various enzymes and metabolites). The glycosylation profile of a protein product may be measured using any protocol and technology known in the art, such as by LC-MS (liquid chromatograph-mass spectrometry), or through capillary electrophoresis, for example using a platform such as the LabChip GX Touch Microchip-CE platform (PerkinElmer™).

A product may be associated with a "specification" which provides the values or ranges of values of one or more CQAs, that a product must comply within order to be considered acceptable. A product may be referred to as "in-specification" (or "according to specification", "within specification", etc.) if all of its CQAs comply with the specification, and "non-specification" (or "out-of-specification") otherwise. CQAs may be associated with a set of critical process parameters (CPPs), and ranges of values of the CPPs (optionally maturity-dependent ranges) that lead to acceptable CQAs. A bioprocess run (i.e. a particular instance of execution of a bioprocess) may be referred to as "normal" or "in-specification" if the CPPs are within the predetermined ranges that are believed to lead to acceptable CQAs, and "not normal" or ("out-of-specification") otherwise. Similarly, a bioprocess run may (typically retrospectively) be referred to as "normal" or "in-specification" if the bioprocess resulted in a product with acceptable CQAs (i.e. CQA values that comply with the specification), and "not normal" or ("out-of-specification") otherwise.

As used herein, the term "maturity" refers to a measure of completion of a bioprocess. Maturity is commonly captured in terms of time from the start of a bioprocess to the end of the bioprocess. Therefore, the term "maturity" or "bioprocess maturity" may refer to an amount of time from a reference time point (e.g. the start of the bioprocess). As such, the wording "as a function of bioprocess maturity" (e.g. quantifying a variable "as a function of bioprocess maturity") may in some embodiments refer to "a function of time" (e.g. quantifying a variable "as a function of time, e.g. since the start of the bioprocess"). Conversely, references to a time dependent variable (whether in the text or in equations) should be understood as applicable to any measure of maturity (including but not limited to time), unless the context indicates otherwise. In particular, any measure that increases monotonically as a function of time could be used, such as e.g. the amount of a desired product (or undesired by-product) accumulating in the medium or extracted since the start of a bioprocess, the integrated cell density, etc. may be used. The maturity may be expressed in terms of percentage (or other fractional measure) or as an absolute value that progresses to a value (typically a maximum or minimum value), at which point the bioprocess is considered complete.

As used herein, the term "process condition" refers to any measurable physico-chemical parameter of operation of a bioprocess. Process conditions may include in particular parameters of the culture medium and bioreactor operation, such as e.g. the pH, temperature, medium density, volumetric / mass flowrate of material in / out of the bioreactor, volume of the reactor, agitation rate, etc. Process conditions may also include measurements of the biomass in the bioreactor (e.g. total cell, viable cell density, etc.) or the quantity of a metabolite in a compartment as a whole (including in particular the quantity of a metabolite in any of the cell compartment, culture compartment including culture medium and cells, and the culture medium compartment) of the bioprocess. In particular, process conditions may include one or more process parameters selected from dissolved oxygen, dissolved CO2, pH, temperature, osmolality, agitation speed, agitation power, headspace gas composition (such as e.g. $CO_2$ pressure), flow rates (such as feed rate, bleed rate, harvest rate), feed medium composition and volume of the culture. Process conditions may instead or in addition include one or more biomass related metrics selected from viable cell density, total cell density, cell viability, dead cell density, and lysed cell density.

As used herein, the term "process output" refers to a value or set of values that quantify the desired outcome of a process. The desired outcome of a process may be the production of a product (biomaterial) such as the biomass itself or one or more metabolites, the degradation of one or more metabolites, or a combination of these. Thus, the process output may refer to an amount of biomaterial produced. This may be expressed as a total amount (for example, in grams or other units depending on the product), or a titer (amount per volume of culture in the bioreactor).

The term "metabolite" refers to any molecule that is consumed or produced by a cell in a bioprocess. Metabolites include in particular nutrients such as e.g. glucose, amino acids etc., by-products such as e.g. lactate and ammonia, desired products such as e.g. recombinant proteins or peptides, complex molecules that participate in biomass production such as e.g. lipids and nucleic acids, as well as any other molecules such as oxygen ($O_2$) that are consumed or produced by the cell. As the skilled person understands, depending on the particular situation, the same molecule may be considered a nutrient, a by-product or a desired product, and this may even change as a bioprocess is operated. However, all molecules that take part in cellular metabolism (whether as an input or output of reactions performed by the cellular machinery) are referred to herein as "metabolites". In particular, metabolites may include any suitable analyte, including but not limited to: amino acids (e.g., alanine, arginine, aspartic acid, asparagine, cysteine, cysteine, glutamic acid, glutamine, glycine, histidine, hydroxyproline, isoleucine, leucine, lysine, methionine, phenylalanine, proline, serine, threonine, tryptophan, tyrosine, valine, etc.), saccharides (e.g., fucose, galactose, glucose, glucose-1-phosphate, lactose, mannose, raffinose, sucrose, xylose, etc.), organic acids (e.g., acetic acid, butyric and 2-hydroxy- butyric acids, 3-hydroxybutyric acid, citric acid, formic acid, fumaric acid, isovaleric acid, lactic acid, maleic acid, propionic acid, pyruvic acid, succinic acid, etc.), other organic compounds (e.g., acetone, ethanol, pyroglutamic acid, etc.). References to one or more "metabolite concentrations" may include the concentration of one or more metabolites in the culture medium compartment. Unless the context indicates otherwise, metabolite concentrations typically refer to metabolite concentrations in the culture medium.

## Multivariate statistical models

The term "multivariate statistical model" refers to a mathematical model that aims to capture the relationships between multiple variables. Common multivariate statistical models include principal components analysis (PCA), partial least square regression (PLS) and orthogonal PLS (OPLS). The term "multivariate statistical analysis" refers to the building (including but not limited to the design and parameterisation) and/or using of a multivariate statistical model.

Principal component analysis (PCA) is used to identify a set of orthogonal axes (referred to as "principal components") that capture progressively smaller amounts of the variance in the data. The first principal component (PC1) is the direction (axis) that maximises the variance of the projection of a set of data onto the PC1 axis. The second principal component (PC2) is the direction (axis) that is orthogonal to PC1 and that maximises the variance of the projection of the data onto the PC1 and PC2 axes. The coordinates of a data point in the new space defined by one or more principal components is sometimes referred to as "scores". PCA functions as a dimensionality reduction approach, resulting in scores for each data point that capture the contribution of multiple underlying variables to the diversity in the data. PCA can be used on historical data about a set of runs of a bioprocess, to characterise and distinguish good (normal) and bad (not normal) process conditions. This enables the retrospective identification of when a historical batch has deviated outside of acceptable process conditions, and to interpret which of the individual process variables are most responsible for the deviation observed in the global process condition. This can then be used to investigate how to avoid such a deviation in the future.

PLS is a regression tool that identifies a linear regression model by projecting a set of predicted variables and corresponding observable variables onto a new space. In other words, PLS identifies the relationship between a matrix of predictors $X$ (dimension $mxn$) and a matrix of responses Y(dimension $mxp$) as:

$$X=TP^t+E \qquad (1)$$

$$Y = UQ^t + F \qquad (2)$$

where $T$ and $U$ are matrices of dimension $mxl$ that are, respectively, the $X$ score (projections of $X$ onto a new space of "latent variables") and the Yscores (projections of Yonto a new space); $P$ and $Q$ are orthogonal loading matrices (that define the new spaces and respectively have dimensions $nxl$ and $pxl$); and matrices $E$ and Fare error terms (both assumed to be IID - independent and identically distributed - random normal variables). The scores matrix T summarises the variation in the predictor variables in $X$, and the scores matrix U summarises variation in the responses in Y. The matrix P expresses the correlation between $X$ and U, and the matrix Q expresses the correlation between $Y$ and $T$. The decomposition of $X$ and $Y$ into a matrix of scores and corresponding loadings is performed so as to maximise the covariance between $T$ and U. OPLS is a variant of PLS where the variation in $X$ is separated into three parts: a predictive part that is correlated to Y ($TP$ as in the PLS model), an orthogonal part ($T_{orth}P_{orth}{}^t$ which captures systematic variability that is not correlated to $Y$) and a noise part ($E$ as in the PLS model - which captures residual variation). Partial least squares (PLS) and orthogonal PLS (OPLS) regression can be used to characterise the impact that the process condition has on a desired process output (concentration of product, quality attribute, etc.). This can be performed by fitting an (O)PLS model as described above, with $X$ including the one or more process variables that are believed to have an effect on the process output, and $Y$ including the corresponding measures of process output. This can be used to identify which process variables can be manipulated, and how they should be manipulated, to improve or control a desired output.

The Umetrics® software suite (Sartorius Stedim Data Analytics) further includes so called "batch evolution models" (BEM) which describe the time-series evolution of process conditions, referred to as the process 'path'. The process paths are obtained by fitting an (O)PLS model as described above, but where $X$ includes the one or more process variables that are believed to be of potential relevance, measured at multiple times (maturity values) over the evolution of the process, and $Y$ includes the corresponding maturity values. For example, a set of $n$ process variables may have been measured at m maturity values, and these $nxm$ values may be included as a coefficient in the matrix $X$. The corresponding matrix $Y$ is an $mx1$ matrix (i.e. a vector of length m) of maturity values. The T matrix therefore comprises scores values for each of the m maturity values and each of $l$ identified latent variables that describe the aspects of the process variables that are most correlated with maturity. By training the BEM on process paths that resulted in the desired product quality at the end of the process, a "golden BEM" can be defined that describes the range of process paths that are acceptable for a future batch (leading to CQAs within specifications), using the values of the scores in $T$. This enables the monitoring of batches to know that an ongoing batch is within specification. It also means that if an ongoing batch looks like it will deviate from the accepted range of paths, then an alarm can be raised to the operator to let them know that corrective action needs to be taken to prevent loss of product. Furthermore, the process measurements that are contributing to the deviation in process condition can be highlighted to the operator (by analysing the variables in $X$ that most contribute to the score in $T$ that has been observed to deviate from the expected course) to assist in diagnosing the problem and identifying the appropriate course of corrective action. This can all be done in real-time. Furthermore, operators only need to consider a small set of summary parameters during normal batch operation with the option to drill-down into specifics with an appropriate subject matter expert only when something is going wrong.

**Machine learning models**

According to the present invention, machine learning models are used to predict one or more critical quality attributes in a bioprocess. The term "machine learning model" refers to a mathematical model that has been trained to predict one or more output values based on input data, where training refers to the process of learning, using training data, the parameters of the mathematical model that result in a model that can predict outputs values with minimal error compared to comparative (known) values associated with the training data (where these comparative values are commonly referred to as "labels"). The term "machine learning algorithm" or "machine learning method" refers to an algorithm or method that trains and/or deploys a machine learning model. The machine learning models used in the present disclosure can be seen as regression models in that they capture the relationship between a dependent variable (the variables that are being predicted) and a set of independent variables (also referred to as predictors). Any machine learning regression model may be used according to the present invention. In the context of the present invention, a machine learning model is trained by using a learning algorithm to identify a function $F: u, m, s \rightarrow q_i$ where F is a function parameterised by a set of parameters $\theta$ such that:

$$q_i \approx \hat{q}_i = F(u, m, s | \theta) \qquad (3)$$

where $\hat{q}_i$ is a predicted CQA value, $q_i$ is the (true / observed) CQA value that the machine learning model aims to predict, and $\theta$ is a set of parameters identified as satisfying equation (4):

$$\theta = argmin_\theta L(q_i, \hat{q}_i) \qquad (4)$$

where L is a loss function that quantifies the model prediction error based on the observed and predicted CQA value. The specific choice of the function $F$, parameters $\theta$ and function $L$ as well as the specific algorithm used to find $\theta$ (learning algorithm) depends on the specific machine learning method used. Any method that satisfies the equations above can be used within the context of the present invention, including in particular any choice of loss function, model type and architecture. In embodiments, a machine learning model ($F(.|\theta)$) is a linear regression model. A linear regression model is a model of the form according to equation (5), which can also be written according to equation (5b):

$$Y = X\beta + \varepsilon \qquad (5)$$

$$y_i = \beta_0 + \beta_1 x_{i1} + .. \beta_p x_{ip} + \varepsilon_i \quad i = 1, ..., n \qquad (5b)$$

where Y is a vector with n elements yi (one for each dependent variable), X is a matrix with elements $x_{i1}..x_{ip}$ for each of the p predictor variables and each of the n dependent variables, and n elements of 1 for the intercept value, $\beta$ is a vector of p+1 parameters, and $\varepsilon$ is a vector of $n$ error terms (one for each of the dependent variables).

In embodiments, a machine learning model is a random forest regressor. Random forest regressors are described in e.g. Breiman, Leo. "Random forests." Machine learning 45.1 (2001): 5-32. A random forest regressor is a model that comprises an ensemble of decision trees and outputs a class that is the average prediction of the individual trees. Decision trees perform recursive partitioning of a feature space until each leaf (final partition sets) is associated with a single value of the target. Regression trees have leaves (predicted outcomes) that can be considered to form a set of continuous numbers. Random forest regressors are typically parameterized by finding an ensemble of shallow decision trees. In embodiments, a machine learning model is an artificial neural network (ANN, also referred to simply as "neural network" (NN)). ANNs are typically parameterized by a set of weights w that are applied to the inputs x of each of a plurality of connected neurons in order to obtain a weighted sum ($x^Tw$) that is fed to an activation function ($\Phi$) to produce the neuron's output. A commonly used activation function is the rectified linear activation function:

$$\Phi(z) = \max(0, z) \qquad (6)$$

where z is the linear combination $x^Tw$. The parameters of a neural network can be trained using a method called backpropagation (see e.g. Rumelhart, David E., Geoffrey E. Hinton, and Ronald J. Williams. "Learning representations by back-propagating errors." Nature 323.6088 (1986): 533-536) through which connection weights are adjusted to compensate for errors found in the learning process, in combination with a weight updating procedure such as stochastic gradient descent (see e.g. Kiefer, Jack, and Jacob Wolfowitz. "Stochastic estimation of the maximum of a regression function." The Annals of Mathematical Statistics 23.3 (1952): 462-466). The process can be repeated through a number of iterations over the training called an epoch. At each epoch, the whole training data or a subset thereof can be used (set through a hyperparameter referred to as "batch size"), such as e.g. a random subset, in order to introduce noise in the training and increase the likelihood that he model will generalize. The training data can be divided into a training and a validation set, where the validation set is used during the training to reduce the risk of overfitting to the training data (i.e. increase the likelihood that the model will generalize to new data). The validation loss may be computed in the same way as the training loss, using the validation set, and the validation loss may be used to decide when to stop the training to avoid overfitting (for example, when the validation loss stops decreasing). This may be referred to as "early stopping". Instead or in addition to this, a maximum number of epochs may be set as a hyperparameter of the training process.

Suitable loss functions for use in regression problems such as those described herein include the mean squared error, the mean absolute error and the Huber loss. Any of these can be used according to the present invention. The mean squared error (MSE) can be expressed as:

$$L(\cdot) = MSE(q_i, \hat{q}_i) = (q_i - q)^2 \qquad (7)$$

The mean absolute error (MAE) can be expressed as:

$$L(\cdot) = MAE(q_i, \hat{q}_i) = |q_i - \hat{q}_i| \qquad (8)$$

The MAE is believed to be more robust to outlier observations than the MSE. The Huber loss (see e.g. Huber, Peter J. "Robust estimation of a location parameter." Breakthroughs in statistics. Springer, New York, NY, 1992. 492-518) can be expressed as:

$$L\left(\delta_i, \widehat{\delta_i}|a\right) = \begin{cases} 0.5(q_i - \hat{q}_i)^2 & \text{for } |q_i - \hat{q}_i| < a \\ a|q_i - \hat{q}_i| - 0.5a^2 & \text{otherwise} \end{cases} \qquad (9)$$

where $\alpha$ is a parameter. The Huber loss is believed to be more robust to outliers than MSE, and strongly convex in the neighborhood of its minimum. However, MSE remains a very commonly used loss functions especially when a strong effect form outliers is not expected, as it can make optimization problems simpler to solve.

In embodiments, a machine learning model comprises an ensemble of models whose predictions are combined. Alternatively, a machine learning model may comprise a single model. In embodiments, a machine learning model may be trained to predict a single CQA. Alternatively, a machine learning model may be trained to jointly predict a plurality of CQAs. In such cases, the loss function used may be modified to be an (optionally weighted) average across all variables that are predicted, as described in equation (10):

$$L_M(q, \hat{q}) = \frac{1}{n} \sum_{i \in Q} \alpha_i L(q, \hat{q}_i) \qquad (10)$$

where $\alpha_i$ are optional weights that may be individually selected for each of the CQAs $i$, Q is the set of all CQAs, $q$ and $q$ are the vectors of actual and predicted values of CQAs. Optionally, the values of $q_i$ may be scaled prior to inclusion in the loss function (e.g. by normalizing so that the labels for all the jointly predicted variables have equal variance), for example to reduce the risk of some of the jointly predicted $\hat{q}_i$ dominating the training.

Similar considerations apply to machine learning models used to predict specific secretion / consumption rates of one or more metabolites ($\delta_i$), as will be described further below.

**Metabolic condition**

The terms "cell metabolic condition" (also referred to herein as "metabolic condition" or "cell condition") refer to the value of one or more variables that characterise the metabolism of cells in a bioprocess (i.e. metabolic activity of the cells in a bioprocess). This may include in particular the specific transport rate of a metabolite into / out of cells - also referred to herein as the specific consumption rate (e.g. when the metabolite is primarily consumed by the cells, such as a nutrient) or the specific secretion rate (when the metabolite is primarily produced by the cell; this can also be referred to as a specific production rate (SPR), particularly when the metabolite is a desired product), or any variable that is derived from a set of variables that includes one or more of these (e.g. using multivariate analysis techniques as will be described further below). For example, in some embodiments the metabolic condition of a cell culture can be expressed as *[metabolic condition]* = *f*($\delta$, *u*, *m*, *s*) where f is a function that transforms an original set of variables into one or more variables that capture the relationship between the original variables, $\delta$ are the specific secretion / consumption rates of one or more metabolites, *u* are process variables such as temperature, pH, etc., *m* are metabolite concentrations, and s are variables representing the state of the cell culture system. A state of a cell culture system can be a variable that is modelled by a system of differential equations that together form a kinetic growth model, as will be explained further below. For example, state variables of the cell culture system can include one or more of viable cell density, lysed cell density, total cell density, dead cell density, or related variables such as cell viability. These can be obtained using a state space model as will be explained further below. Function f can be obtained, for example, using PCA, PLS or OPLS. The cell consumption or secretion/production rate of a metabolite (i.e. the specific transport rate of the metabolite into/out of cells) and the concentration of the metabolite inside a cell (which may be expressed in terms of units of mass per volume or per cell) may be considered to represent metabolic variables (in that they characterise the metabolism of the cell). Note that a metabolite may be transported both into and out of cells (e.g. a metabolite may be both consumed and produced), in which case the specific transport rate quantifies the combined effect of the movement in both directions. In other words, the specific transport rate of a metabolite quantifies the net amount of metabolite transported between the cells and the liquid medium (e.g. as a change in the amount of metabolite in the medium, reflecting both movement from the medium to the cells and vice versa). Further, the concentration of the same metabolite in a compartment of the bioprocess (e.g. in the bulk composition or the liquid medium, which may be expressed in terms of units of mass per volume) may be considered to represent a process variable (in that it characterises a macroscopic process variable). For example, the concentration of oxygen or glucose in the liquid medium (e.g. in mass/volume) may be considered to be a process variable (also referred to herein as "process parameter"), describing the process at a macroscopic level (process condition), whereas the concentration of oxygen or glucose in the cells (e.g. in mass/cell) may be considered to be a metabolic variable, describing the metabolic condition of the cells.

### State space model - Kinetic growth model

The term "kinetic growth model" refers to any model that captures the kinetic of a cell population in a bioprocess. Accordingly, a kinetic growth model may be used to monitor or simulate the number of live cells (and other culture-related parameters) in a bioreactor and to make predictions about the number of cells in the bioreactor at a future point in time. For example, a kinetic growth model may include one or more differential equations that model the maturity-dependent (typically, time-dependent) behaviour of one or more cell population variables. Cell population variables are a specific type of process conditions that characterise the viable, dead, lysed and/or total cell population in a bioprocess. Typical cell population variables include the viable cell density (VCD), dead cell density, and lysed cell density, which respectively capture the concentration of viable, dead and lysed cells in a bioreactor. In embodiments, a kinetic growth model uses the Monod equation to capture cell growth rates as a function of the concentration of a limiting nutrient. One example of a kinetic growth model is provided in equations (11) to (14) below, which describe respectively changes in the viable cell density $x_v$, the dead cell density $x_d$, the total cell density $x_t$ and the lysed cell density $x_l$ as a function of time/maturity:

$$\frac{dx_v}{dt} = \left(\mu_{eff} - \mu_d - \frac{F_b}{V}\right) x_v \qquad (11)$$

$$\frac{dx_d}{dt} = \mu_d x_v - \left(k_l + \frac{F_b}{V}\right) x_d \qquad (12)$$

$$\frac{dx_l}{dt} = k_l x_d - \frac{F_h + F_b}{V} x_l \qquad (13)$$

$$x_t = x_v + x_d + x_l \qquad (14)$$

In equations (11)-(14), $F_b$ and $F_h$ are the bleed and harvest rates, respectively (see above and Figure 1), V is the reactor volume, $\mu_{eff}$, $\mu_d$, and $k_l$ are the effective growth, effective death, and lysing rates, respectively. Equation (11) captures the following assumptions: (i) live cells are formed from live cells at the effective growth rate $\mu_{eff}$; (ii) live cells can exit the reactor through the bleed stream $F_b$ (if present); (iii) live cells can be transformed into dead cells at the effective death rate $\mu_d$; and (iv) no live cells exit the reactor through the harvest stream $F_h$ (in other words, where a harvest stream is present it includes a perfect cell retention filter - which may be a valid assumption when any cells exiting the bioreactor through the harvest stream represent a negligible amount). The calculation of the effective growth rate is critical to the functioning of this model and is presented in detail below.
Equation (12) captures the following assumptions: (i) dead cells are formed from live cells at the effective death rate $\mu_d$; (ii) dead cells are converted into lysed cells through a first-order process with rate $k_l$; (iii) dead cells can exit the reactor through the bleed stream $F_b$ (if present); and (iv) no dead cells exit the reactor through the harvest stream $F_h$. The calculation of the effective death rate is presented in detail below. Equation (13) captures the following assumptions: (i) lysed cells are formed from dead cells at rate $k_l$; (ii) lysed cells can exit the reactor through the bleed stream $F_b$ (if present); and (iv) lysed cells can exit the reactor through the harvest stream $F_h$ (if present). Equation (14) captures the assumption that cells are either viable, dead or lysed. This can be used to calculate one of the variables from the others, for example lysed cells (which typically cannot be measured directly), by closing the balance on living and dead cells.
In embodiments, the death process (captured through the parameter $\mu_d$) can be modelled as resulting from a combination of a base death rate and a toxicity factor, for example as provided in equation (15) below:

$$\mu_d = k_d + k_t \phi_t \qquad (15)$$

In equation (15), $k_d$ is the primary (base) death rate, $k_t$ is the "toxicity rate" and $\phi_t$ is a measure of toxicity. The variable $\phi_t$ may represent the concentration of one or more components that have a toxic effect on cells. In embodiments, $\phi_t$ is

assumed to be equal to the concentration of lysed cells $x_l$ (i.e. $\phi_t = x_l$). In other embodiments, $\phi_t$ is assumed to be equal to the concentration of an unknown biomaterial that accumulates in the culture as a result of cell growth and inhibits cells growth and/or is toxic to the cells, which is designated as $\phi_b$ (such that $\phi_t = \phi_b$). The evolution of this variable can be captured for example using equation (16) below:

$$\frac{d\phi_b}{dt} = x_v - \frac{F_h + F_b}{V}\phi_b \quad (16)$$

which assumes that the unknown product is produced at a rate that is proportional to the viable cell density $x_v$ and that the product can exit the bioreactor through the bleed and harvest streams ($F_b$, $F_h$), if present.

In embodiments, the growth process (captured through the parameter $\mu_{eff}$) is modelled as the product of a growth rate under ideal conditions $\mu_{max}$ (where growth rate is assumed to be maximised), and one or more factors that describe the influence of other system variables on growth. These factors may in some cases take one of three functional forms: substrate limited $\eta_s$, quadratic $\eta_q$ or inhibitory $\eta_i$. The relationship between these factors and the resulting effective growth rate can be captured in equation (17):

$$\mu_{eff} = \mu_{max}\eta_s\eta_q\eta_i \quad (17)$$

where the correcting factor $\eta_s$ captures the contribution of $N_s$ substrate limiting variables, $\eta_q$ captures the contribution of $N_q$ quadratic influencing variables and $\eta_i$ captures the contribution of $N_i$ inhibitory variables. $N_s$ can be equal to 0 (no substrate limiting variable), 1 (one substrate limiting variable), or any natural number. $N_q$ can be equal to 0 (no quadratic influencing variable), 1 (one quadratic influencing variable), or any natural number. $N_i$ can be equal to 0 (no inhibitory variable), 1 (one inhibitory variable), or any natural number. The correction factors above can be calculated as products of a plurality of correcting factors that each capture the contribution of a substrate limiting / quadratic influencing / inhibitory variable, as shown in equations (18)-(20) below:

$$\eta_s = \prod_{n=1}^{N_s}(\eta_{s,n}) \quad (18)$$

$$\eta_q = \prod_{n=1}^{N_q}(\eta_{q,n}) \quad (19)$$

$$\eta_i = \prod_{n=1}^{N_i}(\eta_{i,n}) \quad (20)$$

In embodiments, inhibitory effects are captured using correction factors of the form provided by equation (21) below:

$$\eta_{i,n} = \frac{1}{\left(\frac{z_n}{\theta_{i,n}}\right)^3 + 1} \quad (21)$$

where $z_n$ is the concentration of a substance that has a growth inhibitory effect, and $\theta_{i,n}$ is a parameter that represents the level of $z_n$ above which inhibition occurs. **Figure 2A** shows an example of the value of a growth inhibition factor as a function of the concentration of the growth inhibitory substance whose effect is modelled by the correction factor, for different values of the parameter $\theta_{i,n}$. The variable $\phi_t$ in equations (15) and (16) above may be seen as a special case of an inhibitory effect where the concentration of the inhibitory substance depends on the viable cell density (e.g. because the inhibitory substance is produced by or as a result of the presence of the cells). The variable $\phi_t$ can also sometimes be modelled using a formulation as provided in equation (21). Therefore, equation (17) can in some embodiments be written as:

$$\mu_{eff} = \mu_{max}\eta_s\eta_q\eta_i \frac{1}{\left(\frac{\phi_t}{\theta_t}\right)^3 + 1} \quad (22)$$

where $\theta_t$ (or $\theta_b$ as the case may be) is a coefficient representing the inhibition in growth from accumulation of the

biomaterial $\varnothing_t$ (where $\varnothing_t$ can be e.g. equal to $x_i$ or $\varnothing_b$). As the skilled person understands, in some embodiments there may be more than one substance $\varnothing_b$, each of which can be modelled using a respective term in equations (17) and (22) and a respective equation (16). Examples of substances that have a growth inhibitory effect and that produced by or due to the presence of the cells in the culture (i.e. substances $\varnothing_b$) include toxic by-products such as e.g. ammonia. Examples of substances that have a growth inhibitory effect that is not necessarily related to the viable cell density include substances that are included in the culture medium to have a desired effect but that may also have a (ideally slight) growth inhibitory effect on the cell culture (e.g. antibiotics).

In embodiments, substrate limiting effects are captured using correction factors of the form provided by equation (23) below:

$$\eta_{s,n} = tanh\left(2\,\frac{z_n}{\theta_{s,n}}\right)^2 \quad (23)$$

where $\theta_{s,n}$ is a parameter which represents the approximate level below which the variable $z_n$ (substrate that is growth limiting) starts to have a limiting effect on growth. **Figure 2B** shows an example of the value of a substrate limitation factor as a function of the concentration of the limiting substrate whose effect is modelled by the correction factor, for different values of the parameter $\theta_{s,n}$. In equation (23) the factor 2 is used to give the parameter $\theta_{s,n}$ an intuitive biological meaning as the approximate concentration $z_n$ below which a limiting effect can be seen (such as e.g. a value such that the inhibitory function crosses the -0.95 threshold when the value of falls below that of $\theta_{s,n}$). Any other value can be used for this factor, resulting in the same behavior with the effect that the parameter $\theta_{s,n}$ is adjusted accordingly and no longer has the same intuitive interpretation. In particular, this factor can be omitted entirely (i.e. set to 1). Similarly, in equations (21) and (22), the term that is cubed (i.e. ($z_n$ /$\theta_{i,n}$)) may comprise a coefficient that gives the parameter $\theta_{i,n}$ an intuitive biological meaning as the approximate concentration above which a limiting effect can be seen (such as e.g. a value such that the inhibitory function crosses the -0.95 threshold when the value of reaches that of $\theta_{i,n}$, for example 0.37). Examples of substances that have a substrate limiting effect include nutrients such as glucose, amino acids, etc.

In embodiments, quadratic effects are captured using correction factors of the form provided by equation (24) below:

$$\eta_{q,n} = exp\left[-\frac{1}{25}\left(\frac{z_n - \mu_{q,n}}{\theta_{q,n}}\right)^2\right] \quad (24)$$

where $u_{q,n}$ is a parameter that represents the target value (i.e. the value at which maximum growth occurs) and $\theta_{q,n}$ is a parameter that represents the "spread"' of the effect. The factor 1/25 used to give the parameter $\theta_{q,n}$ an intuitive meaning where a value of $\theta_{q,n}$ = 1 means that the quadratic effect crosses a 95% threshold at $\pm 1$ around the target value $\mu_{q,n}$. Any other value can be used for this factor, resulting in the same behavior with the effect that the parameter $\theta_{q,n}$ is adjusted accordingly and no longer has the same intuitive interpretation. In particular, this factor can be omitted entirely (i.e. set to 1).The use of factors that provide an intuitive interpretation of parameters such as $\theta_{s,n}$ and $\theta_{q,n}$ may be advantageous in that it may make it easier for a user to set these parameters (using e.g. biological knowledge or assumptions) or realistic boundaries for these parameters. **Figure 2C** shows an example of the value of a quadratic effect factor as a function of the concentration of the substance whose effect is modelled by the correction factor, for different values of the parameter $\mu_{q,n}$ with a fixed value of $\theta_{q,n}$ =1. **Figure 2D** shows an example of the value of a quadratic effect factor as a function of the concentration of the substance whose effect is modelled by the correction factor, for different values of the parameter $\theta_{q,n}$ with a fixed value of $\mu_{q,n}$=5.

As the skilled person understands, other equations can be used instead or in addition to the above to model cell population variables and factors that affect these variables. These equations together may form a state space model (sometimes also referred to as a state observer). Indeed, they describe the evolution of the state of the cell culture (also referred to herein as "cell state") in terms of a series of state variables $x_v$, $x_d$, $x_t$, $x_i$ as a function of inputs to the system including e.g. the concentration of various substances that influence the cell state.

This state space model (comprising a kinetic growth model) can be augmented to include one or more equations that capture the evolution of the concentration of one or more metabolites in the bulk culture (including in particular nutrients, by-products and the desired product), as described below (such a e.g. equation (26) and equivalents, or any other such equation capturing the material balance in equation (25)).

**State space model - Bulk concentration of metabolites**

Equations that represent the evolution of the bulk concentration of metabolites (including in particular nutrients, by-products and the desired product) can be expressed based on a material balance equation such as equation (25) below:

$$[total\ change\ of\ metabolite\ amount\ in\ reactor] =$$
$$[total\ flow\ of\ metabolite\ into\ reactor] - [total\ flow\ of\ metabolite\ out\ of\ reactor] +$$
$$[secretion\ of\ metabolite\ by\ cells\ in\ reactor] -$$
$$[consumption\ of\ metabolite\ by\ cells\ in\ reactor\ ] \qquad (25)$$

Equation (25) expresses in mathematical form the conservation of mass in the system (reactor), for a metabolite under investigation. At every time point t, equation (25) must be satisfied. The flows of metabolite in equation (25) may be expressed as mass flows or molar flows (as the latter can be converted to the former and vice-versa using molar mass, such that the conservation of mass expressed in the equation is verified regardless of the units chosen), and the skilled person would be able to convert one into the other. As such, references to mass flows are intended to encompass the use of the corresponding molar flows with corresponding adjustments for consistency of units within an equation. Similarly, references to concentrations may refer to the mass or molar concentrations. The flow of the metabolite into the bioreactor depends on the value of the feed flow $F_F$ and the concentration of the metabolite in this flow (if this flow is present, i.e. $F_F \neq 0$). The flow of the metabolite out of the bioreactor depends on the value of the harvest flow $F_H$ (if present) and the value of the bleed flow $F_B$ (if present), and the concentration of the metabolite in these respective flows. The consumption and secretion of a metabolite by the cells in the bioreactor depends on the viable cell density in the reactor and on a variable called the "specific transport rate" (sometimes also referred to as "metabolic rate"), which can also be referred to as ""specific consumption rate" (typically, if it is negative, i.e. the metabolite is being consumed by the cells) or the "specific secretion/production rate" (typically, if it is positive, i.e. the metabolite is being produced by the cells) of the metabolite by the cells. Therefore, the material balance described in equation (25) can be written for a general system (e.g. as illustrated on Figure 1), for metabolite $i$ as equation (26) below:

$$\frac{d\,(m_i)}{dt} \quad = \quad \frac{F_F}{V} * m_{F,i} \quad - \quad \frac{F_H}{V} * m_{H,i} \quad - \quad \frac{F_B}{V} * m_{B,i} \quad + \quad \delta_{m,i} * x_v \quad (26)$$

where $\delta_{m,i}$ is the specific transport rate of metabolite i by the cells in the culture, $m_i$ is the concentration of metabolite i in the reactor, V is the volume of the culture in the bioreactor, $m_{F,i}$ is the concentration of metabolite i in the feed flow, $m_{H,i}$ is the concentration of metabolite i in the harvest flow, $m_{B,i}$ is the concentration of metabolite i in the bleed flow, $x_v$ is the viable cell density in the reactor, and $F_F$, $F_H$ and $F_B$ are the volumetric feed, harvest and bleed flow rates, respectively (although mass flow rates can equally be used with the appropriate coefficients for densities of the respective flows). In

embodiments, the term $\delta_{m,i} * x_v$ can be replaced by a term $\delta_{m,i} * x_v * (\frac{m_i}{m_i+\varepsilon})$ where ε is a constant that is chosen to ensure that the values of $m_i$ that are below the detection limit for the metabolite do not cause errors in the estimation of specific transport rates. Typically, ε is chosen to be approximately equal to the detection limit for the metabolite (e.g. it can be chosen as 0.05 where the metabolite concentration is standardised).

Equation (25) assumes that the harvest flow 28A contains the only material leaving the system through the auxiliary harvest flow 28C (i.e. the auxiliary harvest flow and the return flow do not need to be included in the model as the metabolite only leaves the system through the harvest flow), and that the cell separation device 28 functions such that the harvest flow 28A can be assumed to contain no cells. Equation (26) can be adapted to include an auxiliary harvest flow 28C (and corresponding $m_{A,i}$ and density $\rho_A$ if a mass flow rate is used) and a return flow 28B (and corresponding $m_{R,i}$ and $\rho_R$). Further, equation (26) can be amended to model the removal of some cells through the harvest flow. In other words, additional terms can be added to equation (26) and some can be removed depending on the set-up of the bioprocess and the assumptions made. Examples of a few common bioprocess set ups and their corresponding simplified equations are provided below.

As the skilled person understand, the general equation in (25) can be expressed differently depending on the operating mode (e.g. fed-batch, unfed-batch, etc.) and the assumptions made (e.g. variable volume, variable concentration in the various flows and in the bioreactor, etc.). The skilled person would be able to express and solve equation (25) accordingly, in light of the teaching provided herein. Further, whether a particular assumption is reasonable may depend on the situation, and the skilled person would be able to verify whether this is the case using well known techniques. For example, the skilled person would be able to verify whether the volume of a culture is constant (e.g. by examining the amounts of material flowing in and out of the bioreactor or using a level sensor), whether the medium density is constant (e.g. using a hydrometer), whether the concentration of one or more metabolites is the same in one or more compartments and/or flows (e.g. using one or more metabolite sensors to measure metabolite concentration separately in these compartments and/or flows), etc. The skilled person would further be aware that a particular assumption may be reasonable in one case but not in another. For example, the concentration of a small molecule metabolite in the culture medium may be likely to be the same in the bioreactor and in the out flows (harvest and/or bleed flows), whereas the concentration of a macromolecule may differ between the bioreactor and one or more of the out flows if the macromolecule is likely to be held up on filters or other structures.

Importantly, such a model (referred to herein as "material balance model" or "bulk metabolite concentration model") captures the relationships between extracellular fluxes of metabolites and the concentration of the metabolites in the culture medium. Thus, they provide information about the metabolism of the cells from an external point of view (i.e. in terms of how it affects the composition of the bulk fluid in the bioreactor). The model does model intracellular metabolic fluxes, which are captured using metabolic models that model fluxes along reactions that together form the cell's metabolic network, and which are significantly more complex (and thus more difficult to solve and requiring extensive data and/or assumptions to parameterise). As such the models used herein advantageously provide information about the metabolic state of the cells while remaining relatively simple to solve and parameterised, allowing to obtain important system state variables such as the specific transport rates of metabolites (as will be explained further below) in a simple and efficient manner. These important system state variables can then be used to predict CQAs as further described herein.

**Calculation of specific transport rates using material balance equations**

The specific transport rate (i.e. specific consumption or production/secretion rate, depending on whether the rate has a positive or a negative sign as seen from the perspective of the reactor) of metabolite i (denoted $\delta_{m,i}$ above) is an important variable that captures aspects of the metabolic condition of the cells in the bioprocess. Further, where the metabolite is a desired product, its specific secretion/production rate provides a useful indication of the productivity of the cell culture. These variables $\delta_{m,i}$ (one for each metabolite of interest) can be calculated at a particular time point using material balance equations as will be described below, together with measurements of the concentration of the respective metabolite and the viable cell density at two successive time points.

The specific transport (consumption / secretion) rate of a metabolite at a particular time point can be calculated based on equation (26) (or its simplified variants as explained below), using known (i.e. measured or simulated) values of the metabolite concentration and the viable cell density, and using a first order finite difference approximation. For example, using such an approximation, equation (26) can be solved for $\delta_{m,i}$ at time k (denoted $\delta_{m,i}(t_k)$ or $\delta_{m,i,k}$) according to equation (27):

$$\delta_{m,i}(t_k) = \frac{1}{IVCD_k V_k}\left[\frac{(F_{F,k+1}+F_{F,k})}{2}m_{F,i}(t_{k+1}-t_k) - \frac{(F_{B,k+1}+F_{B,k})}{2}m_{B,i}(t_{k+1}-t_k) - \right.$$

$$\left.\frac{(F_{H,k+1}+F_{H,k})}{2}m_{H,i}(t_{k+1}-t_k) - V_k\big(m_{i,k+1}-m_{i,k+1}\big)\right] \quad (27)$$

where the subscripts k and $k+1$ indicate a value at the $k^{th}$ and $k+1^{th}$ time points where values for the metabolite concentration and viable cell density are available, and $IVCD_k$ is the integrated viable cell density between the time points k and $k+1$. Note that the consumption rate at the $k^{th}$ observation is forward looking, meaning that it represents the consumption rate for the time interval $k \rightarrow k+1$.

The specific transport (consumption / secretion) rates of one or more metabolites of interest can be used as variables of a metabolic condition model that classifies the metabolic condition of the cells e.g., into an optimal or suboptimal state or category for biomaterial production, as will be explained further below.

For a perfusion culture (where a feed flow $F_F$, a bleed flow $F_B$ and a harvest flow $F_H$ are present), equation (26) can be simplified by making a few assumptions. For example, assuming that the metabolite concentration is the same everywhere in the culture medium in the bioreactor, and hence also in the harvest flow and in the bleed flow (in other words, assuming that concentration gradients within the reactor are negligible such that $m_{B,i} = m_{H,i} = m_i$), and that the number of cells lost in the bleed and harvest flows is negligible, equation (26) can be written as:

$$\frac{d(m_i)}{dt} = \frac{F_F}{V}*m_{F,i} - \frac{(F_H+F_B)}{V}*m_i + \delta_{m,i}*(x_v) \quad (26a)$$

Assuming further that the volume of the culture is constant (i.e. $F_F = F_H + F_B$), that the flows are constant, and using a first-order finite difference approximation for the derivative, equation (26a) can be resolved for the metabolite specific consumption/secretion rate at time $t_k$ as:

$$\delta_{m,i}(t_k) = \frac{V*(m_{i,k+1} - m_{i,k}) - F_F*m_{F,i,k}*(t_{k+1} - t_k) + F_F*m_{i,k}*(t_{k+1} - t_k)}{V*IVCD_k} \qquad (27a).$$

For a fed-batch culture (where a feed flow is present, but no bleed flow or harvest flow are present, i.e. $F_H = F_B = 0$), equation (26) can be written as:.

$$\frac{d\,(m_i)}{dt} \quad = \quad \frac{F_F}{V} * m_{F,i} \quad + \quad \delta_{m,i} * (x_v) \quad (26b)$$

Using a first-order finite difference approximation for the derivative, equation (26b) can be resolved for the metabolite specific consumption/secretion rate at time $t_k$ as:

$$\delta_{m,i}(t_k) = \frac{F_F*m_{F,i,k}*(t_{k+1} - t_k) - ((V_{k+1} + V_k)/2)*(m_{i,k+1} - m_{i,k})}{((V_{k+1} + V_k)/2)*IVCD_k} \qquad (27b).$$

The approach in equation (27b) may be particularly useful in embodiments where the feedflow is continuous or semi-continuous, such as e.g. in drip feed flows. In embodiments where a bolus feed strategy is implemented (i.e. the feed flow is provided as relatively large instantaneous additions), equation (26b) can be rewritten using a pseudo metabolite concentration $pm_i$, that allows the feed flow to be eliminated from equation (26b), i.e. :

$$\frac{d\,(pm_i)}{dt} \quad = \delta_{m,i} * (x_v) \quad (26d).$$

For metabolites that are provided in the feed flow, a pseudo metabolite concentration $pm_i$ may be obtained by: (i) using the measured (or otherwise determined, such as e.g. based on the initial reactor volume and the volumes provided in the one or more feed bolus) reactor volume and known feed concentrations to determine how much of the metabolite is added to the reactor with each feed, and (ii) subtracting the value in (i) from all measurements of the metabolite's concentration after the feed. For metabolites that are not present in the feed (or that can be assumed not to be present in the feed), a pseudo metabolite concentration $pm_i$ may be obtained by: (i) using the measured (or otherwise determined, such as e.g. based on the initial reactor volume and the volumes provided in the one or more feed bolus) reactor volume to determine the change in concentration due to dilution that is caused by each feed, and (ii) adding the value in (i) from all measurements of the metabolite's concentration after the feed. Equation (26d) can be resolved for the metabolite specific transport rate at time k using a first-order finite difference approximation for the derivative:

$$\delta_{m,i} = \frac{\frac{dpmi}{dt}}{x_{v,k}} = \frac{pm_{i,k+1} - pm_{i,k}}{IVCD_k} \qquad (27d).$$

Equation (27c) can also be written as:

$$\delta_{m,i}(t_k) = \frac{(m_{i,k+1} - m_{i,k} - mAdd_{i,k}/V_k)}{IVCD_k} \qquad (27e)$$

where $m_{i,k}$ is the concentration of metabolite i at time k, $mAdd_{i,k}$ is the amount of metabolite i in the bolus addition of metabolite $i$ at time $k$, $V_k$ is the total volume in the bioreactor, and $iVCD$ is the integrated Viable Cell Density. Further, where the metabolite is a product of the cells (i.e. a metabolite that is not expected to be present in the feed, such as e.g. a desired product), the specific production rate of the metabolite can be written as:

$$\delta_{m,i}(t_k) = \frac{(m_{i,k+1} - m_{i,k})}{IVCD_k} \qquad (27f)$$

where $\delta_{m,i}(t_k)$ can also be written as $q_{IgG}(t_k)$ and $m_{i,k+1}, m_{i,k}$ can also be written as $C_{IgG,k+1}$, $C_{IgG,k}$ to represent that the metabolite is a desired product such as e.g. a recombinant antibody (IgG).

For an unfed-batch culture (where no feed flow, bleed flow or harvest flow is present, i.e. $F_F = F_H = F_B = 0$), equation (26) can be written as:

$$\frac{d\,(m_i)}{dt} \quad = \quad \delta_{m,i} * (x_v) \qquad (26c)$$

which can be resolved for the specific consumption /secretion rate at time k as:

$$\delta_{m,i,k} = \frac{\frac{dmi}{dt}}{x_{v,k}} = \frac{m_{i,k+1} - m_{i,k}}{IVCD_k} \qquad (27c).$$

Any methods to calculate an integrated viable cell density may be used in the methods described herein. For example, $IVCD_k$ may be calculated using equation (28):

$$IVCD_k = \left( \alpha\, x_{v,k} + \beta\, x_{v,k+1} \right) * (t_{k+1} - t_k) \qquad (28)$$

where the coefficients $\alpha$ and $\beta$ weight the relative influence of the two viable cell density values and are such that $\alpha + \beta = 1$. For example, the two values may be weighted identically, i.e. $\alpha = \beta = 0.5$. In embodiments, $\alpha$ and $\beta$ are chosen such that $\alpha > \beta$ (for example $\alpha = 0.6$ and $\beta = 0.4$). These coefficients may be chosen to reflect the observed cell growth behaviour, and may be chosen independently for each time point. Where exponential growth can be assumed, the integrated viable cell density can be calculated using a log transform. For example, the integral cell density may be calculated using equation (28a):

$$IVCD_k = x_{v,k} * e^{u_k(t_{k+1} - t_k)} \qquad (28a)$$

where

$$\mu_k = \frac{ln\left(x_{v,k+1}/x_{v,k}\right)}{t_{k+1} - t_k} \qquad (28b)$$

The equations for $\delta_{m,i}$ at time k as described above (or any corresponding equation that may be defined in view of the configuration of the process and the set of assumptions made) can be solved using the known (typically measured) biomass and metabolite concentrations at times/maturities k and k+1 to obtain a metabolite transport rate at every time point / maturity value where the above measurements are available. Further, this can be performed individually for each measured metabolite. The resulting metabolite transport rates characterise the metabolic condition of the cells in the culture as a function of maturity, and are expressed as an amount of metabolite (mass or moles) per cell per unit of maturity (i.e. typically per unit of time). This represents very valuable information regarding the metabolic condition of the cells, which information can be used by a metabolic condition model (e.g. a multivariate statistical model such as a PCA, PLS or OPLS) to monitor the cell culture as explained above. Note that in all equations for specific transport rates, the signs of all the terms can be inverted depending on whether a negative rate is taken to mean that the cells consume the metabolite and a positive rate is taken to mean that the cells produce the metabolite (i.e. the rate is seen from the perspective of the culture medium), or the opposite (i.e. a positive rate means that the cells consume the metabolite and a negative rate means that the cells produce the metabolite, in other words the rate is seen from the perspective of the cells compartment).

**Prediction of specific transport rates using machine learning**

Using the above approach, specific transport rates may only be accurately calculated at time points where metabolite concentration and viable cell density data is available. Alternatively, machine learning approaches (i.e. machine learning algorithms that train and/or deploy particular machine learning models) may be used to predict the specific transport rates of one or more metabolites at a future time point, based on the known (measured or calculated) values of one or more variables that characterise the bioprocess at one or more previous time point.

Thus, in embodiments, the specific consumption/secretion rate of a metabolite is obtained as $\delta_i = f_{ML,i}(u,m,s)$ where $f_{ML,i}$ is a model that has been trained to predict the specific consumption/secretion rate $\delta_i$ (alone or jointly with other $\delta_i$) as a function of one or more variables selected from process variables u (e.g. temperature, pH, etc.), one or more metabolite concentrations m (where metabolite concentrations can also be considered to form a part of the process variables, i.e. the notation u can refer to physico-chemical process variables and/or to metabolite concentrations in the bulk medium), and one or more variables s representing the state of the cell culture system (e.g. viable cell density, lysed cell density, total cell density, cell viability). For the avoidance of any doubt, any of the categories of variables may be absent, i.e. the model $f_{ML,i}(u,m,s)$ may comprise no variables selected from process variables $u$, no variables selected from metabolite concentrations $m$, and/or no variables selected from cell culture state variables s (provided that at least one variable of at least one of these categories is included). In embodiments, the one or more variables comprise at least a metabolite concentration m. Thus, in some embodiments, the process variables and/or the cell state variables may be absent, and the trained model may predict the specific consumption/secretion rate $\delta_i$ based at least (or solely) on one or more metabolite concentrations. Preferably, the one or more metabolite concentrations include the concentration of the metabolite for which a specific consumption rate is being predicted, and/or a metabolite whose concentration is highly correlated with that of the metabolite whose specific consumption rate is being predicted (such as e.g. an immediate product or precursor thereof). Without wishing to be bound by theory, it is believed that a machine learning that performs satisfactorily at the task of predicting a specific transport rate may be obtained using only metabolite concentrations as these are naturally correlated to the specific transport rates. However, process and cell culture state variables may carry complementary information, and a machine learning model may advantageously learn to use this information to improve its prediction accuracy. Thus, including more and/or a greater variety of predictive variables (such as e.g. including variables from one or more or each of the *u, m* and *s* categories) may result in a model that has improved predictive accuracy and/or that is able to achieve good and/or improved level of accuracy in a greater variety of conditions.

In embodiments, the machine learning model is trained to predict a specific transport rate for one or more metabolites, based on input values comprising the known (measured or calculated) values of one or more variables that characterise the bioprocess at a time point k, or at a plurality of time points (*k, k-1,...*). For example, the input values may comprise the known (measured or calculated) values of one or more variables that characterise the bioprocess at a first time point *k* and a second time point *k-1.* The input variables may comprise values of one or more variables at each of a plurality of time points, where the one or more variables may be chosen independently for each time point. For example, the one or more variables at one time point may partially overlap, fully overlap, or may not overlap with the one or more variables at another time point. In some embodiments, missing values may be present in the training data (the data used to train the model) or in the data provided to the model in use. Training and/or using the machine learning model may comprise imputing one or more missing values. Imputation methods are known in the art. Imputation methods suitable for use in the present context include e.g. linear interpolation, mean substitution, etc. The machine learning model may be trained to predict a specific transport rate for one or more metabolites at a future time, based on input values comprising the known (measured or calculated) values of one or more variables that characterise the bioprocess at one or more time points *k, k-1,* etc. In other words, the training data used may be such that the model predictions based on data at one or more time points *k, k-1,* etc. are evaluated against known corresponding values at a time *j > k, k-1,...* The plurality of time points in the input data used for training may be separated by a predetermined time period (e.g. 1 hour, 2 hours, 3 hours, 12 hours, 1 day, 2 days, etc.). For example, the input data used for training may comprise values at one time point and at a second time point separated from the first time point by a fixed period. During training of the model, the model predictions based on data at one or more time points *k, k-1,* etc. may be evaluated against known corresponding values (label values) at a time *j > k, k-1,* ... where the time point j is separated from one or more of the time points *k, k-1,...* by a predetermined time period. In a simple example, the machine learning model is trained to predict a specific transport rate for one or more metabolites on the next day (i.e. in 1 day / 24 hours) from the day of the latest one of the input values. In this example, if the input values comprise two time points separated by a fixed period of one day, then the machine learning model is trained to predict a specific transport rate for one or more metabolites on the next day based on the values of one or more variables on the present day and the preceding day.

The time periods (whether between input values or between input values and predicted values) may be approximately the same for the whole training data set. Alternatively, the training data may comprise sets of input values and/or input values and corresponding known (label) values that are not separated by the same time difference. For example, the training data may comprise measurements for a plurality of bioprocesses, where in some of the plurality of bioprocesses data was acquired every day, whereas in others data was acquired every half day. Preferably, the training data used comprises sets of input values and input values and corresponding label values that are separated by approximately the same time (or maturity, as the case may be) differences. In the example above, this can be achieved by only using measurements associated with consecutive days for the training data that includes more than daily measurements, or conversely by imputing measurements for time points in which measurements were not acquired. The trained machine learning model may advantageously be used to predict specific transport rates associated with time differences between prediction and latest input value and/or between multiple input values that are similar to the corresponding time differences in the training data. Reference to times and time differences may refer to corresponding maturities and maturity differences. Further, a machine learning model that has been trained to predict specific transport rates based on input values comprising values at a plurality of time points may be used to predict specific transport rates based on input values comprising missing values (e.g. comprising fewer time points than the number of time points that the machine learning model was trained on). This may be the case for example when values for a single time point are available (e.g. when the machine learning model is used to make a prediction based on initial conditions). Thus, for example, a machine learning model trained to predict specific transport rates based on input values comprising values at two consecutive time points may be used to predict specific transport rates based on input values comprising value at one time point and zero or imputed values for the other of the two consecutive time points that the machine learning model expects as input. In general, missing data may be imputed using a variety of approaches such as replacing a missing value by the mean, median or mode of a corresponding set of values, a value drawn randomly from a corresponding set of values, etc. Alternatively, machine learning algorithms that support missing values may be used.

Such algorithms may include k-nearest neighbours or classification and regression trees. For example, the machine learning model may be a random forest.

**Use of specific transport rates in state space model**

As explained above, equations capturing the evolution of bulk metabolite concentrations can be used to calculate specific secretion / consumption rates at particular time points at which metabolite concentration measurements are available. The specific transport rates calculated using material balance equations can be used to identify that a fault has occurred, for example using a metabolic condition model as explained above. However, these equations do not enable the prediction of the value of the specific consumption / secretion rates at future time points. Thus, it is also not possible to precisely calculate the concentration of the metabolites that can be expected at a future time point, since this requires the time dependent specific transport rates to be included in equation (26) (or equivalents). This in turn limits the ability to calculate the cell state variables that can be expected at such a future time point, since the concentration of metabolites that are substrate limiting, inhibitory or have a quadratic effect will influence the cell state variables (through the kinetic growth model as explained above).

Alternatively, the specific transport rates of one or more metabolites of interest may be predicted by a machine learning model, as explained above. These predictions can be integrated into the state space model by including them in equation (26) and equivalents, i.e. by augmenting the kinetic growth model described above with the following equations for one or more metabolites:

$$\frac{d\,(m_i)}{dt} \quad = \quad \frac{F_F}{V}*m_{F,i} \quad - \quad \frac{F_H}{V}*m_{H,i} \quad - \quad \frac{F_B}{V}*m_{B,i} \quad + \quad f_{ML,i}(u,m,s)*x_v*\left(\frac{m_i}{m_i+\varepsilon}\right)$$
$$(29)$$

where $\varepsilon$ can be set to 0 or a value that reflects the detection limit of the metabolite as explained above, and $(u,m,s)$ in $f_{ML,i}$ are values for the respective variables at one or more preceding time points. Such an augmented state space model can be solved at every time point t to predict new values for $m_i$, $x_v$, $x_d$, and $x_l$, which in turn can be used to predict new values for the specific transport rates at the next time point using the machine learning model, which prediction can be plugged back into the state space model to predict new values for $m_i$, $x_v$, $x_d$, and $x_l$, and so on and so forth.

**Prediction of critical quality attributes using machine learning**

**Figure** 3 is a flowchart illustrating a model deployment procedure through which critical quality attributes of a bioprocess can be predicted according to the present disclosure. At step 300, values for a plurality of process variables 300a and metabolite concentrations 300b (where the process variables and metabolite concentrations can collectively be referred to as process condition variables), and values of one or more system state variables 200c such as cell state variables and/or specific transport rates associated with a bioprocess are received. The values received at step 300 are used at step 310 by a trained machine learning model to calculate one or more predictions of critical quality attributes which are output at step 320. The predictions may then be used at step 330 for monitoring, controlling, optimising or simulating a bioprocess. All of the values received at step 300 may have been measured using sensors, inferred from measurements, predicted by a model or set by a user (or a combination of these). Preferably, at least some of the values have been calculated by a state space model as described herein. For example, the values received at step 300 preferably include one or more system state variables including: one or more cell state variables calculated using a kinetic growth model as described herein, one or more specific production/consumption rates calculated using a material balance model (bulk metabolite concentration model) as described herein or a machine learning model trained to predict specific production/consumption rates as described herein, and/or one or more metabolite concentrations calculated using a material balance model (bulk metabolite concentration model) as described herein. In embodiments, the values received at step 300 include at least one cell state variable (such as e.g. viable cell density, dead cell density, lysed cell concentration, total cell density, concentration of a biomaterial that accumulates in the culture as a result of cell growth and inhibits cells growth and/or is toxic to the cells, e.g. as provided in equations (11)-(14)) calculated using a state space model comprising a kinetic growth model and optionally a material balance model as described herein. In embodiments, the values received at step 300 include at least one specific transport rate calculated using a material balance model or a machine learning model as described herein. In embodiments, the values received at step 300 include at least one cell state variable that is calculated using a kinetic growth model as described herein, preferably wherein the kinetic growth model is part of a state space model comprising the kinetic growth model and a material balance model. For example, the at least once cell state variable may include lysed cell density (also referred to as "lysed cell concentration"). The use of cell state variables that are determined using a model as described herein rather than measured enables more informative predictions of CQAs to be made because this expands the range of possible informative metrics that can be used for making such predictions beyond what is commonly or easily measurable. In embodiments, the values received at step 300 include at least one metabolite concentration calculated using a state space model comprising a kinetic growth model and a material balance model as described herein. Preferably, the values received at step 300 include at least one process parameter selected from temperature, pH, dissolved oxygen, medium composition (e.g. the concentration of one or more metabolites and/or one or more other species in the fresh medium and/or in the bulk fluid, also referred to as bulk fluid composition), and the perfusion rate (if used).

Further details of a process for obtaining the values received at step 300 are provided on Figure 3B. At step 340, a bioprocess is run (to completion or to a current maturity) and process data is obtained at step 350. The process data may comprise one or more measurements from sensors and/or one or more process conditions that are set as parameters of the bioprocess, some of which may be considered to represent critical process parameters according to a previously established specification. At step 360, a specific production/consumption rate is calculated for one or more metabolites. This step may use material balance equations (also referred to herein as a "material balance model" or "bulk metabolite concentration model") as described herein (see section "Calculation of specific transport rates using material balance equations"), a machine learning model as described herein (see section "Prediction of specific transport rates using machine learning"), or a combination thereof. For example, some specific transport rates may be calculated using material balance equations and other may be calculated using machine learning model, for example depending on the metabolite concentration data available (e.g. where metabolite concentration data is available this can be used to calculate a specific transport rates for this metabolite using material balance equations, and one or more specific transport rates for other metabolites for which concentration data is not available may be predicted using a machine learning model). At step 370, the one or more specific transport rates obtained at step 360, together with any relevant process conditions received at step 350, may be used to calculate new metabolite concentrations using a material balance model as described above (see section "Use of specific transport rates in state space model"), which in turn may be used to calculate cell state variables such as the viable cell density (VCD), dead cell density, and lysed cell density, using a kinetic growth model as described above (see section "State space model - kinetic growth model"). The material balance model and kinetic growth model together may form a hybrid state space model, and the variables calculated using these models, together with the specific transport rates may be referred to as "system states". In embodiments where the metabolite specific transport rates are calculated from available metabolite data exclusively, step 370 may not use the specific transport rates and may instead calculate cell state variables using the kinetic growth model. In such cases, the material balance model is used at step 360, and the kinetic growth model is used at step 370, these steps together using a hybrid state space model and producing a set of system states comprising values for one or more metabolite specific transport rates and one or more cell state variables. At step 310, the process data obtained at step 350, the metabolite specific transport rates obtained at step 360, and the further system state variables obtained at step 370 are used to predict one or more critical quality attributes using a machine learning model that has been trained to predict the one or more CQAs from such input data.

The predictions obtained at steps 310/320 can be used as part of a process of simulating, monitoring and/or controlling a bioprocess for a variety of purposes. For example, within the context of simulating a bioprocess, the predicted CQAs can be used to establish a quantitative mapping of process conditions (including critical process parameters (CPP)) and CQA for improved process understanding and more informed establishment of operational targets for CPPs.

Indeed, the impact of various process conditions on CQAs can be quantified (by simulating the processes using the hybrid state space model with different sets of process conditions, and predicting the corresponding CQAs) in order to assess the influence of these process conditions on the CQAs. This can be performed for example using a grid search over the process conditions assessed and examining the map thus obtained, or by deriving from such simulated data a Jacobian matrix quantifying the change in CQAs as a function of a change in process conditions in various operating areas of interest. Still within the context of simulating a bioprocess, the CQA predictions may be used for running optimization studies to identify optimal conditions of CPP and other conditions to maximize yield and maintain quality. For example, a desirability function may be set which penalises any output that is outside of a predetermined specification for CQAs, while optimising one or more further objectives such as maximising yield. This desirability function can be used to evaluate the output of bioprocesses simulated using various sets of process conditions, as part of an optimisation process to identify process conditions that optimise the desirability function. Within the context of monitoring a bioprocess, the predicted CQAs may be used for real-time estimation of the quality of the product. This can in turn be used to inform further steps such as the implementation of a corrective action for quality deviations or for the implementation of downstream operations which may need to take those quality metrics into account. Further, this can be combined with a simulation step whereby the potential effect of a corrective action can be assessed prior to its implementation, in order to identify an appropriate corrective action. Within the context of process control, the methods described herein may be used as a real-time soft-sensor for CQAs, which can be incorporated in advanced control strategies (closed loop control). For example, the predicted CQAs may be used to identify whether a corrective action should be taken, for example because the predicted CQAs have deviated from a predetermined target range or value, and to automatically implement the corrective action (optionally after having determined a suitable corrective action based on the predicted CQAs or one or more further predicted CQAs associated with a simulation step to predict the effect of one or more candidate corrective actions on the CQAs), for example by controlling one or more effectors.

The machine learning model may be trained to predict one or more CQAs, based on input values comprising values of one or more variables that characterise the bioprocess (e.g. process conditions, metabolite concentrations, cell state variables and/or specific transport rates) at one time point k, or at a plurality of time points ($k$, $k-1$,...). For example, the machine learning model may be trained to predict one or more CQAs, based on input values comprising the values of one or more variables that characterise the bioprocess at a first time point $k$ and a second time point $k-1$. The predicted CQAs may be associated with a time point (i.e. considered to be representative of the expected CQA at said time point, and evaluated against the CQA at said time point during training) that is the current/latest time point k or a future time point j $k$, $k-1$,... The input variables may comprise values of one or more variables at each of a plurality of time points, where the one or more variables may be chosen independently for each time point. Thus, the one or more variables at one time point may fully overlap (i.e. the one or more variables may be the same for each of the plurality of time points), partially overlap or may not overlap (i.e. the one or more variables may be different between at least two of the plurality of time points) with the one or more variables at another time point. Missing values may be present in the training data (the data used to train the model) or in the data provided to the model in use. Thus, training and/or using the machine learning model may comprise imputing one or more missing values. Imputation methods suitable for use in the present context include e.g. linear interpolation, mean substitution, etc. The plurality of time points in the input data used for training may be separated by a predetermined time period (e.g. 1 hour, 2 hours, 3 hours, 12 hours, 1 day, 2 days, etc.), which may be a fixed period set independently or identically between any two consecutive time points. Similarly, time point j may be separated from the closest time point k by a predetermined time period. For example, the machine learning model may be trained to predict a CQA on the next day (i.e. in 1 day) from the day of the latest one of the input values. In this example, if the input values comprise two time points separated by a fixed period of one day, then the machine learning model is trained to predict a CQA on the next day based on the values of one or more variables on the present day and the preceding day.

The time periods (whether between input values or between input values and predicted values) may be approximately the same for the whole training data set. Alternatively, the training data may comprise sets of input values and/or input values and corresponding known (label) values that are not separated by the same time difference. For example, the training data may comprise measurements for a plurality of bioprocesses, where in some of the plurality of bioprocesses data was acquired every day, whereas in others data was acquired every half day. Preferably, the training data used comprises sets of input values and input values and corresponding label values that are separated by approximately the same time (or maturity, as the case may be) differences. In the example above, this can be achieved by only using measurements associated with consecutive days for the training data that includes more than daily measurements, or conversely by imputing measurements for time points in which measurements were not acquired. The trained machine learning model may advantageously be used to predict CQAs associated with time differences between prediction and latest input value and/or between multiple input values that are similar to the corresponding time differences in the training data. Reference to times and time differences may refer to corresponding maturities and maturity differences. Further, a machine learning model that has been trained to predict CQAs based on input values comprising values at a plurality of time points may be used to predict CQAs based on input values comprising missing values (e.g.

comprising fewer time points than the number of time points that the machine learning model was trained on). This may be the case for example when values for a single time point are available (e.g. when the machine learning model is used to make a prediction based on initial conditions). Thus, for example, a machine learning model trained to predict CQAs based on input values comprising values at two consecutive time points may be used to predict CQAs based on input values comprising value at one time point and zero or imputed values for the other of the two consecutive time points that the machine learning model expects as input. In general, missing data may be imputed using a variety of approaches such as replacing a missing value by the mean, median or mode of a corresponding set of values, a value drawn randomly from a corresponding set of values, etc. Alternatively, machine learning algorithms that support missing values may be used. Such algorithms may include k-nearest neighbours or classification and regression trees. For example, the machine learning model may be a random forest.

The prediction of one or more CQAs using machine learning may be applied in two ways depending on the use case and process model. In a first case, the methods may be used to estimate the final CQA values of a batch or fed-batch process, using input data selected from process trajectories from the start of the process to the current maturity. In other words, a machine learning model may be used to predict one or more CQAs at a time j corresponding to the end of the bioprocess, using input data at one or more maturities *k, k-1,...* selected from the start of the process to the current maturity (i.e. k is at most the current maturity). This may be particularly useful in cases where the bioprocess has a defined end, such as e.g. in a batch or fed-batch bioprocess, as in such cases the product is typically harvested and characterised at the end of the process. Thus, the ability to predict the final CQAs at any maturity during operation of the bioprocess may be particularly advantageous in such cases. In a second case, the methods may be used to estimate the current value of one or more CQAs, using input data selected from process trajectories over a moving window of historical data (1 day for example) up to the current time point. In other words, a machine learning model may be used to predict one or more CQAs at a time k corresponding to the current time, using input data at one or more maturities *k, k-1,...* selected from the start of the process to the current maturity (i.e. k is at most the current maturity). The input data may be selected to include data at one or more maturities *k, k-1,...* within a time window preceding the current time, such as e.g. in the 1 or 2 days preceding the current time k. This may be particularly advantageous in cases where the bioprocess does not have a defined end, such as e.g. in a perfusion process, as in such cases the product is typically harvested throughout at least some of the process (and hence the value of CQAs of the product harvested while the process is ongoing is of relevance). An appropriate time window may be set during the training process, by identifying the characteristics of the input data (e.g. which input variables and time points) that results in the best performing machine learning model (e.g. the machine learning model that has the smallest loss when assessed on suitable validation and/or test data), or using prior knowledge about the properties of the bioprocess, or a combination of the above (Such as e.g. by testing different sets of input data using prior knowledge to identify the relevant sets of input data to be tested).

As mentioned above, although multivariate approaches to identify bioprocesses that are operating "outside of specification" exist, their current implementation only allows to determine whether a bioprocess is currently expected to be operating normally based on the values of specific critical process parameters, rather than based on the influence of any process parameters on CQAs through their effect on the cell state and metabolism. They do not allow the prediction of whether a bioprocess will operate normally at a future time point, or whether a bioprocess will operate normally if one or more process conditions are changed. The present approach improves on this situation, by enhancing measured data with transformations that extract biologically relevant metrics (such as specific consumption rates of metabolites and states from the hybrid state space model), and the use of more flexible modeling approaches (machine learning models) that are better suited for fitting the variation of CQA.

**Figure 4** is a flowchart illustrating a model calibration procedure, which can be used to obtain a calibrated machine learning model for predicting one or more CQAs of a bioprocess (such as the model 310 in Figure 3A). At step 400, values for a plurality of process variables 400a and metabolite concentrations 400b (where the process variables and metabolite concentrations can collectively be referred to as process condition variables), and values of one or more system state variables 400c associated with a plurality of bioprocesses are obtained. These may have been measured using sensors (as will be explained further below), and/or inferred from measurements (for example by using a kinetic growth model and/or a material balance model as described herein to obtain one or more system state variables). The values received at step 400 comprise input data that can be used to train the machine learning model, and together represent a training data set. At step 430, data that can be used to verify the output of the machine learning model (i.e. labels or data from which labels can be calculated) is also received, which comprises one or more measured CQAs associated with the bioprocesses for which data was obtained at step 400. This also forms part of the training data set. At step 410, one or more of the values received are provided as input to a machine learning model which provides as output a set of predictions of one or more CQAs at step 420. These may represent a subset of the training data set, for example when the training data set is divided into a training set and a validation set and/or when training data is sampled from a training set at every training iteration. The predicted CQAs are compared to corresponding measured CQAs obtained at step 430. Alternatively, the predicted CQAs may be compared to corresponding measured values indirectly, by computing one or more further values based on the predicted CQAs and comparing the one or more values to corresponding measured values (or values derived therefrom). As an example, a predicted yield may be used to calculate the concentration of a corresponding product in the culture, and this may be compared to a corresponding measured value (i.e. a concentration that is measured or derived from measured values). Comparing the predicted and corresponding known values typically comprises calculating the value of a loss function as explained above. At step 440, the loss value calculated is used by an optimisation algorithm to modify the machine learning model.

Steps 400, 410, 420, 430 and 440 are typically repeated using different or partially overlapping sets of input values received at step 400, all of which collectively constitute the training data. The process may be repeated until one or more stopping criteria are met. For example, stopping criteria may include a maximum number of iterations, a threshold on the amount of change in the value of the loss function (for example when assessed on a validation data set that forms part of the training data set) compared to one or more preceding iterations, a target loss function value having been reached at one or more iterations, etc. As a result, a trained machine learning model is obtained, which can be used to make predictions as explained in relation to Figure 3. The process may be repeated using different types of machine learning models and/or different model architectures in order to identify a particular machine learning model that has one or more desired characteristics such as e.g. a desired speed of computation, a desired prediction accuracy, etc. Models with different architectures may differ by their input variables (such as e.g. different lag values, values at different maturities, values for different variables), by their output variables (such as e.g. each model predicting a different set of CQAs), by the parameters of their hidden layers in the case of neural networks (e.g. number of layers, number of nodes per layer, activation function used, etc.), by the number of models used in an ensemble of models and/or the way in which predictions from the models in the ensemble are combined, and/or by the hyperparameters used for training. One or more machine learning models may then be provided to a user for deployment as explained in relation to Figure 3. The one or more machine learning models may use different sets of input variables, for example so that the user can choose to use the machine learning model for which they are able to provide input data. A plurality of machine learning models may be provided which each predict a different CQA or set of CQAs, for example so that the user can choose to use the one or more machine learning models that provide predictions that are of interest to them.

Machine learning models that jointly predict a plurality of CQAs may be advantageous when the plurality of CQAs are influenced by at least some of the input variables in a similar way. For example, CQAs that are impacted in a similar way by a change in metabolism that is reflected in or influenced by the value of the input variables used may advantageously e jointly predicted. CQAs that are influenced by changes in the metabolism in a different way from other CQAs may advantageously be predicted using a machine learning model that does not jointly predict other CQAs that are not influenced in the same way. These situations may be identified by trial-and-error, for example by testing different combinations of input and output variables, and/or using prior knowledge of the factors that are likely to influence the values of the plurality of CQAs to be predicted, and/or using multivariate statistical approaches to identify sets of one or more CQAs that are independent of one another (or conversely that are correlated with each other).

**Figure 5** shows an embodiment of a system for monitoring and/or controlling a bioprocess according to embodiments of the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is operably connected, such as e.g. through a network 6, to a bioprocess control system comprising a bioreactor 2, one or more sensors 3, and one or more effectors 4. The computing device 1 may be a smartphone, tablet, personal computer or other computing device. The computing device 1 is configured to implement a method for monitoring and/or controlling a bioprocess, as described herein. In alternative embodiments, the computing device 1 is configured to communicate with a remote computing device (not shown), which is itself configured to implement a method of monitoring a bioprocess, as described herein. In such cases, the remote computing device may also be configured to send the result of the method of monitoring a bioprocess to the computing device 1. Communication between the computing device 1 and the remote computing device may be through a wired or wireless connection, and may occur over a local or public network such as e.g. over the public internet. Each of the sensor(s) 3 and optional effector(s) 4 may be in wired connection with the computing device 1, or may be able to communicate through a wireless connection (i.e. through a network 6), such as e.g. through WiFi, as illustrated. The connection between the computing device 1 and the effector(s) 4 and sensor(s) 3 may be direct or indirect (such as e.g. through a remote computer). The one or more sensors 3 are configured to acquire data that relates to a bioprocess being performed in the bioreactor 2, which can be implemented as illustrated on Figure 1). The one or more effectors 4 are configured to control one or more process parameters of the bioprocess being performed in the bioreactor 2.

The one or more sensors 3 may each be on-line sensors (sometimes also referred to as "inline sensors"), which automatically measure a property of the bioprocess as it progresses (with or without requiring a sample of the culture to be extracted), or off-line sensors (for which a sample is obtained whether manually or automatically, and subsequently processed to obtain the measurement). Each measurement from a sensor (or quantity derived from such a measurement) represents a data point, which is associated with a time (or corresponding maturity) value. The one or more sensors 3 may comprise a sensor that is configured to record the biomass in the bioreactor 2, referred to herein as a "biomass sensor". This is typically in the form of a viable cell density or parameter from which viable cell density can be estimated. The biomass sensor may record a physical parameter from which the biomass in the bioreactor (typically in the form of the total cell density or the viable cell density) can be estimated. For example, biomass sensors based on optical density or capacitance are known in the art. The one or more sensors may further comprise one or more sensors that measure the concentration of one or more metabolites, referred to herein as "metabolite sensors". A metabolite sensor may measure the concentration of a single or a plurality of metabolites (such as e.g. from a few metabolites to hundreds or even thousands of metabolites), in the culture as a whole, the culture medium compartment, the biomass compartment (i.e. the cells as a whole), or specific cellular compartments. Examples of metabolite sensors are known in the art and include NMR spectrometers, mass spectrometers, enzyme-based sensors (sometimes referred to as "biosensors", e.g. for the monitoring of glucose, lactate, etc.), etc. Most of the commonly used metabolite sensors measure the concentration of a metabolite in the culture medium. As used herein, sensors 3 (such as e.g. metabolite and biomass sensors) may also refer to systems that estimate the concentration of a metabolite or the amount of biomass from one or more measured variables (e.g. provided by other sensors). For example, a metabolite sensor may in practice be implemented as a processor (e.g. processor 101) receiving information from one or more sensors (e.g. measuring physical/chemical properties of the system) and using one or more mathematical models to estimate the concentration of a metabolite from this information. For example, a metabolite sensor may be implemented as a processor receiving spectra from a near infrared spectrometer and estimating the concentration of metabolites from these spectra. Such sensors may be referred to as "soft sensors" (by reference to their "measurements" being obtained using a software rather than by direct measurement). The one or more sensors 3 may further include one or more sensors that measure further process conditions such as pH, volume of the culture, volumetric / mass flowrate of material in / out of the bioreactor, medium density, temperature, etc. Such sensors are known in the art. Whether one or more sensors 3 that measure further process conditions are necessary or advantageous may depend on at least the operating mode and the assumptions made by the material balance module as will be explained further below. For example, where the bioprocess is not operated as an unfed-batch, it may be advantageous to include one or more sensors measuring the amount and/or composition of the flows entering and/or leaving the bioreactor. Further, where the material balance module does not assume a constant volume in the bioreactor, it may be advantageous to include a sensor that measures the volume of liquid in the bioreactor (such as e.g. a level sensor). The measurements from the sensors 3 are communicated to the computing device 1, which may store the data permanently or temporarily in memory 102. The computing device memory 102 may store one or more trained machine learning algorithm(s) as described herein. The processor 101 may execute instructions to predict one or more critical quality attributes and optionally one or more specific transport rates using a respective trained machine learning model and the data from the one or more sensors 3 and/or simulated data, as described herein (such as e.g. by reference to Figure 3). Note that a system as described by reference to Figure 5 may also be used to train a machine learning model as described herein (such as e.g. by reference to Figure 4).

In the context of process simulation, some of the values may have been measured and others may have been predicted or set by a user. For example, a user may want to investigate the impact of implementing a particular change in one or more process conditions on one or more metrics of bioprocess performance. For this purpose, one or more values may be set based on measured time courses and others may be set to represent the intended change. Alternatively, all of the values may be set to represent a set of conditions that is intended to be used. In embodiments using machine learning to predict specific transport rates, the values comprise at least initial conditions that are sufficient for the machine learning model to predict a first set of specific transport rates. These predictions can be used to calculate future metabolite concentrations and/or cell states, for example using material balance equations and/or a kinetic growth model as described herein. These values can be fed back to the machine learning model predicting the specific transport rates, optionally in combination with further process parameters that are intended to be set by the user. A new set of predictions can then be obtained as above, and the process can be repeated as many times as desired. As above, the predictions themselves may provide an indication of the metabolic condition of the cells in the bioprocess that is being simulated. Further, the metabolite concentrations and/or cell states calculated as part of the simulation process for example using material balance equations and/or a kinetic growth model as described herein may also provide information that is indicative of the performance of the cell culture. The metabolite concentrations and/or cell states calculated as part of the simulation process can then be used to predict one or more critical quality attributes using one or more machine learning models as described herein. This application may be referred to as a "digital twin" because the simulation replicates real process conditions *in silico* in order to understand their effects on cell culture performance. Such simulation processes can be useful for example to investigate the impact of a process condition (e.g. temperature profile, pH, dissolved oxygen profile, agitation, medium composition, flow parameters, etc.) on the critical quality attributes of a bioprocess. In such cases the initial conditions may include all starting process conditions that are used by the machine learning model including concentrations of metabolites, and the process conditions at each subsequent time point (e.g. used as inputs to the machine learning model and/or used as parameters of the material balance / kinetic growth model) other than metabolite concentrations (which will be calculated by the model) may be set to those that are investigated.

In the context of process optimisation, the above described simulation process may be integrated as part of an optimisation process through which a plurality of process conditions or combinations thereof are investigated and compared in view of one or more desirability criteria including at least one critical quality attribute criterion. For example, a further desirability criterion may include the concentration of a desired product or total amount of desired product produced over a predetermined amount of time.

As the skilled person understands, the accuracy of the predictions from any machine learning model depends on a combination of the data that has been used for training the model and the particular use of the model. For example, a machine learning model may provide more accurate predictions when the input data has characteristics in terms of time differences between a plurality of time points that are similar to at least some of the training data that has been used to train the model. As another example, a machine learning model that has been trained using training data that reflects a wide variety of process conditions may provide more accurate predictions for previously unseen process conditions than a machine learning model that has been trained using training data that captures a much narrower set of conditions. Thus, without wishing to be bound by theory, it is believed that for the purpose of predictive process monitoring (where the process is expected to run within normal conditions), a machine learning model trained using data that is representative of a (typically relatively narrow) set of normal conditions (conditions that are known to result in a process within specification) may be sufficient. Conversely, for the purpose of simulation or optimisation, a machine learning trained using data that is representative of a variety of conditions is likely to perform better. However, note that such simulation or optimisation is simply not possible in the absence of prediction, such that even an imperfect machine learning model may provide an advantage. As a further example, a machine learning model may provide more accurate predictions when the input data has characteristics in terms of process parameters such as e.g. agitation/mixing, air delivery, temperature profile (e.g. whether a temperature shift was implemented or not), etc. that are similar to at least some of the training data that has been used to train the model. For example, a model trained using data obtained from bioprocesses run in an Ambr® 250 bioreactor, a 2l and/or up to a 10001 bioreactor may provide more useful predictions for bioprocesses run in any of these bioreactors than for bioprocesses run with reactors that have a different geometry and mixing parameters such as e.g. an Ambr® 15 bioreactor.

Specific embodiments of the use of a state space model for bioprocess monitoring, control, simulation and optimisation will now be described, where the state space model can be used to obtain variables from which critical quality attributes can be predicted.

**Hybrid model for predictive bioprocess monitoring, control, simulation and optimisation**

**Figure 6** shows an exemplary embodiment of a computing architecture for a hybrid model comprising a state space model and critical quality attributes prediction model as described herein. A kinetic and metabolic state observation system 105 comprises a plurality of specific processing modules, including a kinetic growth model 150, a metabolic condition model 160, a state correction model 175, a process monitoring engine 180, a flagging and alerts engine 185, and a consumption rate and secretion rate module 190. The metabolic condition model 160 may comprise additional modules, including a multivariate statistical modeling engine 165 (also referred to herein as a PCA and PLS statistical modeling engine) and a data-driven machine learning engine 170. The kinetic growth model 150 and the metabolic condition model 160 together form a model referred to as a "hybrid model". The kinetic model is a model designed to determine amounts of live cells, lysed cells, viable cells, and cell density, etc (as explained above). The metabolic condition model 160 is a model designed to provide quality control information about the bioprocess, such as product titer and information about the product and attributes of the bioprocess (e.g., by-products, etc.). The output of the metabolic condition model 160 may feed into the kinetic growth model 150, i.e. some of the values calculated using the metabolic condition model 160 may be used as input variables in the kinetic growth model 150. The state correction model 175 may be used to update the estimate of the states of the kinetic growth model 150 based on experimental data. An error may be derived based on differences between measured experimental output and estimated hybrid model output, and the parameters of the hybrid model may be adjusted based on the error signal to drive the error signal to zero as a function of time.

Kinetic growth model 150 may be a state space model based on the Monod growth equations (see equations (11)-(24)) and material balance equations (metabolite bulk concentration equations, see equations (25)-(29)). Thus, the kinetic growth model 150 may comprise the kinetic growth model and the bulk metabolite concentration models described above, together forming a state space model. As described above, the Monod growth equations and metabolite material balance equations are a series of differential equations that may be used to describe cell growth (e.g., microbial cell growth), cell density and viable cell density, total cells (e.g., viable cells, dead cells and lysed cells), etc. Inputs to the kinetic growth model may include temperature, feed conditions, pH, etc. and outputs include state estimates. The kinetic growth model may be seen as a classical state space model. In this context, parameters that are being modeled internally are referred to as states. For example, these parameters may include $x_v$, $x_d$, $x_l$, $m_i$. The kinetic growth model may be used to monitor the number of live cells (and other parameters) in a bioreactor and to make predictions about the number of cells in the bioreactor at a future point in time. In addition to the kinetic growth equations (equations (11)-(24) above) and the material balance equations (e.g. equation (28) or any of equations (25)-(26) and equivalents), the kinetic growth model may comprise a separate equation to describe the rate of change of a desired biomaterial as a function of time. This can be represented as:

$$\frac{dIgG}{dt} = Qp\big(m, \delta_{m,i}(t), u\big)x_v \qquad (30)$$

wherein $\frac{dIgG}{dt}$ is the rate of change of the biomaterial as a function of time, $Qp$ is a function with inputs comprising metabolite concentrations $m$, $\delta_{(m,i)}(t)$ is the specific production or specific consumption rate of a metabolite at the current time, $u$ is a set of independent variables (i.e. variables that are provided as input to the model and for which the model does not calculate new values, for example these may include process parameters such as temperature, pH, etc.), and $x_v$ is viable cell density. Where the biomaterial is itself a metabolite, equation (30) can take the form of a material balance equation as described above (and as such may be included as part of a set of material balance equations). Similarly, where the biomaterial is the biomass itself, equation (30) can take the form of the relevant kinetic growth equation, such as the equation capturing the viable cell density. In other words, equation (30) may already form part of the model as described above if the desired biomaterial is biomass or a metabolite already captured in the material balance equations included in the model. Outputs of the kinetic growth model 150 may include product titer, metabolite concentration, viable cell density, and viability. The kinetic growth model 150 may also be used to calculate specific consumption (or secretion) rates for one or more metabolites at a given time from measured data as explained above. Alternatively, the specific consumption (or secretion) rate for one or more metabolites may be predicted for a future time using a trained machine learning model as explained above. Further, it is also possible for some specific transport rates to be calculated using the kinetic growth model and measured data, and for other specific transport rates to be predicted using a machine learning model.

Metabolic condition model 160 performs two functions: (i) predicting one or more critical quality attributes of the process, and (ii) optionally classifying the internal metabolic state of the system, e.g., into an optimal or suboptimal state or category for biomaterial production. To perform both of these tasks, the metabolic condition model 160 uses as input one or more of: process conditions, measurements such as metabolite measurements, state space variables from the kinetic growth model 150 (such as e.g. variables of the growth equations such as viable cell density and/or variables of the material balance equations such as metabolite concentrations), and specific transport rates of metabolites. Optionally, input into the metabolic condition model may include temperature, feed conditions, etc. In general, the metabolic condition model is independent from the kinetic growth model, and metabolic condition monitoring may be performed independently of kinetic growth. In embodiments, the metabolic condition model may be used to enhance the kinetic growth model by providing estimates of titer and/or quality to improve titer prediction. To classify the internal metabolic state of the system, the metabolic condition model may use as minimal inputs the specific consumption/specific production of metabolites. As explained above, these may be calculated from metabolite concentrations and viable cell density data, or may be predicted using a machine learning model. The metabolic condition model optionally uses as input additional measured parameters and/or unmeasured states to improve prediction of product titer and/or quality. In some embodiments, the metabolic condition model comprises a statistical modeling engine 165 for building principal component analysis (PCA) or partial least squares (PLS) or orthogonal partial least squares (OPLS) models using the specific consumption/production rates (and optionally, additional parameters measured from the process or states) as inputs, and producing one or more multivariate scores as outputs. Statistical modeling engine may comprise any suitable engine, including (PCA) model, a partial least squares (PLS) model, a partial least squares discriminant analysis (PLS-DA) model, and/or an orthogonal partial least squares discriminant analysis (OPLS-DA) model. PCA may be used to characterize the variation within a data set, i.e. in this case to characterize metabolic variation. PLS may be used to correlate metabolic variation to productivity (titer) or production of an important quality metric (product quality). Techniques for performing PLS may be found, e.g., in Wold et al., PLS-regression: a basic tool of chemometrics, Chemometrics and Intelligent Laboratory Systems 58 (2001) 109-130. Techniques for performing PCA may be found, e.g., in Wold et al., Principal Component Analysis, Chemometrics and Intelligent Laboratory Systems 2 (1987) 37-52. Both PCA and PLS may be used to reduce dimensionality of a data set, i.e. to extract a set of summary variables that capture as much as possible of the variability in the data. PCA, a type of unsupervised dimension reduction technique, allows data to be summarized by linear combinations of variables without losing a significant amount of information. PLS, a type of supervised dimension reduction technique, is applied based on correlation between a dependent variable and independent variables. These techniques are considered to be within the scope of one of skill in the art.

In some embodiments, the output of the metabolic condition model 160 feeds into the kinetic growth model 50.

The metabolic condition model 160 may also allow visualization of the metabolic condition of the cells in the bioprocess. Therefore, the output of the metabolic condition model 160 may serve as both an input to the kinetic growth model 150, as well as facilitates monitoring and visualization of the metabolic condition of the cell metabolism. The metabolic condition model may contain or be linked to a data-driven machine learning engine 170. Machine learning engine 170 may comprise one or more machine learning models such as a neural net, a deep learning model, or other machine learning model. Machine learning engine 170 may be trained using known techniques to classify the state of the biological system/bioreactor into an optimal or suboptimal state. Additionally, machine learning engine 170 may be trained using known techniques to classify the state of metabolite(s) into an optimal or suboptimal state. In embodiments, machine learning engine 170 performs classifications comparable to statistical modeling engine 165, and in some cases, with higher accuracy and precision than statistical modeling engine 165. In embodiments, the machine learning engine 170 further comprises one or more machine learning models trained to predict specific production/consumption rates of one or more metabolites as described herein. The machine learning engine 170 further comprises one or more machine learning models trained to predict critical quality attributes as described herein. To predict critical quality attributes as described herein, the metabolic condition model 160, and more particularly the machine learning engine 170 uses as inputs one or more variables of the kinetic growth models such as e.g. metabolite concentrations. Optionally, inputs into the metabolic condition model for the purpose of predicting CQAs may include process conditions such as temperature, feed conditions, etc.

State correction model 175 mitigates error in the system. The difference between the output of the hybrid model and the measured data may be determined, and provided as an error signal to state correction model 175. The state correction model acts to minimize the error, and to drive the error signal to zero as a function of time. State correction may correspond to techniques associated with an extended Kalman filter. In embodiments, the state correction model 175 may be a separate module, or optionally, may be integrated into the kinetic growth model. Process monitoring engine 180 may interface with a plurality of sensors which measure one or more parameters associated with the bioreactor. These parameters may include temperature, oxygen levels, feed conditions, pH or other aspects of the bioprocess which may be monitored in real-time or in near real-time. These measurements may be provided to the hybrid model for simulating the bioreaction. These measurements may also be provided to the metabolic condition module 160 to monitor cell metabolism and generate CQA predictions. Flagging and alerts engine 185 monitors the system for process deviations. If an output of the bioreactor deviates from its expected/predicted output, an alert is provided to the user. For example, the bioprocess may be suspended by the system until the process is corrected. As another example, the system may compensate for the deviation (e.g., adjusting the feed or process conditions to reach a desired state (e.g., an optimal state)). Flagging and alerts engine 185 may also send notifications to a user regarding the state of the bioreactor. For example, a notification may be sent to a user, when the internal metabolic state is classified into a suboptimal category, or where one or more critical quality attributes is/are not within specification.

Consumption rate and secretion rate module 190 determines the specific consumption rates and specific production rates of metabolites/analytes in the bioprocess reactor. This can be performed using known metabolite concentrations and material balance equations as explained above, or using a machine learning model as described above, by calling the machine learning engine 170. The specific consumption data is used by the metabolic condition model 160. Outputs from the metabolic condition model 160 may be provided into the kinetic growth model to predict product titer. Controller 195 may receive feedback (e.g., output of the bioreactor) to control the bioreactor to automatically adjust the experimental process conditions to minimize deviation from optimal process conditions.

Database 130 contains various types of data for the kinetic and metabolic state observation system 105. Training data 132 comprises data to train the metabolic condition model 160 (in particular the machine learning engine 170) to predict CQAs. Training data 132 may further comprise data to identify kinetic model coefficients, calculate metabolite specific consumption/production rates (including in some embodiments data to train the machine learning model that predicts specific consumption/secretion rates), and/or to train the metabolic condition model 160 to classify the state of the cells into an optimal or sub-optimal state or determine estimate the amount of a production of a biomaterial such as the specific productivity. Process conditions 134 correspond to the process conditions of the current bioprocess reaction. Process conditions 134 may also contain ideal process conditions that have been experimentally determined. These conditions may be supplied to the hybrid model (150, 160) to facilitate process monitoring of the current bioprocess operation or simulation/forecasting of the bioreactor. The output 136 is the output of kinetic and metabolic state observation system 105, and this output may be subtracted from the output of the experimental system in order to generate an error signal which is fed back into the input of the hybrid model.

Measurements of the metabolites and cell density may be obtained by process monitoring engine 180, and provided to the specific consumption and secretion rate model 190 to determine the specific consumption rate or the specific secretion rate of the metabolites, either by using the material balance equations as described above or by calling the machine learning engine 170. The specific consumption rate and specific secretion rate may be provided as input to the metabolic conditioning model. The specific consumption rate and specific secretion rate allow for translating process conditions into an amount each cell is consuming or producing for each of the metabolites (i.e. a metabolic condition variable). In embodiments, the specific consumption rate and specific secretion rate may be provided to the metabolic condition model 160, wherein the PCA and PLS statistical model engine 165 and/or data-driven machine learning engine 170 classifies the state of the cell to determine whether the system is in an optimal or a suboptimal condition, for example, with respect to a process parameter (e.g., temperature, feed concentration, pH, etc.).

The architecture above may be used to monitor a bioprocess, as illustrated on **Figure 7.** Using this architecture, instead of simply using measurable characteristics of the bioprocess for monitoring purposes, the internal states of the bioprocess (i.e. the characteristics of the cell culture and the metabolism of the cells) can be estimated, providing a richer picture of the state of the bioprocess. For example, a set of process parameters (also referred to as "inputs" or "output" of the bioreactor, depending on whether the parameters are set as operating conditions or whether they are results of the activity of the cells in the bioprocess) may be measured at step 705 and used as inputs to the hybrid model. These may include metabolite concentrations, viable cell densities (VCD), product titer, product quality, viability of cells, product quality, temperature, pH, dissolved oxygen (DO), etc. The process parameters are typically measured as a function of time. In embodiments, a zero-order hold is used to estimate the value over sampling intervals. The measured process parameters are provided to the kinetic growth model 150. The kinetic growth model can use some of this data (in particular, initial conditions) to initialise the state values (e.g. cell culture variables and metabolite values), including $x_v$, $x_d$, $x_l$, $m_i$ at step 710. The kinetic growth model 150 can also be initialised with parameters at step 715 which may be provided by a user, retrieved as defaults or received together with the measured data. The kinetic growth model 150 can then be used to determine values for the states $x_v$, $x_d$, $x_l$, $m_i$ at step 725, simply by solving the equations in the model. This can be achieved by integrating the set of kinetic equations in the kinetic growth model from the time at the beginning of the reaction to the current time, given the measured values of process parameters. Solving the kinetic growth model requires knowledge of the specific consumption/production rates at every modeled time point. This knowledge can be obtained at step 720 from previous experiments, i.e. by calculating the rates from metabolite concentration and viable cell density measurements in previous experiments and assuming that these also apply to the present process (especially if the values obtained at corresponding times are used). Alternatively, one or more specific consumption/secretion rates can be predicted at step 720 using a machine learning model based on process parameters and/or kinetic growth model state estimates at previous time points, as described herein. The output of the kinetic growth model 150 may include product titer, metabolite concentration, viable cell density, and viability. As the skilled person understands, the kinetic growth model may include equations that capture the change in concentration of one metabolite, or a plurality of metabolites. Similarly, the specific consumption/secretion rates for one or a plurality of metabolites may be predicted and may be used by the metabolic condition model.

If the cells experience fluctuations or deviations in process conditions (e.g., changes in temperature, increases or decreases in metabolites, feed condition alterations, etc.), the cells may enter a suboptimal state, and the output (e.g., titer of a biologic under production) may be suboptimal. Therefore, the kinetic growth model 150 may be supplemented with the metabolic condition model 160. This provides a way to predict critical quality attributes, and optionally to estimate the internal state of the cell, thereby correlating output production with environmental variables (via state variables) to optimize production. This may be particularly valuable in a multidimensional system having a large number of states, including some states that are correlated with each other, because in such cases determining which process condition(s) to adjust is challenging. Thus, metabolic condition model 160 provides a way to predict CQAs, to estimate the internal state of the cell, and to correlate output production with environmental conditions (variables) to optimize titer production. Further, if the system deviates from an optimal range, a user may receive a notification, prompting the user to correct the bioprocess to return the reaction to optimal conditions. In some embodiments, the system may autocorrect feed or environmental conditions to return the system to optimal conditions. For example, by providing feedback to a controller controlling the bioreactor, the experimental process conditions may be automatically adjusted to minimize deviation from the optimal process conditions. An optimization routine may further be used to determine process adjustments, as described below.

The functioning of an exemplary metabolic condition model 160 will now be described. The metabolic condition is determined at step 750 using the metabolic condition model, based on the specific consumption and production rates that have been calculated and/or predicted as explained above, at step 745. At step 745, the value of one or more CQAs is also predicted, although this is not used to determine the metabolic condition, which represents an additional piece of information that can be used to monitor a bioprocess. The metabolic condition may optionally further be used to calculate a specific productivity, or one or more quality attributes, at step 755. This may be done for example using (O)PLS models that have been trained to predict these values from the metabolic condition. The specific productivity (production of target protein) may be used in the kinetic growth model to estimate the product titer. For the purpose of process monitoring, the current metabolic condition can be classified at step 760 using one or more classification methods (such as e.g. machine learning classifiers or by comparing the current metabolic condition with a metabolic condition or range of conditions that are deemed to correspond to a normal and/or optimal state). Further, the data driven methods provided herein (e.g., PCA and PLS statistical modeling engine 165 and data driven machine learning engine 170) may be used to reduce the dimensionality of the system, and/or allow identification of conditions which impact titer. For example, when looking at a system having a high number of state variables (including e.g. a plurality of process conditions, metabolite concentration, growth variables and specific consumption/secretion rates) including variables that are correlated, it can be difficult to even identify deviations from optimality, let alone determine which process conditions to adjust in order to impact titer. The present techniques provide granular and specific control over the bioreactor, by identification of process variables that are outside an optimally determined range. In embodiments, optimal refers to a range for a process or feed condition that corresponds to optimized production of titer. However, any definition of optimality may be used.

The output of the hybrid model (e.g., state estimates, metabolic states, etc.) or of the kinetic growth model alone (in embodiments where a metabolic condition model is solely used to predict CQAs and not to determine a metabolic condition) may be compared with the output of the bioreactor at step 730, and the difference between the measured parameters and estimated parameters may be fed back through state correction model 175 into the input of the hybrid model 220, to improve the model. The state correction model 175 seeks to modify parameters to minimize the difference between the measured bioreactor output and the hybrid model output, to drive the error signal to zero as a function of time. In general, state estimators may be used to estimate the internal state of a system, when the state of the system is not directly measureable. In particular, Kalman filters may be used to determine an optimal estimate of the internal system states based on indirect measurements in a noisy environment, at step 740. That is, based on process conditions and kinetic models, a Kalman filter may be used to optimally estimate the internal state(s) of the system. Kalman filters are especially suitable for producing optimal estimates of system states in noisy systems. In this example, the state correction model 175 may comprise a Kalman filter or extended Kalman filter. The Kalman filter or extended Kalman Filter (EKF) may be used to determine optimal state values, wherein the error is combined with uncertainty in model state estimates, uncertainty in the state measurements, and covariance of the errors. Kalman filters may be applied to the kinetic growth model to improve the accuracy of the estimates of cell states provided by the model. The kinetic growth model and/or the metabolic condition model may be calibrated (a process which refers to the identification of suitable parameters in the models) using historical training data as known in the art.

The hybrid model described above may also be used for optimisation. Optimization may involve searching through various input variables to find a set that meets one or more critical quality attributes, optionally while maximizing titer or other desired result, typically using an optimization algorithm. Optimisation can be performed at run time, for example when monitoring a bioprocess as described above, in order to predict process conditions that may be used to restore an optimal state in a process that has been identified using the hybrid model to perform in a suboptimal manner. Optimisation can also be performed independently of any particular run, such as e.g. to identify optimal process conditions for a future bioprocess. Input variables typically include nutrient additions and independent process parameters such as temperature and pH. An optimisation process may proceed as described below, by reference to **Figure 8**. A set of trajectories for input variables ($u_i$), which may include nutrient additions, is received at step 810. At step 815, the state values (i.e. the states of the kinetic growth model) are initialized from initial measured data ($x_v$, $x_d$, $x_l$, $m_i$). The equations of the kinetic growth model are then integrated at step 820 to determine growth and metabolite trajectories over suitable time ranges. At step 825, one or more CQAs are predicted using the growth and metabolite trajectories (or parts thereof) that have been calculated at step 825, as described above. Thus, according to the present invention, CQAs are predicted using a machine learning model that takes as input predictor variables that include at least one value that is modeled as a state of a model that captures kinetic growth and material balance (such as e.g. variables of growth equations such as viable cell density and/or variables of material balance equations such as metabolite concentrations, and/or specific transport rates). In other words, CQAs are predicted using a machine learning model that takes as input at least one predictor variable that is not a measured variable of the process, and is instead modeled as described herein. Optionally, the cell metabolic condition is determined at step 830 using the metabolic condition model, and the metabolic condition is classified using data-driven classification (by the multivariate statistical modeling engine and/or the data driven machine learning engine). At optional operation 835, specific productivity and product quality are predicted using the metabolic condition model (e.g. using a multivariate model trained to predict titer from metabolic condition variables) and/or the kinetic growth model (e.g. by integrating the equation capturing the change in titer and/or quality of the product), and titer and quality of biomaterial is predicted from this. This step is different from the prediction of CQAs performed at step 825, which uses a machine learning model taking as inputs values of one or more state variables of the kinetic growth model. Indeed, at optional step 835, the metabolic condition variables that are optionally determined at step 830 (such as e.g. variables obtained by PCA or (O)PLS applied to metabolic condition the specific transport rates and one or more other state variables) are used to predict titer and features of the quality of the material that can be obtained from this such as e.g. the presence of by-products etc using a multivariate model. By contrast, at step 825 a machine learning model is used to directly predict CQAs from variables of the system equations integrated at step 820. The method of Figure 8 may be repeated using a different set of input variable trajectories and/or initial state values, in order to identify values of these variables that satisfy one or more optimality criteria, including one or more optimality criteria that apply to the predicted CQAs, such as e.g. achieving values of one or more CQAs within specification. An optimization algorithm implements steps to explore a space of possible values in order to identify one or more sets of values that are satisfy these optimality criteria.

Finding the optimal adjustments or settings for a process may be defined as finding the set of manipulated variables (*u* or independent variables, in this case, process conditions and feeds) that minimize (or maximise) a mathematical objective function j. For example, an objective function to maximize the titer at a future time point $t_{(t+k)}$ may take the following form:

$$j = -\widehat{IgG}\big|_{[t+k]'} = f_{IgG}\left(x\big|_{[t]}, u\big|_{[t]}\right) \qquad (31)$$

where $\widehat{IgG}\big|_{[t+k]}$ is the predicted titer at the future time point, $x\big|_{[t]}$ are the current value of the states, and $u\big|_{[t]}$ are the set of manipulated variables to be implemented between now [t] and the future time point [t+k]. In embodiments, multiple objectives are simultaneously optimized. This may be performed by weighting the objectives based on their importance. For example, to maximize titer and maintain a quality metric on target, the objective function may take the following form:

$$j = -\theta_{IgG}\widehat{IgG}\big|_{[t+k]} + \theta_q\left(\hat{q}\big|_{[t+k]} - q_{sp}\right)^2 \qquad (32)$$

where $\theta$ are the relative weights for each parameter to be optimized, and $q_{sp}$ is the target or set point for the quality parameter q. There is a similar function ($f_q$) to the function for IgG to predict the future quality variable at a future time point. Further, constraints may be added to the function. For example, the optimization objective to maximize titer subject to maintaining quality within operating specifications may be governed by equation (32) subject to the following constraint:

$$q_{min} \leq \hat{q}\big|_{[t+k]} \leq q_{max} \quad (33).$$

Additionally, in order to prevent the optimization algorithm from selecting a new set of inputs that are infeasible, limits may also be placed on *u*. Additionally, the extent to which the optimisation algorithm modifies conditions *u* when exploring the space of possible values or when providing an instruction to change current operating conditions to a controller (e.g. where a suboptimal state has been identified and the model has been used together with an optimisation algorithm to identify changes to the process conditions that could correct this) can be tuned by placing penalties on changes in *u* from recipe or current settings. This prevents the controller from making erratic or large changes to the process conditions that provide minimal improvement to the objective parameters. The complete objective function then is described as finding the optimal set of feasible u that maximizes titer (IgG) and maintains quality on target and within specified limits. This may be governed by the following equation:

$$j = -\theta_{IgG}\widehat{IgG}\big|_{[t+k]} + \theta_q\left(\hat{q}\big|_{[t+k]} - q_{sp}\right)^2 + \theta_u\left(u\big|_{[t]} - u_{sp}\right)^2 \qquad (34)$$

subject to the constraint in equation (33) and $u_{min} \leq u\big|_{[t]} \leq u_{max}$ wherein $\theta_u$ are the penalty weights for *u* (note there may be more than one *u*), and $u_{sp}$ is the target value of *u*, which is usually a setpoint value or the current value.

The techniques provided herein provide models which accurately mimic cell behavior in a bioreactor. The predicted VCD and viability profiles were showed to match measured experimental values for various feed, pH, and temperature profiles, using experimental data. As can be seen on **Figure 9,** the hybrid model described above was able to replicate experimentally measured behaviour, and to identify different functional cell states. In particular, as shown in figures 9A-9C, a shift in temperature predicted by the kinetic growth model to inhibit growth was confirmed to do so. A pH shift appears to slightly increase cell death rates, but does not appear to inhibit growth rates. The cells seem to adapt and recover well from glucose and glutamine depletion. Therefore, no significant change is observed in growth, as the cells likely metabolize other carbon sources. As shown on Figure 9D, cell state classification using a data-driven approach (PCA, in this case) applied to specific consumption rates calculated based on the measured data was able to identify that a bioprocess was operating in a suboptimal range.

Additionally, the hybrid model effectively acts as a soft sensor for cell metabolism and metabolites, allowing monitoring and characterization of metabolite specific consumption and production, as well as monitoring and characterization of changes in cell state and metabolic activity. Unlike other models, which do not estimate or otherwise account for lysed cells, the hybrid model takes into account the number of lysed cells, which influences bulk fluid toxicity. This approach allows the hybrid model to be more accurate than other models that do not account for this feature, and at longer time scales than other models. Other advantages of the hybrid model include having an increased knowledge of cell metabolism and of factors that drive cell growth, cell death, viability, titer and product quality. The hybrid model also provides the ability to simulate performance of new process conditions (e.g., feeding, temp, pH profiles, etc.) to maximize productivity and to observe cell state (e.g., metabolic activity, etc.) or changes thereof. In other aspects, perfusion performance may be predicted from fed batch operation. These techniques provide for improved forecasting, improved prediction of CQAs, and also optionally of titer and other product related metrics based on monitoring and prediction. Present techniques are applicable to a wide variety of application areas including a simple univariate metabolite state estimator, a comprehensive multivariate metabolite state estimator, live systems (e.g., digital twin simulation), etc. Accordingly, present techniques are in an improvement in the field of bioreactor control and biologics manufacturing.

### *Examples*

Exemplary methods of calibrating a machine learning model, as well as exemplary methods for predicting CQAs in a bioprocess will now be described.

### *Materials and Methods*

### *Data*

The data used in these examples was collected from 6 batches of cell cultures in miniature bioreactors (Ambr250™) with a working volume of approximately 200 ml. Each individual batch was allowed to run for 8-14 days. One batch corresponds to one full operating cycle: filling the bioreactor, growth phase, production phase and decay phase followed by emptying the vessel. The cells in the bioreactor were CHO (Chinese Hamster Ovary) cells producing a recombinant antibody (denoted "IgG"). The concentration of this product is referred to as the "titer". In this example, bolus additions of ammonia were performed to assess the cell's response to ammonia stress (which is not relevant to the present example). Over a period of 8-14 days that the batches are active the state of the bioreactor was measured once per day. The following variables were measured: viable cell density (VCD), cell viability (which may be obtained as VCD/TCD where the TCD is the total cell density, which may be equal to VCD+DCD where DCD is the dead cell density - in the present case the cell viability and VCD were obtained by measuring the TCD then counting cells in the presence of a dye, such as e.g. a fluorescent dye, that dyes live cells to obtain the VCD), dissolved oxygen (DO), pH, temperature, volume, volume sampled, integral of viable cells (IVC) and integral viable cell concentration (IVCC) (calculated from the measured VCD), and amount of ammonia added (through bolus additions, in mmol). Additionally, the concentrations of a plurality of metabolites were also measured once a day by mass spectrometry, including: alanine, choline, histidine, methionine, threonine, glucose, arginine, cysteine, isoleucine, phenylalanine, tryptophan, lactate, asparagine, glutamate, leucine, proline, tyrosine, ammonia, aspartic acid, glycine, lysine, serine, and valine. Finally, the glycan profile of the product was measured once at the end of the operating cycle for each batch, by LC-MS. This included values for the following variables: GOF-N, G2, G0, G2F, G0F, G1FS1, G1, G1', G1F, G1 F', G2S1, G2FS1' and G2FS1. An additional variable (G2S1') was available for 4 out of the 6 batches.

The data for each measurement used for training was standardised (each observation $X$ was scaled to

$$Z = \frac{X-\mu}{\sigma}$$

where $\mu$ is the mean value of the variable and $\sigma$ is the standard deviation). Standardisation is expected to improve the speed and stability of the training as it reduces the risk of variables with large values having a disproportionate impact on the training because of their values, not because of their importance to the prediction. Standardisation results in all variables Z being distributed with a mean=0 and a standard deviation=1.

Two of the batches lacked measurements for the temperature, dissolved oxygen and pH. In order to keep as many batches as possible for training, the temperature, dissolved oxygen and pH variables were removed from all batches and were not used. Thus, information related to the bolus additions of ammonia was the only process parameter used in this example. Note that the above mentioned process parameters are expected to provide useful information to the model when available. However, the examples below demonstrate the robustness of the approach even in the presence of practical limitations in relation to the process parameter information that is available.

The term "label" refers to the (assumed) true value of a variable that is predicted by a machine learning model. In these examples, machine learning models were trained to predict the glycan profile variables measured for each batch. Thus, the labels for each batch are the values of the glycan profile variables. All labels were standardised across the training data set, as explained above.

*Simulated variables*

A state space model as described herein (see above, referred to as 'hybrid state space model' (HSSM) and implemented in an in-house Python library) was used to simulate the value of a plurality of state variables for each batch using initial conditions including: the measured concentration of the metabolites listed above at the start of the process, the initial bulk concentration of any other metabolite modelled in the HSSM (based on medium composition), the cell density (including viable cell density and dead cell density), initial lysed cell concentration (assumed to be 0), initial biomaterial concentration (assumed to be 0), and any model inputs (i.e. known process variables, whether set or measured, such as pH, temperature, volume, and dissolved oxygen). The state variables simulated included: glutamine concentration (from equation (26)), viable cell density (VCD; from equation (11)), cell viability (VCD/TCD, from equations (11)-(14)), dead cell density (from equation (12)), lysed cell density (from equation (13), biomaterial (from equation (16), this is an unknown biomaterial that accumulates in the cell culture as a result of cell growth), product concentration (titer, from equation (26) or equation (30)), and time. Further variables were derived from these state variables including effective growth rate (calculated using equation (22)), death rate (equation (15)), and specific productivity of titer (predicted using a PLS model based on metabolic conditions as explained in relation to Figure 7). The measurements of the concentrations of the metabolites listed above (i.e. alanine, choline, histidine, methionine, threonine, glucose, arginine, cysteine, isoleucine, phenylalanine, tryptophan, lactate, asparagine, glutamate, leucine, proline, tyrosine, ammonia, aspartic acid, glycine, lysine, serine, and valine) were used to calculate the specific transport rates used in the model (using equation (27c)).

*Machine learning models*

In these examples, three machine learning models were independently trained: a first model used as input all measured variables available at a single maturity corresponding to the latest time point at which the measurements were available, a second model used as input a subset of the measured variables available (in particular, volume, viable cell density, glutamate, glucose, lactate and ammonia, which are commonly measured in industrial bioprocesses) at a single maturity corresponding to the latest time point at which the measurements were available, a third model used as input all state variables and variables derived therefrom produced by simulation of the bioprocesses as described above, in addition to the metabolite concentrations, all at a single maturity corresponding to the latest time point at which metabolite concentration measurements were available.

Thus, the first model used 30 input variables: Volume, Volume sampled, Viability, Viable cell density (VCD), Integral of Viable Cells (IVC), Integral Viable Cell Concentration (IVCC), amount of ammonia added, and concentrations of 23 metabolites (alanine, choline, histidine, methionine, threonine, glucose, arginine, cysteine, isoleucine, phenylalanine, tryptophan, lactate, asparagine, glutamate, leucine, proline, tyrosine, ammonia, aspartic acid, glycine, lysine, serine, and valine), all measured / simulated at a single time point (the latest available measurement time point, which was 8.5 days for two of the batches and 14.5 days for the other four). In Example 2, all metabolite concentrations used were simulated using the hybrid state space model described herein and measured initial metabolite concentrations. Indeed, as explained above, when metabolite concentrations are not available for a particular time point, it is possible to simulate them using the hybrid state space model described herein. In other words, simulated values can be obtained for e.g. time points corresponding to the latest planned measurement time point of the process, such that the CQAs can be predicted before the planned measurement time point has in fact been reached, enabling e.g. corrective action to be implemented.

The second model used 6 input variables: volume, viable cell density, glutamate, glucose, lactate and ammonia concentrations, all measured / simulated at a single time point (the latest available measurement time point).

The third model used 34 input variables (all measured / simulated at the latest available time point): measured concentrations of 23 metabolites (alanine, choline, histidine, methionine, threonine, glucose, arginine, cysteine, isoleucine, phenylalanine, tryptophan, lactate, asparagine, glutamate, leucine, proline, tyrosine, ammonia, aspartic acid, glycine, lysine, serine, and valine), simulated concentration of glutamine, simulated viable cell density, simulated cell viability, simulated dead cell density, simulated lysed cell density, simulated product concentration, biomaterial, time (i.e. 8.5 days for two of the batches and 14.5 days for the other four), and variables derived from simulated variables including the effective growth rate, death rate, and specific productivity of titer.

All models were trained to jointly predict all glycan profile features. Two versions of the third model were trained: one using all batches but without G2S1' as output, and one with only the 4 batches for which G2S1' was available. The first and second models were both trained using all batches but without G2S1', G1', G1F', and G2FS1', as output.

All networks were feed-forward neural networks (NN). A variety of architectures were tested (data not shown) before choosing fully connected neural networks with three-hidden layers. A plurality of lag values (no lag, lag=1, lag=2) were tested, but as mentioned above architectures using a single time point for input values were used in the final networks.

All networks were 4-hidden-layer multilayer perceptrons (MLP) with 15 hidden nodes in each layer. For the first model, the input layer of the model had 30 nodes and the output layer had 10 nodes (G0F-N, G0, G0F, G1, G1F, G2, G2F, G1FS1, G2S1 and G2FS1). For the second model, the input size was 6 and the output size was 10 (G0F-N, G0, G0F, G1, G1F, G2, G2F, G1FS1, G2S1 and G2FS1). For the third model, the input size was 34 and output size was 13 or 14 (depending on whether G2S1' is predicted or not, i.e. GOF-N, G2, G0, G2F, G0F, G1FS1, G1, G1', G1F, G1F', G2S1, G2FS1', G2FS1, and (G2S1')).

ReLU (rectified linear unit) was used as activation function for all networks, in each of the four layers of the networks.

*Training of the machine learning models*

The training, or fitting, of the network is done by using a training dataset. This dataset contains input values and corresponding true output values, or labels. The input values are fed through the network and its output is compared to the labels by a loss function.

A loss function determines how close the network prediction was to the label, if the loss is 0, then the network prediction is the same as the label. A common loss function for regression problems is the mean squared error (MSE). This loss, or error, is then fed backwards through the network, calculating the gradients of the loss function with respect to the weights. This is called back-propagation of the error. Using the gradients the weights are then adjusted by an optimizer, for example based on gradient descent, to minimize the loss function.

Typically the network trains on the entire dataset many times, and each time it iterates through the dataset is called an epoch. Calculating the loss and gradients of the whole dataset can be very costly both in computation time and memory, so usually the loss is calculated on a small sample of the dataset, called a batch. Calculating the gradient and updating weights on a small sample of the dataset will result in a less accurate step toward minimizing the loss function for the entire dataset, but doing it many times will result in similar results while using less computing power resources and time.

In the present examples, Adam (Diederik P. Kingma, Jimmy Ba, arXiv:1412.6980, Dec 2014) was used as optimizer, MSE was used as loss function, and the batch size was 1 for all networks. For the training of the first and second models, early stopping with a validation set was not used, instead a fixed number of 75 epochs was used for all folds. For the training of the third model, early stopping with the test set was used. When the training was finished, the test set's values of the predictor variables were passed forward through the network to make predictions. These predictions were compared to the actual measured values, and the error was computed by a norm-wise relative error (equation (35):

$$RE = \frac{|x - \hat{x}|}{|x|} \qquad (35)$$

where $x$ is the true value and $\hat{x}$ is an approximation to $x$. The mean value of the errors from all 6 folds was computed together with its standard deviation. When early stopping was used (third model), the model was trained until no decrease in validation loss is observed, after which the model with the lowest validation loss was used. The training stopped after 20 consecutive epochs with no decrease in validation loss (typically 60-100 epochs in total). Of note, such an "early stopping" approach would have also been suitable for training the other two models.

The present examples use k-fold cross-validation, which is based on the idea of repeating the training and testing computation on different randomly chosen subsets of the original dataset. The procedure is such that a partition of the dataset is formed by splitting it into k non-overlapping subsets. The test loss is then estimated by calculating the average test loss across k trials. On trial i the i-th subset of the data is used as test set and the rest of the data is used for training.

In the present case, 6-fold cross-validation was used. To assign validation sets, observations from a single complete batch were assigned as validation set instead of the standard practice assigning observations at random. Since observations within a batch are correlated, this strategy reduces risk of overly optimistic validation performance. Thus, a 6-fold leave-one-out cross-validation was used, with one batch as test set and the other 5 as training set.

*Evaluation*

The predictions of the machine learning models were compared with a baseline constructed by simply taking the average label value over all batches, but leaving out the one that was to be predicted. For example, the baseline prediction of 'G0F-N' for batch 6 would be the average of the measurements of 'G0F-N' for batches 1 to 5. This reflects the situation where, in the absence of the present method, the only way to predict the value of a CQA is by using a value derived from a set of batches that are assumed to have followed a similar course and hence likely to have a product with the same CQAs.

For both the machine learning predicted values and the baseline predicted values, prediction errors were calculated as MSEs, and a 95% confidence interval was calculated for each MSE using:

$$(\hat{\mu}_m - 1.96 SE(\hat{\mu}_m), \hat{\mu}_m + 1.96 SE(\hat{\mu}_m)) \qquad (36)$$

$$SE(\hat{\mu}_m) = \frac{\sigma}{\sqrt{m}} \qquad (37)$$

where $\hat{\mu}$ is the estimated mean of the MSE for each fold, $m$ is the number of samples (folds, in this case), $SE(\hat{\mu}_m)$ is the standard error, and $\sigma$ is the standard deviation of the samples (approximated by $\hat{\sigma}$, the estimated standard deviation).

### *Example 1 - measured variables*

In this example, machine learning models were trained as explained in the methods section to predict a plurality of glycan profile features using measured metabolites and cell culture variables, and the predictions of the networks were compared to the corresponding baseline values calculated from previously acquired data as explained above.

The results of this can be seen on **Figure 10.** Figures 10A and 10B show the predictions from the first model for two representative batches (respectively on Figures 10A and 10B), together with the corresponding (true) measured value. These figures show that the first model was able to predict most of the glycan peaks with relatively high accuracy (average relative error around 10-20%). On Figure 10C, each plot compares: on the left, the MSE between the CQAs predicted by the first model for a glycan profile feature and the corresponding labels (where the height of the bar represents the average MSE over the 6 folds of the cross-validation, and the error bars indicate the 95% confidence interval); in the middle, the MSE between the CQAs predicted by the second model for a glycan profile feature and the corresponding labels (where the height of the bar represents the average MSE over the 6 folds of the cross-validation, and the error bars indicate the 95% confidence interval); and on the right, the MSE between the CQAs calculated as an average over 5 batches for a glycan profile feature and the corresponding labels (where the height of the bar represents the average MSE over the 6 batches and the error bars indicate the 95% confidence interval around this average).

The norm-wise relative errors calculated for each of the models and each of the glycan profile features on Figure 10C were compared to each other using individual paired t-tests. The p-values from these tests are shown in Table 1, where the first column compares the prediction from the first model to the corresponding baseline prediction, and the second column compares the prediction from the first model to the corresponding prediction from the second model. As can be seen in Table 1, the predictions from the first model were significantly more accurate (p-value < 0.05) than the baseline predictions for at least 4 of the predicted quality attributes (G0, G1, G1FS1 and G2S1). The predictions from the first model were more accurate than the baseline predictions for all other predicted quality attributes, but these failed to reach significance (although most came close), most likely due to the small size of the training data set. Table 1 also shows that the first model performed significantly better than the second model for at least 5 of the predicted quality attributes (G0, G0F, G1, G2S1, G2FS1). This indicates that there are benefits to including more predictive variables in the models. In particular, this underlines the assumption that the use of additional variables derived from a state space model as described above would advantageously increase the prediction accuracy of these models. Indeed, the data shows that using more predictive variables of the type that can be calculated by such a state space model improves the prediction of the model (compare the second model where some of the cell state variables and metabolite concentrations used in the first model were removed from the set of input variables). As indicated above, it has been shown that such a state space model is able to accurately predict the course of cell state (e.g. viable cell density, lysed cell density, etc.) and metabolic state variables (specific transport rates, metabolite concentrations, etc.). Thus, including these calculated / simulated variables as predictors in the machine learning model would likely increase the prediction accuracy of the machine learning models to a similar extent as when measured counterparts are used, but with the added benefit that the measurement burden is reduced (in practice it is rare for many measurements to be available) and that the prediction can be made while the bioprocess is running (which is not possible when actual measurements are used in many cases as there is a significant delay in obtaining these measurements).

Note that the results from the second model also indicate that even models trained with a relatively small amount of input variables could still be useful as these perform as well as the baseline predictions. However, baseline predictions would only be available when comparative bioprocesses with measured CQAs are available, which may not always be the case.

**Table 1.** p-values from one-sided batch-wise paired t-tests comparing predictions from the first and second models and baseline predictions.

| Glycan profile feature | $RE_F < RE_B$ | $RE_F < RE_R$ |
|---|---|---|
| G0F-N | 0.156137 | 0.341746 |
| G0 | 0.027334 | 0.029195 |
| G0F | 0.095006 | 0.042673 |
| G1 | 0.012038 | 0.016407 |
| G1F | 0.052576 | 0.053633 |
| G2 | 0.103484 | 0.173918 |
| G2F | 0.083867 | 0.055545 |
| G1FS1 | 0.019786 | 0.053571 |
| G2S1 | 0.036021 | 0.019181 |
| G2FS1 | 0.066915 | 0.033573 |

The particular glycan profile features that are being predicted are biologically related to each other through the metabolism of the cells. Therefore, it is plausible that jointly predicting values for multiple glycan features increases the performance of the network, if the network architecture is able to capture the correlations between those values that reflect their biology. However, for other CQAs it may be advantageous to train a separate model for each CQA or for a subset of CQAs to be predicted.

Further, this data indicates that the predictions from these networks may be improved by including lagged values of the predictor variables in the model, in order for the model to be able to use richer information to make the predictions.

### *Example 2 - simulated variables*

In this example, a machine learning model was trained as explained in the materials and methods section using simulated variables in addition to measured variables. The simulated variables were obtained by simulating a bioreactor with initial conditions corresponding to the initial conditions in the data presented in the materials and methods sections, and process conditions other than viable cell densities (which are predicted by the model) set to those in the data (i.e. pH, temperature, DO, volume). The results of this are shown on **Figure 11.**

On Figure 11, each plot compares: on the left, the MSE between a glycan feature predicted by the third machine learning model and the corresponding measured concentration (where the height of the bar represents the average MSE over the 6 batches, and the error bars indicate the 95% confidence interval around this average); and on the right, the MSE between the glycan feature calculated as an average across 5 batches and the corresponding labels (where the height of the bar represents the average MSE over the 5 batches and the error bars indicate the 95% confidence interval around this average).

The data on Figure 11 indicates that very good prediction accuracy can be obtained using this augmented model for all predicted quality attributes. Indeed, the machine learning based predictions were significantly better than the baseline predictions in all cases. Comparing the results in Figures 11 and 10C, it can be seen that augmentation of the training data with variables from the state space model further improved the prediction accuracy of the model in many cases (at least GOF-N, G1F, G2, G2F, G1FS1, G2S1, G2FS1).

### *Equivalents and Scope*

All documents mentioned in this specification are incorporated herein by reference in their entirety.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of', unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

**Claims**

1. A computer-implemented method for monitoring a bioprocess comprising a cell culture in a bioreactor, the method comprising:

    obtaining the values of one or more state variables of a state space model and optionally one or more variables derived therefrom, at one or more maturities, the state space model comprising a kinetic growth model representing changes in the state of the cell culture and optionally a material balance model representing changes in the bulk concentration of one or more metabolites in the bioreactor; and
    predicting the value of one or more critical quality attributes of a product of the bioprocess using a machine learning model trained to predict the value of the one or more critical quality attributes based on input variables comprising values of the one or more state variables or variables derived therefrom, at one or more maturities.

2. The method of claim 1, wherein the method further comprises obtaining values of one or more process conditions including one or more process parameters and/or one or more metabolite concentrations at one or more maturities and the input variables further comprise values of the one or more process conditions, and/or wherein the input variables comprise values of at least one of the one or more state variables.

3. The method of claim 1 or claim 2, wherein the product comprises: one or more biomolecules, such as one or more small or macromolecules produced by the cells, and/or the biomass in the culture, and/or parts thereof such as one or more organelles;
    and/or wherein the one or more critical quality attributes are selected from: the molecular structure of a small molecule or macromolecule that is comprised in the product or is the product, the glycosylation profile of a protein or peptide that is comprised in the product or is the product, the activity of the product, the yield of the product, the presence or absence of one or more host cell protein(s), and the purity of the product.

4. The method of any preceding claim, wherein the one or more state variables or variables derived therefrom comprise at least one variable selected from: a specific transport rate of a metabolite, a bulk fluid concentration of a metabolite, a bulk fluid concentration of a product, the bulk fluid concentration of a biomaterial that accumulates in the culture as a result of cell growth and inhibits cell growth, specific productivity of titer, and a cell state variable, optionally wherein the cell state variable is selected from: viable cell density, dead cell density, total cell density, cell viability, effective growth rate, death rate, and lysed cell density, preferably wherein the cell state variable(s) include lysed cell density; and/or optionally wherein the one or more state variables or variables derived therefrom are obtained using any of equations (11)-(30), and equivalents thereof, in particular any or all of equations (11)-(16), (22), (25)-(27) and equivalents thereof.

5. The method of claim 4, wherein the specific transport rate of a metabolite i is the net amount of the metabolite transported between the cells and the culture medium, per cell and per unit of maturity.

6. The method of any of claims 2 to 5, wherein the one or more process conditions include one or more process parameters selected from dissolved oxygen, dissolved CO2, pH, temperature, osmolality, agitation speed, agitation power, headspace gas composition (such as e.g. $CO_2$ pressure), flow rates (such as feed rate, bleed rate, harvest rate), feed medium composition and volume of the culture; and/or wherein the one or more metabolite concentrations include the concentration of one or more metabolites in the cellular compartment, in the culture medium compartment, or in the cell culture as a whole.

7. The method of any preceding claim, wherein the method further comprises comparing the value of the one or more critical quality attributes to one or more predetermined values, and optionally determining whether the bioprocess is operating normally based on the comparing.

8. The method of any preceding claim, wherein the machine learning model is a regression model, or wherein the machine learning model is selected from a linear regression model, a random forest regressor, an artificial neural network (ANN), and a combination thereof, suitably wherein the machine learning model is an artificial neural network; and/or wherein the machine learning model comprises a plurality of machine learning models, wherein each machine learning model has been trained to predict the values of an individually selected subset of the one or more critical quality attributes; and/or wherein the machine learning model has been trained to jointly predict value of the one or more critical quality attributes.

9. The method of any preceding claim, wherein the machine learning model has been trained to predict the value of the one or more critical quality attributes at a maturity corresponding to the end of the bioprocess based on input variables comprising values of the one or more state variables or variables derived therefrom and optionally values of one or more process conditions, at one or more preceding maturities, optionally wherein the bioprocess is a batch or a fed-batch process; and/or
wherein the machine learning model has been trained to predict the value of the one or more critical quality attributes at current maturity based on input variables comprising values of the one or more state variables or variables derived therefrom and optionally values of one or more process conditions, at one or more maturities including the current maturity and/or one or more preceding maturities, optionally wherein the one or more maturities include the current maturity and/or wherein the bioprocess is a perfusion process.

10. The method of any preceding claim, wherein obtaining values of one or more state variables and optionally one or more process conditions at one or more maturities comprises obtaining values of the one or more state variables and optionally one or more process conditions at a plurality of maturities; and the machine learning model has been trained to predict the one or more critical quality attributes at a latest of the plurality of maturities or a later maturity using inputs comprising the value of the one or more state variables and optionally one or more process conditions for the bioprocess at the plurality of maturities.

11. The method of any preceding claim, wherein obtaining the values of one or more state variables of a state space model or variables derived therefrom at one or more maturities comprises predicting a state trajectory of the bioprocess using the state space model, optionally by finding values of the one or more state space variables that represent a solution of the state space model at the one or more maturities.

12. A computer-implemented method for controlling a bioprocess, the method comprising:

performing the method of any of claims 1 to 11;
comparing the value of the one or more critical quality attributes or values derived therefrom to one or more predetermined values; and
determining on the basis of the comparison whether to implement a corrective action.

**13.** The method of claim 12, further comprising sending a signal to one or more effector device(s) to implement a corrective action if the determining step indicates that a corrective action is to be implemented.

**14.** A method of providing a tool for monitoring a bioprocess comprising a cell culture in a bioreactor, the method including the steps of:

obtaining the values of one or more state variables of a state space model and optionally one or more variables derived therefrom and/or the values of one or more process conditions, at one or more maturities for a plurality of bioprocesses, the state space model comprising a kinetic growth model representing changes in the state of the cell culture and optionally a material balance model representing changes in the bulk concentration of one or more metabolites in the bioreactor; and

using the values obtained to train a machine learning model to predict the value of one or more critical quality attributes based on input variables comprising values of the one or more state variables and/or variables derived therefrom, at one or more maturities.

**15.** A system for monitoring a bioprocess, for controlling a bioprocess and/or for providing a tool for monitoring and/or controlling a bioprocess, the system including:

at least one processor; and

at least one non-transitory computer readable medium containing instructions that, when executed by the at least one processor, cause the at least one processor to perform the method of any of preceding claim;

optionally wherein the system further comprises, in operable connection with the processor, one or more of:

a user interface, optionally wherein the instructions further cause the processor to provide, to the user interface for outputting to a user, one or more of: the value of the one or more critical quality attributes or variables derived therefrom, the result of the comparing step, and a signal indicating that the bioprocess has been determined to operate normally or to not operate normally;

one or more biomass sensor(s);

one or more metabolite sensor(s);

one or more process condition sensors; and

one or more effector device(s).

Fig. 1

Fig. 2A

Fig. 2B

Fig. 2C

Fig. 2D

Fig. 3A

Fig. 3B

Fig. 4

Fig. 5

Fig. 6

Fig. 7

Fig. 8

Fig. 9A

Fig. 9B

Fig. 9C

Fig. 9D

Fig. 10A

Fig. 10B

Fig. 10C

Fig. 11

Europäisches
Patentamt

European
Patent Office

Office européen
des brevets

## EUROPEAN SEARCH REPORT

**Application Number**

**EP 21 18 4299**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/377844 A1 (WOLFGANG, P ET AL.) 3 December 2020 (2020-12-03) <br> * paragraph [0004] - paragraph [0007] * <br> * paragraph [0010] - paragraph [0011] * <br> * paragraph [0018] - paragraph [0020] * <br> * paragraphs [0036], [0057], [0096] * <br> * paragraph [0117] - paragraph [0129] * <br> * paragraph [0410] - paragraph [0411] * <br> * figures 3A,16,17 * <br> * paragraph [0176] * <br> ----- | 1-15 | INV. <br> C12M1/36 <br> G05B13/04 <br> G16B40/00 |
| X | KRÄMER D ET AL: "A hybrid approach for bioprocess state estimation using NIR spectroscopy and a sigma-point Kalman filter", <br> JOURNAL OF PROCESS CONTROL, OXFORD, GB, <br> vol. 82, 26 November 2017 (2017-11-26), <br> pages 91-104, XP085814906, <br> ISSN: 0959-1524, DOI: <br> 10.1016/J.JPROCONT.2017.11.008 <br> [retrieved on 2017-11-26] <br> * Sections 2.1, 2.2, 2.3, 3.1, 3.2, 4.2; Figures 1, 2 and 7; Table 1 * <br> ----- | 1-15 | |
| X | WO 2020/260229 A1 (CYTIVA SWEDEN AB [SE]) 30 December 2020 (2020-12-30) <br> * page 4, line 21 - page 6, line 5 * <br> * page 8, line 1 - page 9, line 2 * <br> * page 13, line 29 - line 30 * <br> * claims 1-17; figures 1,2 * <br> ----- | 1-15 | TECHNICAL FIELDS SEARCHED (IPC) <br><br> C12M <br> G06F <br> G05B <br> G16B |
| E | WO 2021/164957 A1 (SARTORIUS STEDIM DATA ANALYTICS AB [SE]) 26 August 2021 (2021-08-26) <br> * claims 1-15; figure 1 * <br> ----- <br><br> -/-- | 1-15 | |

The present search report has been drawn up for all claims

| Place of search | Date of completion of the search | Examiner |
|---|---|---|
| Berlin | 15 December 2021 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

<table>
<tr><td colspan="2">Europäisches Patentamt<br>European Patent Office<br>Office européen des brevets</td><td>**EUROPEAN SEARCH REPORT**</td><td>**Application Number**<br>EP 21 18 4299</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2021/165495 A1 (SARTORIUS STEDIM DATA ANALYTICS AB [SE]) 26 August 2021 (2021-08-26) * paragraphs [0003], [0122]; claims 1-26 * ----- | 1-12,14 | |
| A | US 2020/202051 A1 (SWAMINATHAN KRISHNA KUMAR [IN] ET AL) 25 June 2020 (2020-06-25) * paragraph [0038] - paragraph [0041] * * paragraph [0044] - paragraph [0051] * * paragraph [0058] * * paragraph [0075] - paragraph [0103] * * claims 1,23 * * figure 6 * ----- | 1-15 | |

TECHNICAL FIELDS SEARCHED (IPC)

The present search report has been drawn up for all claims

| Place of search | Date of completion of the search | Examiner |
|---|---|---|
| Berlin | 15 December 2021 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 4299

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2021

| Patent document cited in search report | | Publication date | Patent family member(s) | | Publication date |
|---|---|---|---|---|---|
| US 2020377844 | A1 | 03-12-2020 | CN | 112119306 A | 22-12-2020 |
| | | | EP | 3732485 A1 | 04-11-2020 |
| | | | JP | 2021508872 A | 11-03-2021 |
| | | | US | 2020377844 A1 | 03-12-2020 |
| | | | WO | 2019129891 A1 | 04-07-2019 |
| WO 2020260229 | A1 | 30-12-2020 | NONE | | |
| WO 2021164957 | A1 | 26-08-2021 | US | 2021262047 A1 | 26-08-2021 |
| | | | WO | 2021164957 A1 | 26-08-2021 |
| | | | WO | 2021165495 A1 | 26-08-2021 |
| WO 2021165495 | A1 | 26-08-2021 | US | 2021262047 A1 | 26-08-2021 |
| | | | WO | 2021164957 A1 | 26-08-2021 |
| | | | WO | 2021165495 A1 | 26-08-2021 |
| US 2020202051 | A1 | 25-06-2020 | CN | 110945593 A | 31-03-2020 |
| | | | EP | 3639171 A1 | 22-04-2020 |
| | | | JP | 2020523030 A | 06-08-2020 |
| | | | US | 2020202051 A1 | 25-06-2020 |
| | | | WO | 2018229802 A1 | 20-12-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160152936 A

- WO 2014020327 A

**Non-patent literature cited in the description**

- **BREIMAN, LEO.** Random forests. *Machine learning,* 2001, vol. 45 (1), 5-32
- **RUMELHART, DAVID E. ; GEOFFREY E. HINTON ; RONALD J. WILLIAMS.** Learning representations by back-propagating errors. *Nature,* 1986, vol. 323 (6088), 533-536
- **KIEFER, JACK ; JACOB WOLFOWITZ.** Stochastic estimation of the maximum of a regression function. *The Annals of Mathematical Statistics,* 1952, vol. 23 (3), 462-466

- Robust estimation of a location parameter. **HUBER, PETER J.** Breakthroughs in statistics. Springer, 1992, 492-518
- **WOLD et al.** PLS-regression: a basic tool of chemometrics. *Chemometrics and Intelligent Laboratory Systems,* 2001, vol. 58, 109-130
- **WOLD et al.** Principal Component Analysis. *Chemometrics and Intelligent Laboratory Systems,* 1987, vol. 2, 37-52
- **DIEDERIK P. KINGMA ; JIMMY BA.** *arXiv:1412.6980,* December 2014